# EUROPÄISCHE PATENTSCHRIFT

(11) **EP 0 442 348 B1**
(45) Veröffentlichungstag und Bekanntmachung des Hinweises auf die Patenterteilung: **17.07.1996**
(21) Anmeldenummer: 91101497.5
(22) Anmeldetag: 05.02.1991
(51) Int. Cl.: C07D 233/84, A61K 31/415, C07D 491/10, C07D 401/12, C07D 403/12, C07D 413/12, C07D 417/12

(54) **Neue Imidazolverbindungen, Verfahren zu deren Herstellung, Arzneimittel auf Basis dieser Verbindungen sowie einige Zwischenprodukte**
Imidazole compounds, process for preparation, medicaments based on these compounds and some intermediates
Composés d'imidazole, procédé de préparation, médicaments à base de ces composés et quelques intermédiaires

(30) Priorität: 10.02.1990 DE 4004061
(43) Veröffentlichungstag der Anmeldung: 21.08.1991
(73) Patentinhaber: HOECHST AKTIENGESELLSCHAFT, 65926 Frankfurt am Main (DE)
(72) Erfinder: Graeve, Rolf, Dr., W-6204 Taunusstein (DE); Okyayuz-Baklouti, Ismahan, Dr., W-6200 Wiesbaden (DE); Seiffge, Dirk, Dr., W-6500 Mainz (DE)

(56) Entgegenhaltungen:
- EP-A- 0 105 575
- EP-A- 0 284 277
- DE-A- 3 504 677
- US-A- 3 932 444

## Beschreibung

Krankheiten des Kreislaufsystems standen in den letzten Jahren mit über 50 % an der Spitze aller Todesfälle. Hier wiederum dominierten hauptsächlich thromboembolische Komplikationen. Trotz weltweiter intensiver Bemühungen um Fortschritte in der Aufklärung von Krankheitsursachen, Charakterisierung und Erkennung relevanter Risikofaktoren und Entwicklung verläßlicher Behandlungsmethoden fehlt bis heute eine zufriedenstellende medikamentöse Therapie (L. Harker, in: Seminars in Thrombosis and Hemostasis, Vol. 12, No. 2, 134-155, 1986; de Gaetano et al., in: Current issues in thrombosis prevention with antiplatelet drugs, 31, 517-549, 1986). Das übergeordnete Ziel einer antithrombotischen, antiischämischen Therapie ist die Korrektur der gestörten Organfunktionen (z.B. der Muskelleistung bei Claudicatio intermittens) und somit eine Verbesserung der Lebensqualität durch Verhinderung einer frühen Invalidisierung und letztlich die Verhütung letaler Ereignisse.

Etwa 5 % aller Menschen über 50 Jahre leiden an peripheren Durchblutungsstörungen, von denen gut 10 % in Gefahr geraten, eine kritische Ischämie der Gliedmaßen (CLI = critical limb ischemia) zu entwickeln. Die Inzidenz der CLI beträgt etwa 500 bis 1000 pro 1 Million Menschen pro Jahr. Etwa 60 % dieser Patienten erhalten einen Gefäßersatz, aber etwa 20 % erleiden das Schicksal einer primären Amputation. Ein Jahr später besitzen nur noch etwa 55 % der Patienten beide unteren Extremitäten, aber schon etwa 25 % haben eine Amputation hinter sich und die restlichen Patienten sind verstorben. Diese kurze Darstellung zeigt in beeindruckender Weise die Notwendigkeit einer frühzeitigen und effektiven medikamentösen Behandlung der peripheren Verschlußkrankheiten auf.

Die bisher bekannten Imidazolsulfonamide sollen hauptsächlich herbizide oder biozide Eigenschaften besitzen (vgl. CA-A-1 222 752 entsprechend EP-A-96 003; EP-A-95 925, EP-A-0 284 277, EP-A-0 298 196 und EP-A-249 938), sich als Textilhilfsmittel oder Weichmacher für Kunststoffe eignen (US-A-3 932 444) oder auch als Carboanhydrase-Hemmer wirken (US-A-2 603 649).

Es wurde nun gefunden, daß eine Reihe neuer Imidazolverbindungen (Imidazolsulfonsäuren und Imidazolsulfonamide) überraschenderweise sehr wertvolle pharmakologische Eigenschaften aufweisen, insbesondere solche, die eine Prophylaxe und Behandlung von Durchblutungsstörungen, speziell von Störungen der Mikrozirkulation und der daraus resultierenden Erkrankungen ermöglichen. Es handelt sich um die Verbindungen der folgenden Formel I; diese Verbindungen und deren physiologisch verträgliche Salze sind daher Gegenstand der vorliegenden Erfindung.

Formel I ist 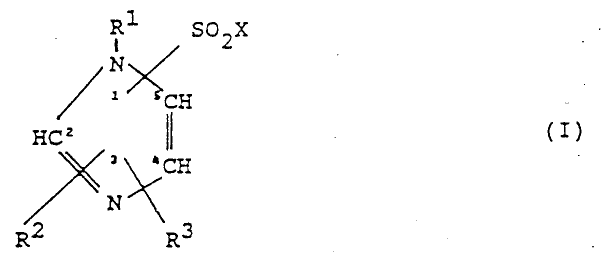
in welcher
- R¹: ist (C₁-C₆)-Alkyl,
- R², R³: sind gleich oder verschieden und jeweils
H,
Halogen (F, Cl, Br, J), vorzugsweise Cl, oder
(C₁-C₃)-Alkyl,
- X: ist OH
oder eine Aminogruppe der Formel II 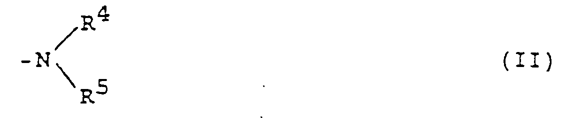 worin
- R⁴: is H oder (C₁-C₇)-, vorzugsweise (C₁-C₄)-Alkyl, ggf. substituiert mit CN, NH₂ oder COOH
- R⁵: ist (C₁-C₈)-, vorzugsweise (C₁-C₅)-Alkylrest,
bei dem - wenn er mehr als 1 C-Atom besitzt - zwischen 2 C-Atomen auch ein Phenylenrest stehen kann und dessen (aliphatische) C-Atome substituiert sind mit 1 oder mehreren der folgenden Gruppen: OH,
(C₁-C₃)-Alkoxy
Phenyl, gegebenenfalls substituiert mit 1-3 OH-, (C₁-C₃)-Alkoxygruppen, (C₁-C₃)-Alkoxy-COOH, und/oder (C₁-C₃)-Alkoxy-COO(C₁-C₄)-alkyl
COOH,
COO(C₁-C₃)-Alkyl,
CONH₂,
CN,
(C₂-C₅)-Alkinyl,
NH₂, 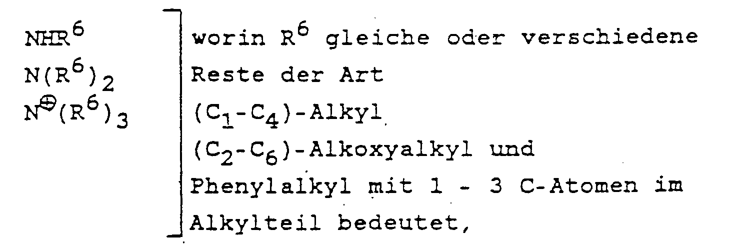 NH-CO-(C₁-C₆)-Alkyl, 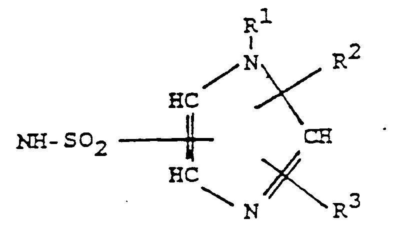 (Bedeutung von R¹, R² und R³ wie vorstehend), monocyclische 5- bis 7-gliedrige gesättigte oder ungesättigte heterocyclische Reste mit 1 N-Atom sowie gegebenenfalls noch einem zusätzlichen N-, O- oder S-Atom im Ring aus der Gruppe Thiomorpholin, Piperazin, Morpholin, Imidazol, Pyrrolidin und Piperidin,
gegebenenfalls
substituiert mit (C₁-C₃)-Alkyl,
Phenyl,
Phenylalkyl mit 1 - 3 C-Atomen im Alkylteil, OH, und/oder
Oxo (= O), einschließlich der offenen und cyclischen Ketalformen mit 2 - 6 C-Atomen im Ketalteil,
und worin das Ring-S-Atom - falls vorhanden - auch zur Sulfoxid (SO)- oder Sulfon (SO₂)-Form oxidiert sein kann,
oder worin
R⁴ und R⁵ zusammen mit dem Amid-N-Atom, an das sie gebunden sind, einen 5- bis 7-gliedrigen heterocyclischen Ring aus der Gruppe Piperazin und Thiomorpholin bilden
und der heterocyclische Ring ansonsten substituiert sein kann mit folgenden Gruppen:
(C₁-C₃)-Alkoxy,
Phenylalkyl mit 1 - 4 C-Atomen im Alkylteil,
Phenyl, gegebenenfalls substituiert mit 1 oder mehreren - vorzugsweise nur 1 - der Gruppen:
(C₁-C₃)-Alkyl,
OH,
(C₁-C₃)-Alkoxy,
(C₁-C₃)-Alkoxy-COOH,
(C₁-C₃)-Alkoxy-COO(C₁-C₄)Alkyl, 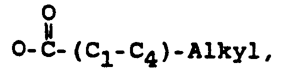
O-SO₂-C₆H₅
O-SO₂-C₆H₄CH₃, 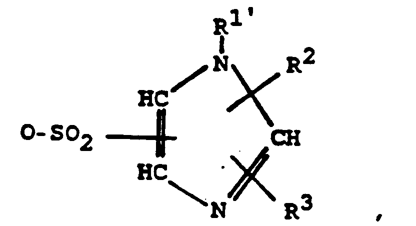
worin R^{1'} die gleiche Bedeutung wie R¹ besitzt und zusätzlich noch = H sein kann, und
R² und R³ die vorerwähnte Bedeutung besitzen,
und das Ring-S-Atom - falls vorhanden - auch zur Sulfoxid (SO)- oder Sulfon (SO₂)-Form oxidiert sein kann.

Bevorzugte Verbindungen der Formel I sind solche, bei denen mindestens eines der folgenden Merkmale vorliegt:
a) R¹ = CH₃ oder C₂H₅
b) R², R³ = gleich oder verschieden und jeweils H, Cl oder CH₃ und
c) der -SO₂X-Rest sich in 2- oder 4-Stellung des Imidazolrings befindet.

Weiterhin sind unter den Verbindungen der Formel I die Sulfonamide bevorzugt; d.s. also die Verbindungen mit 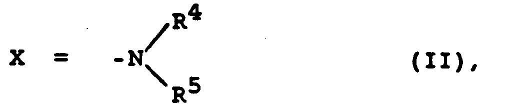
wobei die Reste R⁴ und R⁵ bevorzugt folgende Bedeutung besitzen:
- R⁴ =: H und
- R⁵ =: (C₂-C₅)-Alkylrest, in welchem gegebenenfalls zwischen 2 C-Atomen ein Phenylenrest steht und dessen (aliphatische) C-Atome mit insgesamt 1 oder 2 - vorzugsweise nur mit 1 - der folgenden Gruppen substituiert sind:
Hydroxyphenyl C₆H₄OH
CN
(C₂-C₃)-Alkinyl
NH₂ 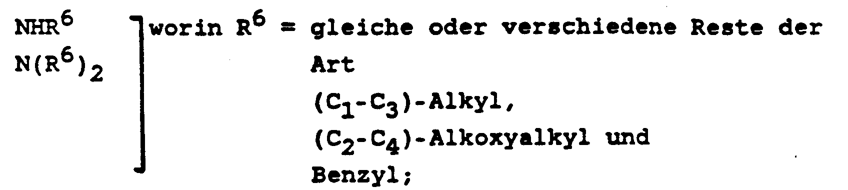 monocyclischer 5- bis 6-gliedriger gesättigter heterocyclischer Rest aus der Gruppe: 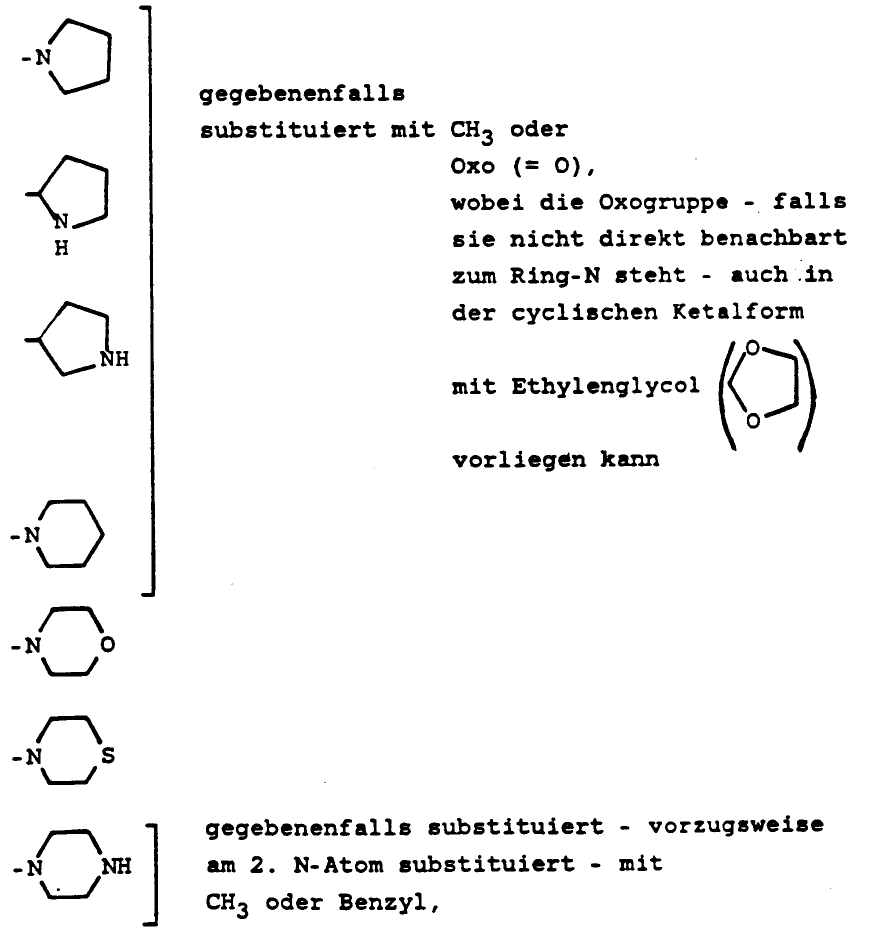 oder R⁴ und R⁵ zusammen mit dem Amid-N-Atom, an das sie gebunden sind, einen gesättigten 6-gliedrigen heterocyclischen Ring bilden der Art 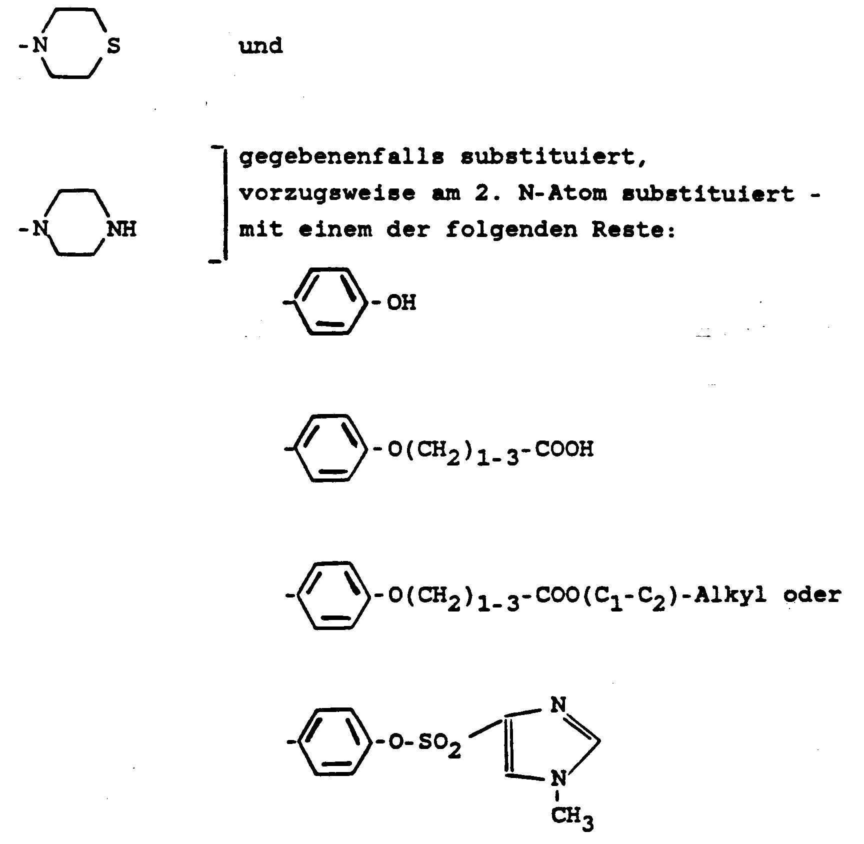
Unter den Sulfonamiden sind wiederum diejenigen mit separaten Resten R⁴ und R⁵ gegenüber denjenigen mit - zusammen mit dem Amid-N - zu einem Ring geschlossenen R⁴ + R⁵ etwas bevorzugt.

Besonders bevorzugte Verbindungen der Formel I sind
N-(2-Morpholinoethyl)-1-methyl-2-imidazolsulfonsäureamid = Verbindung der Formel I, worin
R¹ = CH₃,
R² = R³ = H,
SO₂X-Gruppe in 2-Position und 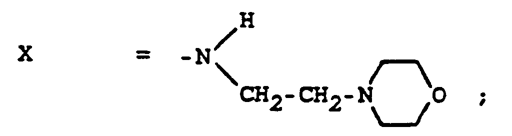
sowie N-(3-Morpholinopropyl)-1-methyl-4-imidazolsulfonsäureamid = Verbindung der Formel I, worin
R¹ = CH₃ ,
R² = R³ = H,
SO₂X-Gruppe in 4-Position und 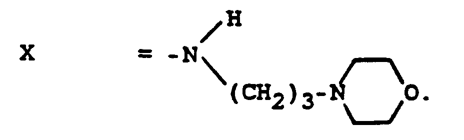
Als physiologisch verträgliche Salze kommen z.B. in Frage:
- wenn in den Verbindungen der Formel I saure Gruppen vorhanden sind (insbesondere wenn X = OH):
   Na-, K-, NH₄-Salze etc.;
- wenn in den Verbindungen der Formel I basische Gruppen vorhanden sind:
   Hydrochloride, Salze mit physiologisch verträglichen organischen Säuren (Essigsäure, Maleinsäure, Fumarsäure etc.), etc.

Einige beispielhafte erfindungsgemäße Verbindungen der Formel I - sowohl nicht besonders bevorzugte als auch bevorzugte - sind:
1-Methyl-4-imidazolsulfonsäure,
1-Ethyl-4-imidazolsulfonsäure,
1-Methyl-2-imidazolsulfonsäure,
5-Chlor-1-methyl-4-imidazolsulfonsäure,
2-Fluor-1-methyl-4-imidazolsulfonsäure,
4-Chlor-1-methyl-5-imidazolsulfonsäure,
1-Methyl-5-imidazolsulfonsäure,
1,2-Dimethyl-5-imidazolsulfonsäure,
N-(3-Morpholinopropyl)-1-methyl-4-imidazolsulfonsäureamid,
N-(2-Morpholinoethyl)-1-methyl-4-imidazolsulfonsäureamid,
N-(4-Morpholinobutyl)-1-methyl-4-imidazolsulfonsäureamid,
N-(5-Morpholinopentyl)-1-methyl-4-imidazolsulfonsäureamid,
N-(3-Morpholino-2-methyl-1-propyl)-1-methyl-4-imidazolsulfonsäureamid,
N-(3-Thiomorpholinopropyl)-1-methyl-4-imidazolsulfonsäureamid,
N-Butyl-N-(3-Morpholino-1-propyl)-1-methyl-4-imidazolsulfonsäureamid,
N-(2-Piperidinoethyl)-1-methyl -4-imidazolsulfonsäureamid,
N-[3-(2-Methylpiperidino)-propyl]-1-methyl-4-imidazolsulfonsäureamid,
N-(5-Piperidinopentyl)-1-methyl-4-imidazolsulfonsäureamid,
N-[8-Aza-1,4-dioxa-spiro(4,5)decyl]-1-methyl-4-imidazolsulfonsäureamid,
N-(2-Pyrrolidionoethyl)-1-methyl-4-imidazolsulfonsäureamid,
N-[2-(1-Methyl-2-pyrrolidinyl)-ethyl]-1-methyl-4-imidazolsulfonsäureamid,
N<3[Bis-(2-methoxyethyl)-amino]-propyl>-4-imidazolsulfonsäureamid,
N-[4-(4-Hydroxyphenyl)-piperazino]-1-methyl-4-imidazolsulfonsäureamid,
N-[3-(4-Benzyl-1-piperazinyl)-propyl]-1-methyl-4-imidazolsulfonsäureamid,
N-[3-(4-Methylpiperazino)-propyl]-1-methyl-4-imidazolsulfonsäureamid,
N-[3-(N-Benzyl-N-methylamino)-1-propyl]-1-methyl-4-imidazolsulfonsäureamid,
N-(3-Morpholinopropyl)-1-methyl-2-imidazolsulfonsäureamid,
N-(2-Morpholinoethyl)-1-methyl-2-imidazolsulfonsäureamid,
N-(3-Morpholinopropyl)-4-chlor-1-methyl-5-imidazolsulfonsäureamid,
N-(3-Morpholinopropyl)-5-chlor-1-methyl-4-imidazolsulfonsäureamid,
N-(3-Morpholinopropyl)-1,2-dimethyl-4-imidazolsulfonsäureamid,
N-(5-Morpholino-1-pentyl)-5-chlor-1-methyl-4-imidazolsulfonsäureamid,
N-(3-Morpholinopropyl)-1-propyl-4-imidazolsulfonsäureamid,
N-(3-Morpholinopropyl)-1-propyl-5-imidazolsulfonsäureamid,
N-(3-Morpholinopropyl)-1-n-butyl-4-imidazolsulfonsäureamid,
N-(3-Morpholinopropyl)-1-n-butyl-5-imidazolsulfonsäureamid,
N-(3-Morpholinopropyl)-1-ethyl-4-imidazolsulfonsäureamid,
N-(1,3-Dimorpholino-2-propyl)-1-methyl-4-imidazolsulfonsäureamid,
N-[4-(4-Hydroxyphenyl)-piperazino]-5-chlor-1-methyl-4-imidazolsulfonsäuramid,
1-(1-Methyl-5-chlor-4-imidazolsulfonyl)-4-[4-(1-methyl-5-chlor-4-imidazolsulfonyloxy)-phenyl]-piperazin,
4-(1-Methyl-4-imidazolsulfonyl)-tetrahydro-4H-1,4-thiazin,
1-[3-(1-Methyl-5-imidazolsulfonyl)-aminopropyl]-2-pyrrolidinon,
N-(3-Methoxypropyl)-1-methyl-4-imidazolsulfonsäureamid,
N-(4-Hydroxyphenethyl)-1-methyl-4-imidazolsulfonsäureamid,
N-(4-Hydroxyphenethyl)-5-chlor-1-methyl-4-imidazolsulfonsäureamid,
1,6-Bis-(5-chlor-1-methyl-4-imidazolsulfonamido)-hexan,
N-(2-Cyanoethyl)-1-methyl-4-imidazolsulfonsäureamid,
N-(5-Cyanopentyl)-1-methyl-4-imidazolsulfonsäureamid,
N-(3-Propargyl)-1-methyl-4-imidazolsulfonsäureamid,
4-[2-(1-Methyl-4-imidazolsulfonyl)-aminoethyl]-phenoxy-essigsäure,
4-[2-(5-Chlor-1-methyl-4-imidazolsulfonyl)-aminoethyl]-phenoxy-essigsäure,
N-(3-Morpholino-1-propyl)-1-methyl-5-imidazolsulfonsäureamid,
1,6-Bis-(1-methyl-4-imidazolsulfonamido)-hexan,
N-[3-(1-Piperazinyl)-propyl]-1-methyl-4-imidazolsulfonsäureamid,
4-Methyl-4-[3-(1-methyl-4-imidazolsulfamoyl)-1-propyl]-morpholiniumjodid,
4-(1-Methyl-4-imidazolsulfonyl)-tetrahydro-4H-1,4-thiazin-1,1-dioxid,
4-[4-(5-Chlor-1-methyl-4-imidazolsulfonyl)-piperazin-1-yl]-phenoxyessigsäureethylester,
N-(6-Aminohexyl)-1-methyl-4-imidazolsulfons äureamid,
N-(3-Aminopropyl)-1-methyl-4-imidazolsulfonsäureamid,
N(3-Thiomorpholinopropyl)-1-methyl-4-imidazolsulfonsäureamid-S-oxid,
N-(3-Methylamino-1-propyl)-1-methyl-4-imidazolsulfonsäureamid,
N-[4-(Morpholinomethyl)-benzyl]-1-methyl-4-imidazolsulfonsäureamid,
N-(3-Dibenzylaminopropyl)-1-methyl-4-imidazolsulfonsäureamid,
N-(3-Dimethylaminopropyl)-1-methyl-4-imidazolsulfonsäureamid,
N-(3-N -Ethyl-N -isopropylaminopropyl)-1-methyl-4-imidazolsulfonsäureamid,
N-[Bis-(2-cyanoethyl)]-1-methyl-4-imidazol-sulfonsäureamid,
N-[2-(2-Pyridyl)-ethyl]-1-methyl-4-imidazolsulfonsäureamid,
N-(5-Carboxypentyl)-1-methyl-4-imidazolsulfonsäureamid und
N-(5-Acetylpentyl)-1-methyl-4-imidazolsulfonsäureamid.

Die Verbindungen der Formel I und ihre physiologisch verträglichen Salze werden erfindungsgemäß dadurch hergestellt, daß man
a) ein Imidazolderivat der allgemeinen Formel III 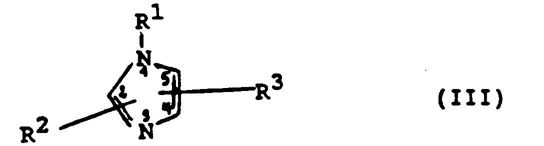 in der R¹, R² und R³ die bei Formel I genannte Bedeutung haben, und eine der Stellungen 4 oder 5 unsubstituiert ist, durch Sulfonierung mittels Schwefelsäure oder Oleum, bevorzugt bei Temperaturen von etwa 150 - 180°C in die erfindungsgemäßen Verbindungen der Formel Ia umwandelt, 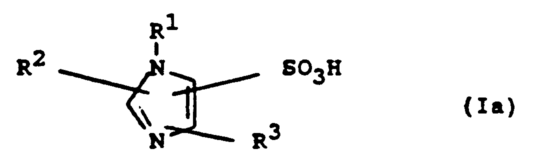 worin R¹, R² und R³ ebenfalls die bei Formel I genannte Bedeutung besitzen, oder daß man
b) ein Imidazolderivat der allgemeinen Formel IV, 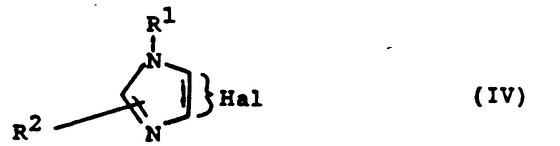 in der R¹ und R² die bei Formel I genannte Bedeutung haben, eine der Stellungen 4 oder 5 ein Halogenatom (Cl, Br, J) trägt und die andere unsubstituiert ist, durch Sulfonierung und anschließende hydrogenolytische Enthalogenierung mittels Edelmetallkatalysatoren, bevorzugt bei Wasserstoffdrucken von etwa 1 - 5 bar in polaren Lösungsmitteln wie Alkohol und/oder Wasser und Raumtemperatur bis etwa 60°C in eine Verbindung der Formel Ib umwandelt 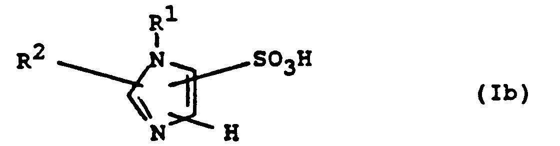 (Bedeutung von R¹ und R² die bei Formel I) - der vorübergehende Schutz der 4- oder 5-Position verhindert hier also die Sulfonierung an dieser Stelle und führt somit zu einheitlichen Produkten - oder daß man
c) ein Imidazolderivat der allgemeinen Formel V, 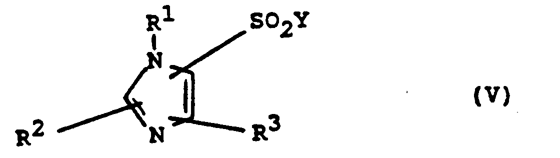 in der R¹, R² und R³ die bei Formel I genannte Bedeutung haben und Y für Halogen, vorzugsweise Chlor, steht, zu den erfindungsgemäßen Sulfonsäuren der Formel Ia mit den dort genannten Bedeutungen für R¹, R² und R³ - bevorzugt mittels Wasser bei Raumtemperatur - hydrolysiert, oder daß man
d) ein Imidazolderivat der allgemeinen Formel VI oder VI', 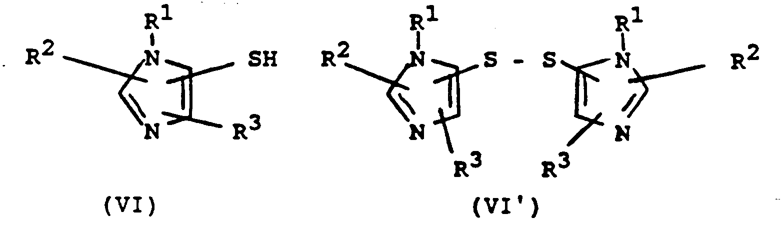 worin R¹, R² und R³ die bei Formel I genannte Bedeutung haben, zu der entsprechenden Imidazolsulfonsäure (Formel Ia) oxidiert,
   vorzugsweise, indem man nach dem Verfahren gemäß EP-A-95 925 mit Chlor zu einem intermediären Imidazolsulfonsäurechlorid oxidiert und direkt anschließend im wäßrigen Medium hydrolysieren läßt,
   oder daß man
e) ein Imidazolsulfonsäurehalogenid der allgemeinen Formel V (s. Variante c), mit einem Amin der Formel H-II, 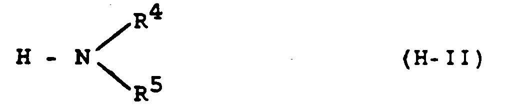 in der R⁴ und R⁵ die gleiche Bedeutung wie in Formel II haben, zu den erfindungsgemäßen Sulfonamiden der Formel Ic umsetzt, 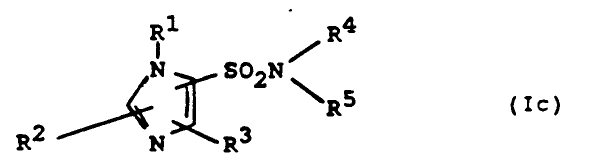 worin R¹ bis R⁵ die bei den Formeln I und II genannten Bedeutungen besitzen.

Diese Reaktion kann einerseits in Abwesenheit von zusätzlichen Säurefängern durchgeführt werden, wobei beim Umsatz von Di- und Poly-aminen die entsprechenden Hydrochloride entstehen, die entweder als solche isoliert oder in die freien Basen übergeführt werden können, welche wiederum entweder als solche isoliert oder in andere Salze, beispielsweise die der Fumarsäure, überführt werden können.

Andererseits kann die Reaktion auch in Gegenwart von Säurefängern, etwa einem Überschuß des umzusetzenden Amins H-N(R⁴)R⁵ (Formel H-II), einem niederen tertiären Amin wie Triethylamin, oder anorganischen Basen wie Kaliumcarbonat erfolgen.

Die Sulfonamidbildung kann in einem wasserfreien, gegenüber den Reaktionspartnern inerten Lösungsmittel, bevorzugt Acetonitril oder Dichlormethan, bei geeigneter Arbeitsweise jedoch auch in protischen Lösungsmitteln, beispielsweise Wasser oder Phenol, jeweils bei Temperaturen zwischen etwa -30°C und den Siedetemperaturen des angewandten Lösungsmittels, vorzugsweise jedoch zwischen etwa 0°C und 30°C, durchgeführt werden.

Die bei diesen Verfahrensvarianten als Ausgangsstoffe verwendeten Amine der Formel H-II sind größtenteils bekannt oder lassen sich nach literaturbekannten Methoden herstellen, überwiegend durch Hydrierung oder Reduktion entsprechend substituierter Nitrile. In Fällen, in denen die Hydrierung nicht anwendbar ist, kann beispielsweise auf die Phthalimid-Methode zurückgegriffen werden. So sei als Beispiel angeführt, daß N-(3-Brompropyl)-phthalimid mit Thiomorpholin zum N-(3-Thiomorpholinopropyl)-phthalimid reagiert, welches durch Hydrazin und anschließende Salzsäure-Einwirkung in das Hydrochlorid des N-(3-Aminopropyl)-thiomorpholins überführt werden kann.

Weiterhin werden die Verbindungen der Formel I und ihre physiologisch verträglichen Salze erfindungsgemäß dadurch hergestellt, daß man
f) Imidazolderivate der allgemeinen Formel VII, 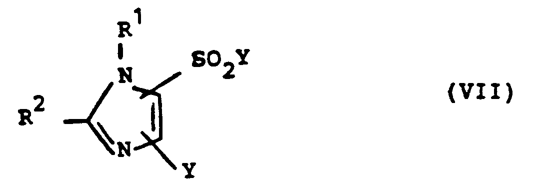 worin R¹ und R² die bei Formel I angegebene Bedeutung besitzen und Y gleiche oder verschiedene Halogenatome (Cl, Br, J) bedeutet, mit Aminen der Formel H-II umsetzt und anschließend der hydrogenolytischen Enthalogenierung, vorzugsweise über Edelmetallkatalysatoren, wie Palladium auf Kohle, unterwirft, um die in 4- oder 5-Stellung unsubstituierten Sulfonamide der Formel Id zu erhalten 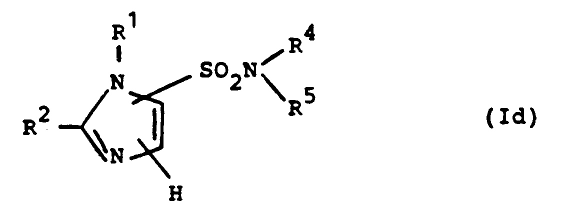 (Bedeutung von R¹, R², R⁴ und R⁵ wie in Formeln I und II), oder daß man
g) ein Imidazolsulfonylhalogenid der allgemeinen Formel V (siehe Variante c) mit einem Trialkylsilylamin der Formel VIII, 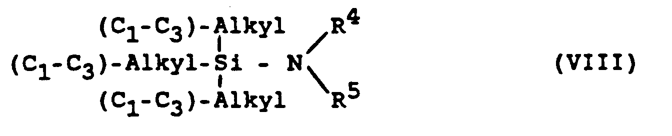 wobei R⁴ und R⁵ die bei Formel II genannte Bedeutung haben und bevorzugter(C₁-C₃)-Alkylrest der Methylrest ist, zur Umsetzung bringt, um die erfindungsgemäßen Sulfonamide der Formel Ic (siehe Variante e) zu erhalten.
   Die silylierten Amine können entweder als reine Verbindungen oder als frisch - z.B. mittels MSTFA (N-Methyl-N-trimethylsilyltrifluoracetamid) bereitete Rohprodukte zum Einsatz kommen. Die Reaktion mit dem jeweiligen Imidazolsulfonylhalogenid V erfolgt gewöhnlich in inerten Lösungsmitteln, wie Dichlormethan oder Acetonitril, bei Temperaturen zwischen etwa -30 bis etwa 120°C, vorzugsweise zwischen etwa -30°C und dem Siedepunkt des Lösungsmittels. Dieses Verfahren liefert direkt die freie Base und ist außerdem angezeigt bei weniger reaktiven und empfindlichen Verbindungen.
h) Ferner können erfindungsgemäße Verbindungen gemäß Formel I (mit X = -N(R⁴)R⁵, wobei R⁴ wenigstens eine NH₂-Gruppe und/oder R⁵ mindestens eine primäre oder sekundäre Amino-Gruppe trägt) und ihre physiologisch verträglichen Salze dadurch dargestellt werden, daß Amine der Formel H-N(R⁴)R⁵, die an ihren Resten R⁴ und/oder R⁵ mindestens eine N-geschützte - bevorzugt N-benzylierte - entsprechende Amino-Gruppe tragen, mit den Imidazolsulfonylhalogeniden der Formel V (siehe Variante c), wie unter e) beschrieben, umgesetzt und die entstandenen Sulfonamide anschließend von der bzw. den Schutzgruppe(n) befreit werden, im Falle der N-Benzyl-Schutzgruppe bevorzugt durch Hydrogenolyse bei niedrigen Wasserstoffdrucken (etwa 1 - 5 bar), wenig erhöhten Temperaturen (Raumtemperatur bis etwa 60°C) und in ethanolisch-wäßriger Ammoniaklösung über Edelmetallkatalysatoren, wie Palladium auf Kohle.
   Aus der Anzahl der Schutzgruppen, die sich für den Schutz der zweiten Aminogruppe vor dem Angriff eines Imidazolsulfonylhalogenids eignen und sich anschließend wieder entfernen lassen, seien - außer der bereits genannten bevorzugten Benzylgruppe - noch folgende hervorgehoben:
   Triphenylmethyl-, Trifluoracetyl-, Benzyloxycarbonyl-, tert.-Butyloxycarbonyl-, Phthalyl-, Formyl- und Acetyl-.
i) Weiterhin lassen sich solche Imidazolderivate der Formel I mit X = -N(R⁴)R⁵, wobei wenigstens einer der Reste R⁴ und R⁵ eine oder mehrere primäre Aminogruppen trägt, dadurch herstellen, daß man Imidazolsulfonylhalogenide der Formel V (siehe Variante c) mit entsprechenden Aminonitrilen analog der Verfahrensweise gemäß Variante e) umsetzt und die so erhaltenen Imidazolsulfonylaminonitrile zu den entsprechenden Aminoverbindungen reduziert, bevorzugt durch katalytische Hydrierung unter Verwendung von Edelmetallkatalysatoren wie Palladium auf Kohle in alkoholisch-ammoniakalischer Lösung bei erhöhtem Wasserstoffdruck (etwa 2 - 5 bar) bei Raumtemperatur bis leicht erhöhter Temperatur (bis etwa 60°C).
j) Außerdem lassen sich Imidazolderivate der Formel I mit X = -N(R⁴)R⁵, wobei R⁵ Träger einer quartären Aminogruppe -N^{⊕}(R⁶)₃ ist, in der die Reste R⁶ gleich oder verschieden sein können, wie folgt darstellen:
   Die Verbindungen der Formel I mit tertiären Aminogruppen -N(R⁶)₂ als Substituent von R⁵ werden mittels eines Alkylierungsmittels wie eines Alkylhalogenids, bevorzugt Jodmethan, eines Schwefelsäureesters, bevorzugt Dimethylsulfat oder eines Arylsulfonsäureesters, bevorzugt p-Toluolsulfonsäuremethylester, in Lösungsmitteln wie Nitromethan, Acetonitril, Alkoholen oder wäßrig-alkoholischen Lösungen, bevorzugt im Bereich von Raumtemperatur bis zur Siedetemperatur des Lösungsmittels, quarternisiert.
k) Weitere erfindungsgemäße Substanzen lassen sich herstellen, indem man Imidazolderivate der allgemeinen Formel I mit X = -N(R⁴)R⁵, wobei R⁵ oder R⁴ + R⁵ zusammen Träger mindestens einer Sulfidgruppe, bevorzugt in Form eines Thiomorpholinringes, sind, zu den entsprechenden Sulfoxiden oder Sulfonen oxidiert. Geeignete Oxidationsmittel dafür sind Natriumjodat in wäßrig-methanolischer Lösung oder Peroxide wie m-Chlorper oxybenzoesäure, Peressigsäure oder Wasserstoffperoxid in Lösungsmitteln wie Chloroform oder Essigsäure bzw. Wasser.
l) Unter den Verbindungen der Formel I lassen sich einige dadurch synthetisieren, daß man Imidazolderivate der allgemeinen Formel I mit X = -N(R⁴)R⁵, wobei R⁵ oder R⁴ + R⁵ zusammen mindestens einen Arylrest tragen, der mit einer oder mehreren phenolischen Hydroxylgruppen substituiert ist, mit Alkylierungsreagenzien, bevorzugt ω-Halogenfettsäurederivaten, zu den entsprechenden Phenolethern alkyliert in Gegenwart von basischen Verbindungen, wie Natriumhydroxid, in einem polaren Lösungsmittel, wie Ethanol, im Temperaturbereich von etwa 0°C bis zum Siedepunkt des Lösungsmittels.
   Sind die Produkte Derivate der ω-Fettsäureester, können diese weiterhin noch der sauren oder alkalischen Hydrolyse unter Standardbedingungen oder der Aminolyse mit Ammoniak-Lösungen oder Lösungen von niederen primären oder sekundären Aminen, bevorzugt Methylamin, unterworfen werden, um die entsprechenden Carbonsäuren bzw. Carbonsäureamide zu ergeben.
m) Diese unter l) genannten phenolischen Imidazolderivate lassen sich auch mit Acylierungsagenzien wie Alkylcarbonsäurechloriden, vorzugsweise denen der Essig-, Propion- oder Buttersäure, mit Arylsulfonsäurechloriden, vorzugsweise Benzol- oder Toluolsulfonsäurechlorid, und mit Imidazolsulfonsäurehalogeniden der allgemeinen Formel V (siehe Variante c), in der R¹ zusätzlich noch Wasserstoff bedeuten kann, zu den entsprechenden Phenolestern umsetzen. Zweckmäßig sind hierbei basische wasserfreie Bedingungen.

Die Verbindungen der Formel I und deren physiologisch verträgliche Salze eignen sich aufgrund ihrer wertvollen pharmakologischen Eigenschaften sehr gut zur Anwendung als Heilmittel.

Erfindungsgegenstand sind daher auch Arzneimittel mit einem Gehalt an mindestens einer Verbindung der Formel I und/oder mindestens einem von deren physiologisch verträglichen Salzen. Die Arzneimittel eignen sich vorzugsweise zur Prophylaxe und/oder Therapie von Durchblutungsstörungen, insbesondere von Störungen der Mikrozirkulation sowie der daraus resultierenden Erkrankungen.

Die aus Durchblutungsstörungen, insbesondere aus Störungen der Mikrozirkulation resultierenden Erkrankungen sind hauptsächlich ischämische Skelett- und/oder Herzmuskelerkrankungen, insbesondere Claudicatio intermittens, Ulcus cruris sowie degenerative und/oder entzündliche Muskelerkrankungen verschiedener Genese mit oder ohne Muskelatrophie, Vasculitiden mit thrombotischen Ereignissen, arterielle und venöse Blutgerinnsel (z.B. Thrombosen, Schock).

Wegen der durchblutungsfördernden Wirkung der erfindungsgemäßen Verbindungen und Arzneimittel, insbesondere im Mikrobereich, sind die Verbindungen und Arzneimittel auch wirksam bei Arteriosklerose, bei der Operationsnachbehandlung zur Verhinderung postoperativer Thrombosen, zur Nachbehandlung von Krebs zwecks Verhinderung bzw. Verminderung der Metastasierung, bei der Behandlung von Patienten, die an Herz-Lungen-Maschinen oder die Nierendialyse angeschlossen sind und schließlich auch von Patienten nach Schlaganfall oder Herzinfarkt
sowie zur Wundheilung nach Traumen und exogenen Noxen.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granula, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Gescbmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung der Formel I und/oder mindestens eines entsprechenden physiologisch verträglichen Salzes enthält. Bei festen Dosierungseinheiten wie Tabletten, Kapseln und Suppositorien, kann diese Dosis bis zu etwa 500 mg, bevorzugt jedoch etwa 50 bis 300 mg, und bei Injektionslösungen in Ampullenform bis zu etwa 150 mg, vorzugsweise aber etwa 10 bis 100 mg, betragen. Zwischen den Dosen der Verbindungen der Formel I und von deren Salzen bestehen dabei nur geringe Unterschiede.

Für die Behandlung eines erwachsenen Patienten sind - je nach Wirksamkeit der Verbindungen gemäß Formel I am Menschen - Tagesdosen von etwa 20 bis 500 mg Wirkstoff, vorzugsweise etwa 50 bis 300 mg, bei oraler Verabreichung und von etwa 5 bis 300 mg, bevorzugt etwa 10 bis 100 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die erfindungsgemäßen Arzneimittel werden dadurch hergestellt, daß man mindestens eine Verbindung der Formel I und/oder mindestens eines von deren physiologisch verträglichen Salzen mit üblichen Träger- sowie gegebenenfalls Zusatz- und/oder Hilfsstoffen in die bzw. eine geeignete Darreichungsform bringt.

Die erfindungsgemäßen Arzneimittel können bei der Herstellung der vorgenannten galenischen Zubereitungsformen auch zusammen mit anderen geeigneten Wirkstoffen, beispielsweise Antithrombotika, Antihyperlipidämika, Analgetika, Sedativa, Antidepressiva, antianginösen Mitteln, Cardiotonika, Antiarrhytmika, Diuretika, Antihypertensiva einschließlich β-Rezeptoren- und Calcium-Blockern, Plasmaexpandern und anderen Vasotherapeutika, formuliert werden.

Schließlich sind auch einige Vor- bzw. Zwischenprodukte zur Herstellung der Verbindungen der Formel I neu und daher ebenfalls Erfindungsgegenstand; es handelt sich um die Verbindungen
1-Methyl-,
1,2-Dimethyl- und
1-Ethyl-4-imidazolsulfonsäurechlorid.

Diese Verbindungen werden vorteilhaft dadurch hergestellt, daß man
a) 1-Methyl- bzw. 1,2-Dimethyl- bzw. 1-Ethyl-imidazol mit Chlorsulfonsäure ClSO₃H, gegebenenfalls unter nachträglichem Zusatz von SOCl₂, zur Reaktion bringt, oder daß man
b) 1-Methyl- bzw. 1,2-Dimethyl- bzw. 1-Ethyl-4-mercaptoimidazol mit Cl₂ oxidierend chloriert.

Nähere Erläuterung der beiden Verfahrensvarianten:
a) Die Umsetzung mit Chlorsulfonsäure erfolgt zweckmäßig bei erhöhter Temperatur, bevorzugt zwischen etwa 130°C und 160°C, möglichst ohne den entstehenden Chlorwasserstoff abzusaugen.
   Die etwaige anschließende Thionylchloridzugabe wird zur besseren Reaktionskontrolle bei leicht erhöhten Temperaturen, bevorzugt zwischen etwa 60 und 80°C, vorgenommen, bei denen das Reaktionsgemisch gut rührbar geworden ist und ein schnelles Abreagieren des Thionylchlorids gewährleistet ist. Durch Aufgießen des Reaktionsgemisches auf eine Eis-Wasser-Mischung läßt sich für diese Imidazolderivate erreichen, daß sich fast reine 1-Alkyl-4-imidazolsulfonsäurechloride abscheiden, während mit entstandene 5-Imidazolsulfonsäurechloride überwiegend in Lösung bleiben und zu Sulfonsäuren hydrolysieren. Zur Vermeidung von Verlusten durch Hydrolyse auch der 4-Imidazolsulfonsäurechloride empfiehlt sich eine schnelle Trocknung, bevorzugt in Lösungsmitteln wie Dichlormethan, mit Trockenmitteln wie Natriumsulfat.
b) 2-Mercaptoimidazole können nach literaturbekannten Methoden mit Chlor, möglichst in stöchiometrischer Anwendung, zu den 2-Imidazolsulfonylchloriden oxidiert werden [R.G. Jones et al., J. Am. Chem. Soc. 71, 4000 (1949)]. Die Bedingungen für die Chloroxidation der 1-Alkyl- sowie 1,2-Dialkyl-4-mercaptoimidazole sind ähnlich (bei etwa -10 bis +10°C in verdünnter Salzsäure).

Die folgenden (Herstellungs-)Beispiele sollen der näheren Erläuterung der Erfindung dienen.

Die Strukturen aller nachstehend beschriebenen Verbindungen wurden durch Elementaranalyse und IR- sowie ¹H-NMR-Spektren gesichert. Im folgenden wird unter Vakuum das der Wasserstrahlpumpe verstanden. Zur Dünnschichtchromatographie wurden Kieselgelplatten (Kieselgel 60F₂₅₄ spezial 0,25 mm, Riedel-de Haen AG, D-3016 Seelze) verwendet.

Die angegebenen Ausbeuten sind nicht optimiert.

Nach den Herstellungsbeispielen folgt dann noch ein pharmakologischer Teil, aus welchem die Wirksamkeit der erfindungsgemäßen Verbindungen hervorgeht; der pharmakologische Teil enthält auch Vergleichswerte gegenüber dem Standardtherapeutikum Pentoxifyllin (= 1-(5-Oxohexyl)-3,7-dimethyl-xanthin).

### (Herstellungs-)Beispiele

### A) Verbindungen der Formel I mit X = OH

### Beispiel 1

### 5-Chlor-1-methyl-4-imidazolsulfonsäure

33 g (0,28 mol) 5-Chlor-1-methylimidazol werden in 200 ml rauchender Schwefelsäure 4 Stunden auf 160 - 180°C erhitzt. Nach dem Erkalten wird das Reaktionsgemisch vorsichtig auf Eis gegeben. Aus der kalten wäßrigen Lösung (etwa 1,5 l) kristallisiert das Produkt aus. Nach zweimaligem Umkristallisieren aus Wasser wird die Titelverbindung in Form grober gelblicher Kristalle mit dem Schmelzpunkt 309 - 310°C erhalten.
Ausbeute: 34 g (46,9 % der Theorie).

### Beispiel 2

### 1-Ethyl-4-imidazolsulfonsäure

5,6 g (29 mmol) 1-Ethyl-4-imidazolsulfonsäurechlorid aus Beispiel C-2 werden bei Raumtemperatur in 70 ml Wasser suspendiert, bis eine klare Lösung entstanden ist. Nach dem Eindampfen im Vakuum wird der Rückstand aus Ethanol/Wasser umkristallisiert, um die Titelverbindung mit dem Schmelzpunkt 278°C zu ergeben. Ausbeute: 5 g (99 % d.Th.)

### Beispiel 3

### 1-Methyl-4-imidazolsulfonsäure

In analoger Weise wie in Beispiel 2 beschrieben erhält man aus 1-Methyl-4-imidazolsulfonsäurechlorid aus Beispiel C-1 in etwa 70 %iger Ausbeute die Titelverbindung mit dem Schmelzpunkt 288 - 289°C, nach Umkristallisieren aus Ethanol/Methanol.

### Beispiel 4

### 1-Methyl-2-imidazolsulfonsäure

In analoger Weise wie in Beispiel 2 beschrieben, erhält man aus 1-Methyl-2-imidazolsulfonsäurechlorid [R.O. Roblin, jr. und J.W. Clapp J. Am. Chem. Soc. 72, 4890 (1950)] in etwa 72 %iger Ausbeute die Titelverbindung mit dem Schmelzpunkt 234 - 236°C, nach Umkristallisation aus Ethanol/Methanol.

### Beispiel 5

### 4-Chlor-1-methyl-5-imidazolsulfonsäure

In analoger Weise wie in Beispiel 2 beschrieben, erhält man aus 4-Chlor-1-methyl-5-imidazolsulfonsäurechlorid [M.H. Fisher, W.H. Nicholson und R.S. Stuart, Can. J. Chem. 39, 1336 (1961)] in etwa 39 %iger Ausbeute die Titelverbindung mit dem Schmelzpunkt 260 - 261°C.

### Beispiel 6

### 1-Methyl-5-imidazolsulfonsäure

Eine Lösung von 3,1 g (17 mmol) 1-Methyl-4-chlor-5-imidazolsulfonsäure aus Beispiel 5 in 100 ml Wasser wird in Gegenwart von 0,3 g Pd/C-Katalysator unter Schütteln bei einem Anfangsdruck von 3,45 bar Wasserstoff bei 25°C bis zur Druckkonstanz hydriert. Der nach Abfiltrieren des Katalysators und Abdampfen des Wassers im Vakuum verbleibende kristalline Rückstand wird aus Ethanol umkristallisiert, um die Titelverbindung mit dem Schmelzpunkt 286 - 287°C zu ergeben.
Ausbeute: 1,5 g (58,5 % d.Th.)

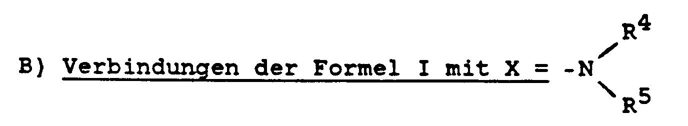

### Beispiel 7

### N-(3-Moryholinopropyl)-1-methyl-4-imidazolsulfonsäureamid (Hydrochlorid)

Zu einer Lösung von 24 ml (0,17 mol) 3-Morpholinopropylamin-1 in 50 ml Acetonitril (bzw Dichlormethan) tropft man eine Lösung von 30 g (0,17 mol) 1-Methylimidazol-4-sulfonsäurechlorid aus Beispiel C-1 in 150 ml Acetonitril. Durch äußere Kühlung mit Eiswasser wird die Temperatur des Reaktionsgemisches auf Raumtemperatur oder darunter gehalten. Es wird 6 h bei Raumtemperatur nachgerührt. Der ausgefallene Niederschlag wird abgenutscht und aus Acetonitril umkristallisiert, um 46 g (85 % d.Th) der Titelverbindung (Hydrochlorid) vom Schmp. 207 - 208°C zu ergeben.

Zur Bildung der freien Base wird das Hydrochlorid in Dichlormethan suspendiert und mit der äquivalenten Menge einer 1N- K₂CO₃ -Lösung geschüttelt. Der nach dem Abtrennen von der wäßrigen Phase, Trocknen und dem Entfernen des Lösungsmittels im Vakuum verbleibende Rückstand wird aus Acetonitril umkristallisiert. Die freie Base zeigt dann einen Schmelzpunkt von 138 - 139°C.

### Beispiel 8

### N-(3-Morpholinopropyl)-1-methyl-4-imidazolsulfonsäureamid

16,7 g (46,5 mmol) 5-Chlor-N-(3-morpholino-1-propyl)-1-methyl-4-imidazolsulfonamid Hydrochlorid aus Beispiel 32 werden in Gegenwart von 3 g Pd/C - Katalysator in 250 ml Wasser bei 25°C und 3,45 bar hydriert. Der nach Filtration und Eindampfen im Vakuum verbleibende Rückstand wird mit gesättigter Pottaschelösung in die freie Base überführt, mit Dichlormethan extrahiert und aus Dioxan/Diisopropylether umkristallisiert, um die kristalline Titelverbindung, identisch mit der gemäß Beispiel 7 zu ergeben. Ausbeute: 6,3 g (43 % d.Th.)

In analoger Weise wie in Beispiel 7 erhält man aus 1-Methyl-4-imidazolsulfonsäurechlorid und dem Amin der Zeilen b die jeweilige Titelverbindung der Zeilen a:

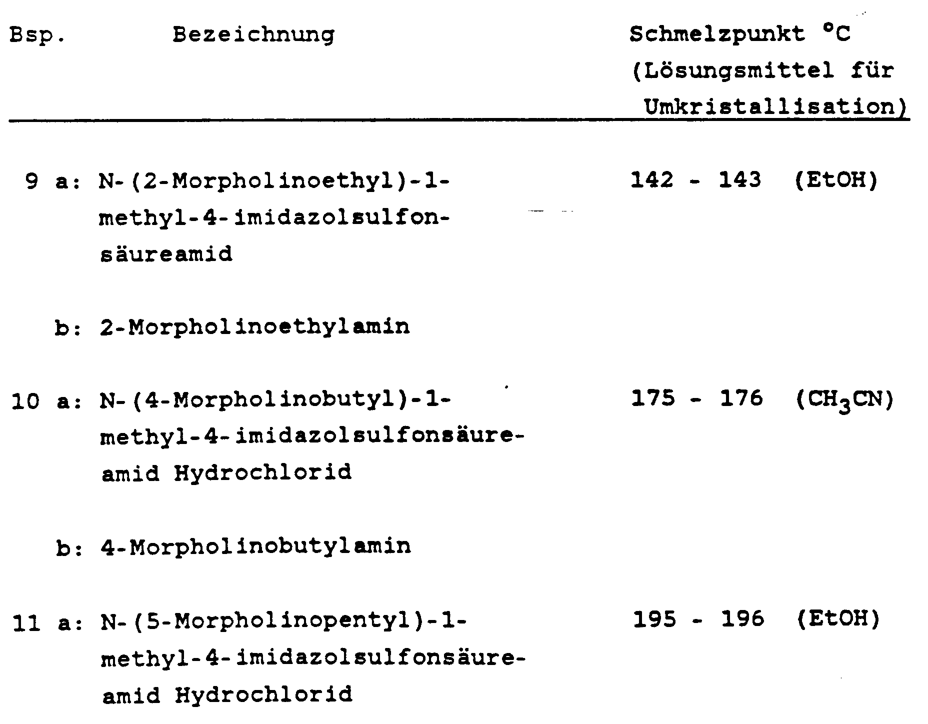

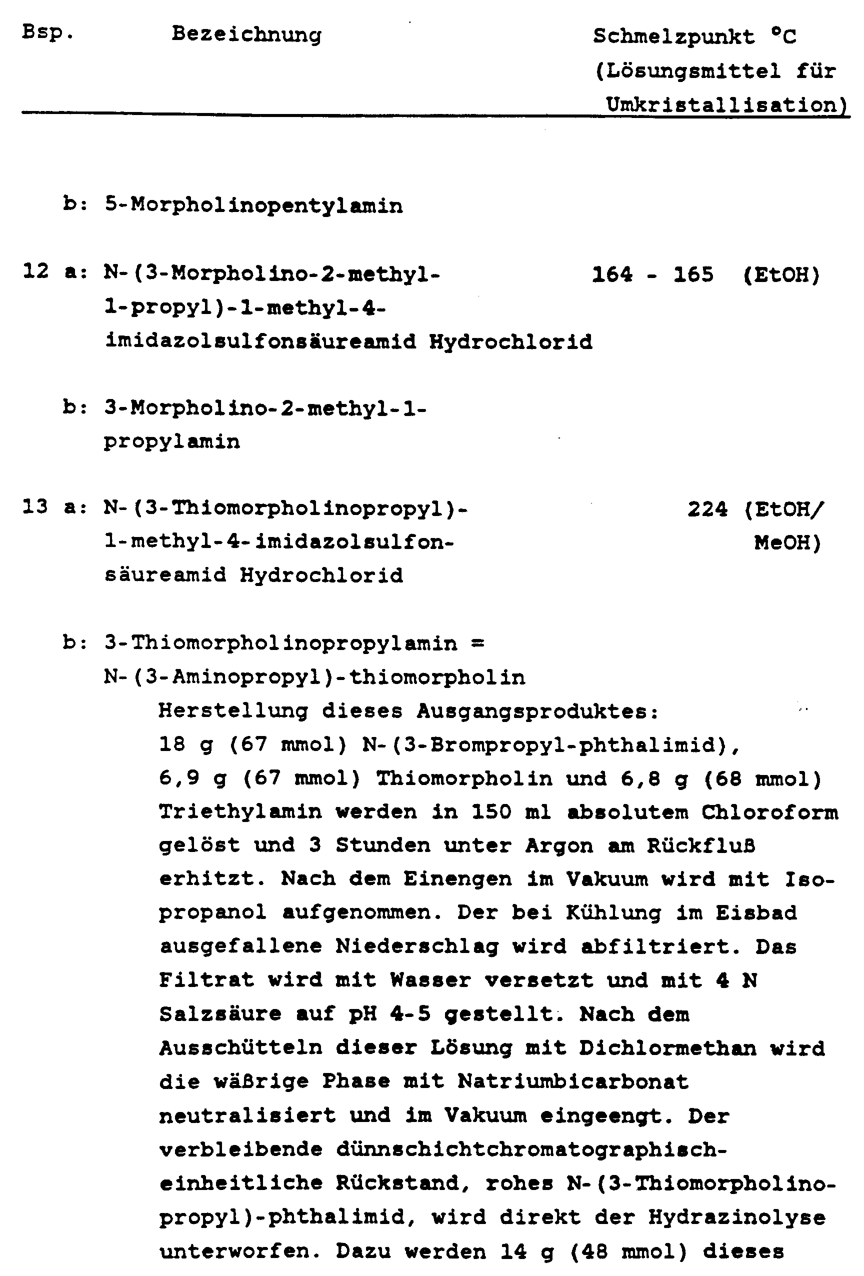

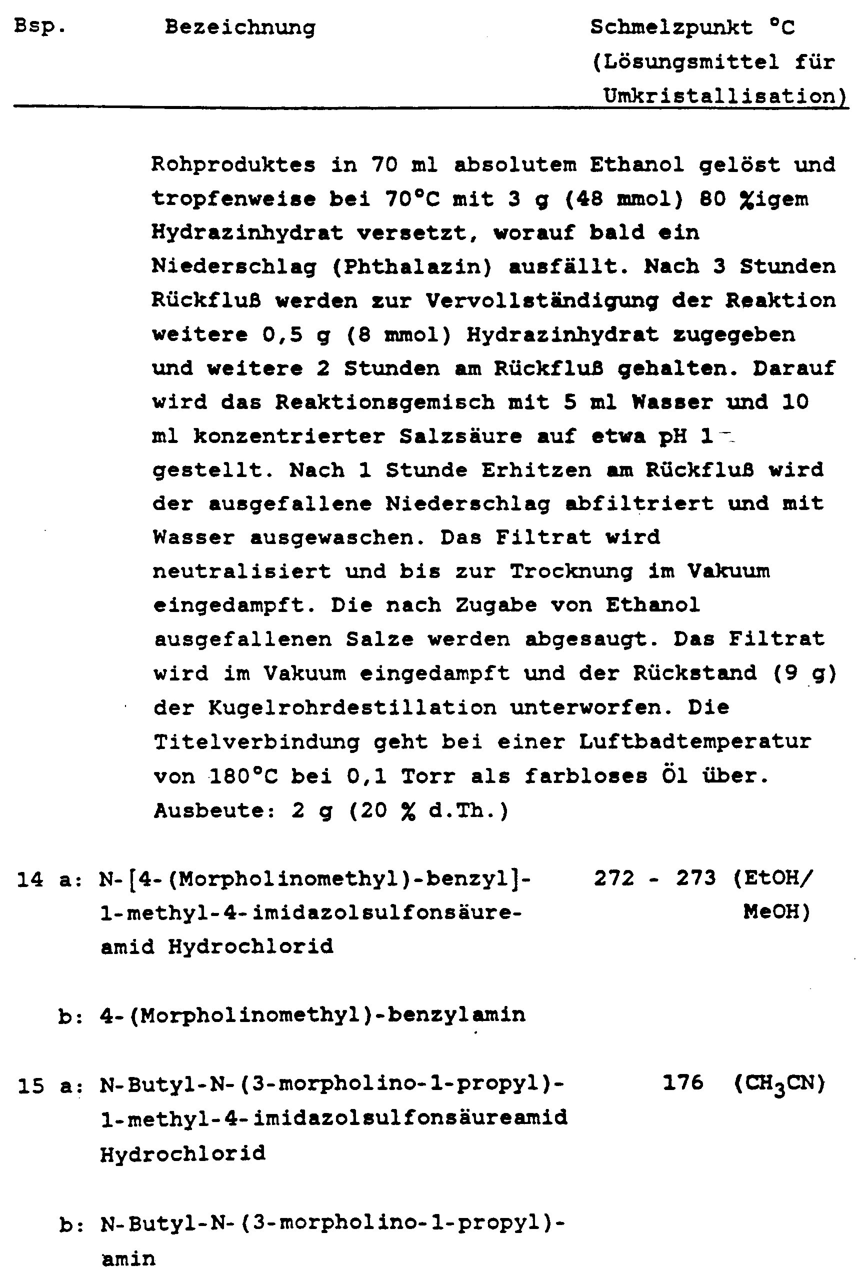

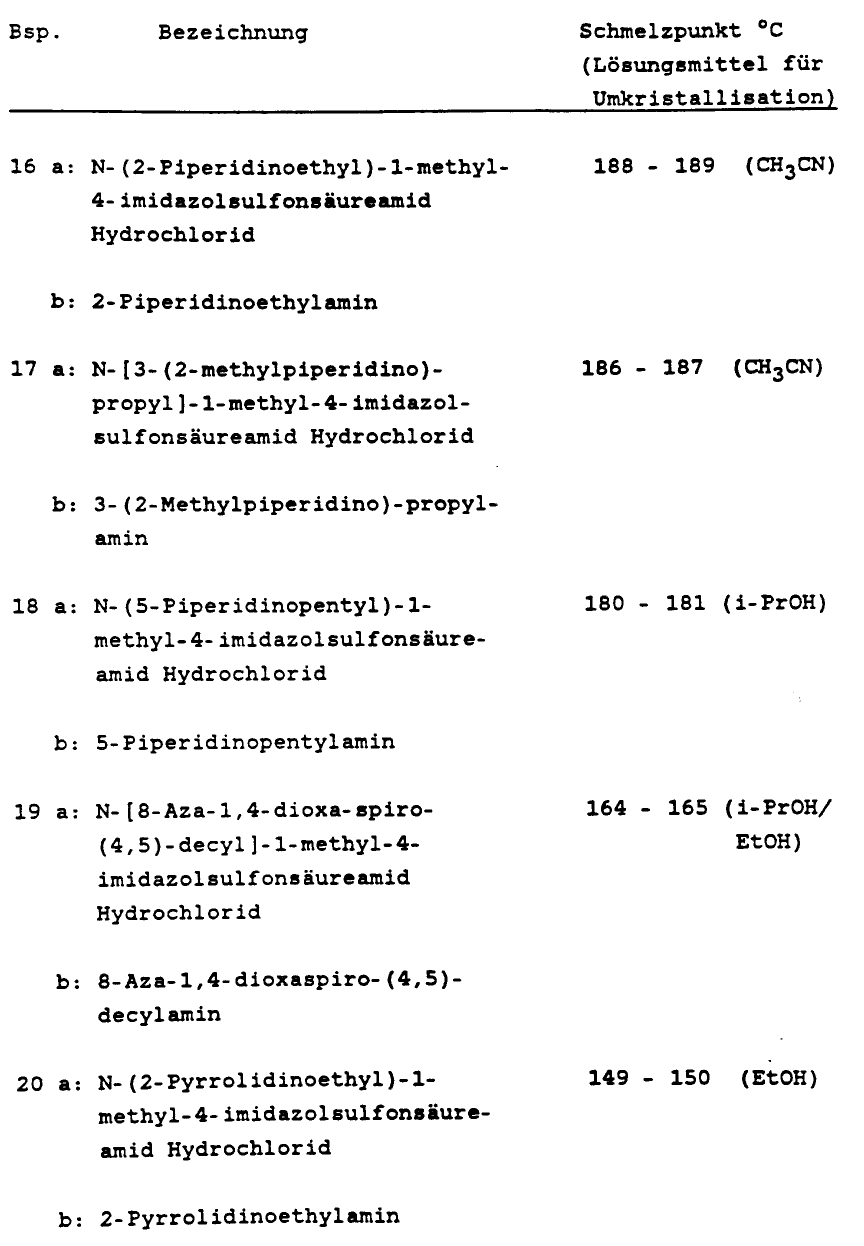

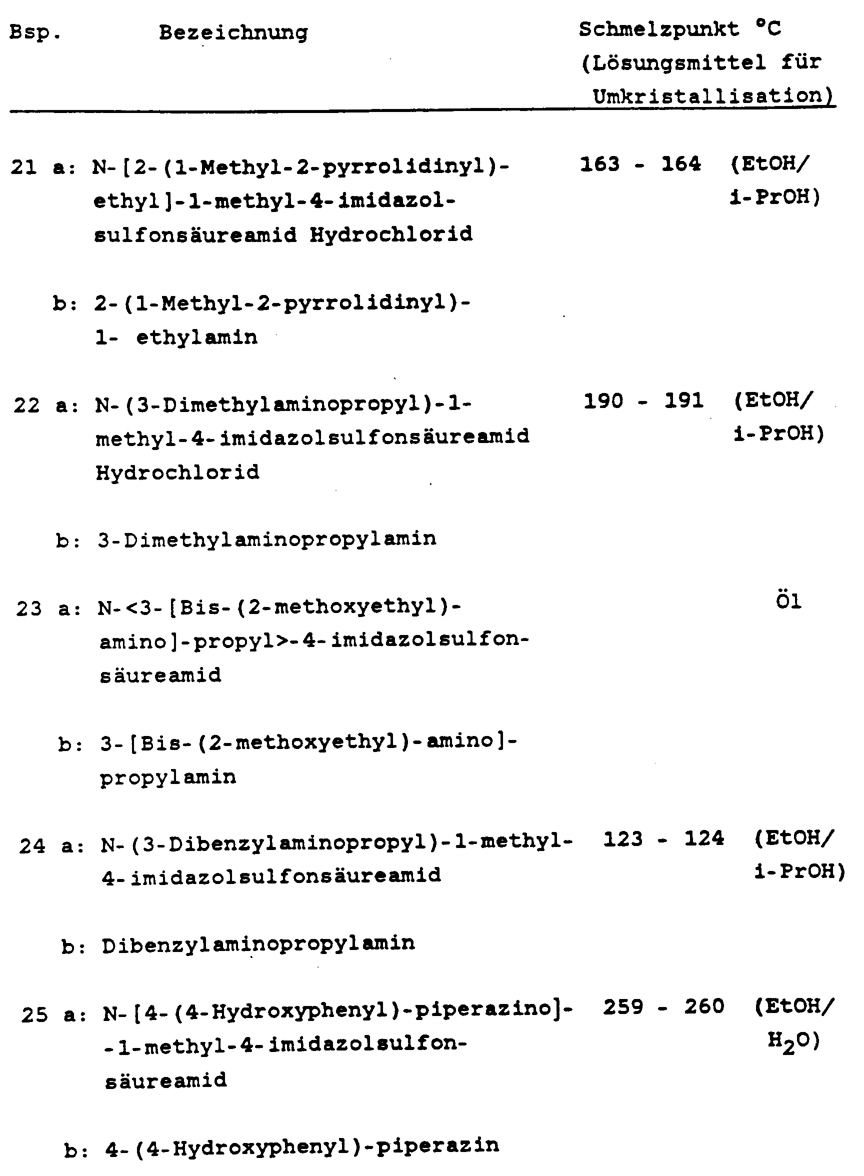

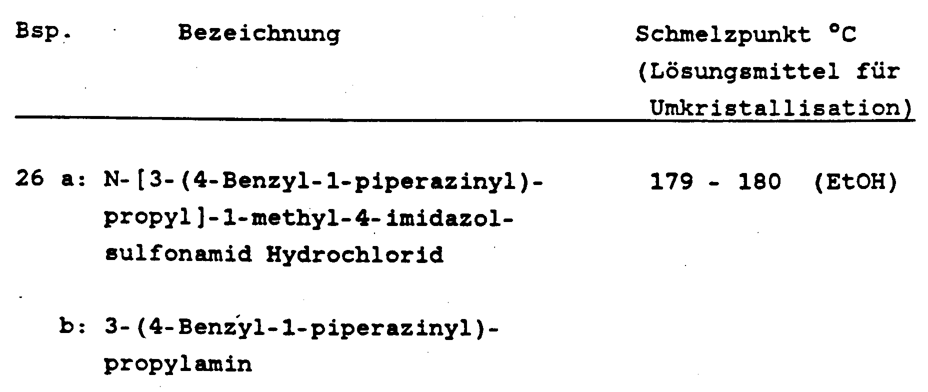

### Beispiel 27

### N-[3-(4-Methylpiperazino)-propyl]-1-methyl-4-imidazolsulfonsäureamid Dihydrogenfumarat

In analoger Weise wie in Beispiel 7 beschrieben, erhält man aus 1-Methyl-4-imidazolsulfonsäurechlorid und 3-(4-Methylpiperazino)-propylamin die freie Base der Titelverbindung. Das nach Zugabe einer ethanolischen doppeltmolaren Menge Fumarsäure in etwa 42 %iger Ausbeute kristallisierende Dihydrogenfumarat schmilzt bei 209 - 210°C.

### Beispiel 28

### N-[3-(N -Benzyl-N -methylamino)-1-propyl]-1-methyl-4-imidazolsulfonsäureamid Hydrogenfumarat

In analoger Weise wie in Beispiel 7 beschrieben, erhält man aus 1-Methyl-4- imidazolsulfonsäurechlorid und 3-(N-Benzyl-N-methylamino)-1-propylamin die freie Base der Titelverbindung. Das nach Zugabe einer ethanolischen äquimolaren Menge Fumarsäure kristallisierende Hydrogenfumarat schmilzt bei 184 - 185°C.
Ausbeute: 53,4 % d.Th.

In analoger Weise wie in Beispiel 7 beschrieben, erhält man
29) N-(3-Morpholinopropyl)-1-methyl-2-imidazolsulfonsäureamid Hydrochlorid, Schmp. 177 - 178°C (aus Ethanol) aus 1-Methyl-2-imidazolsulfonsäurechlorid und 3-Morpholinopropylamin.
**30) N-(2-Morpholinoethyl)-1-methyl-2-imidazolsulfonsäureamid Hydrochlorid,** Schmp. 197 - 198°C (aus Ethanol) aus 1-Methyl-2-imidazolsulfonsäurechlorid und 2-Morpholinoethylamin.
**31) N-(3-Morpholinopropyl)-4-chlor-1-methyl-5-imidazolsulfonsäureamid,** Schmp. 113 - 114°C (aus Ethanol) aus 4-Chlor-1-methyl-5-imidazolsulfonsäurechlorid und 3-Morpholinopropylamin.
**32) N-(3-Morpholinopropyl)-5-chlor-1-methyl-4-imidazolsulfonsäureamid Hydrochlorid,** Schmp. 179 - 180°C (aus Methanol/Isopropanol) aus 5-Chlor-1-methyl-4-imidazolsulfonsäurechlorid und 3-Morpholinopropylamin.
**33) N-(3-Morpholinopropyl)-1,2-dimethyl-4-imidazolsulfonsäureamid Hydrochlorid,** Schmp. 181 - 182°C (aus Ethanol) aus 1,2-Dimethyl-4-imidazolsulfonsäurechlorid und 3-Morpholinopropylamin.
**34) N-(5-Morpholino-1-pentyl)-5-chlor-1-methyl-4-imidazolsulfonsäureamid Hydrochlorid,** Schmp. 218 - 219°C (aus Ethanol) aus 5-Chlor-1-methyl-4-imidazolsulfonsäurechlorid und 5-Morpholinopentylamin.
**35) N-(3-Moryholinopropyl)-1-propyl-4-imidazolsulfonsäureamid und N-(3-Morpholinopropyl)-1-propyl-5-imidazolsulfonsäureamid als** 4,5-Stellungsisomerengemisch zu erhalten aus dem Isomerengemisch der 1-Propyl-4- und 1-Propyl-5-imidazolsulfonsäurechloride aus Beispiel C-4 und N-(3-Aminopropyl)-morpholin im Verhältnis 20,4:79,5 %. Dieses Gemisch ist durch HPLC auf modifiziertem Kieselgel (RP 18, Merck) mittels eines Gemisches von Wasser:Essigsäure:Acetonitril (7,5:1,5:1) getrennt zu eluieren.
**36) N-(3-Morpholinopropyl)-1-n-butyl-4-imidazolsulfonsäureamid und N-(3-Morpholinopropyl)-1-n-butyl-5-imidazolsulfonsäureamid als** 4,5-Stellungsisomerengemisch zu erhalten aus dem Isomerengemisch der 1-Butyl-4- und 1-Butyl-5-imidazolsulfonsäurechloride aus Beispiel C-5 und N-(3-Aminopropyl)-morpholin im Verhältnis 68,1 % : 17,5 %. Dieses Gemisch ist durch HPLC auf modifiziertem Kieselgel (RP 18, Merck) mittels eines Gemisches von Wasser:Essigsäure:Acetonitril (7,5:1,5:1) getrennt zu eluieren.

### Beispiel 37

### N-(3-Morpholinopropyl)-1-ethyl-4-imidazolsulfonsäureamid Hydrogenfumarat

Analog Beispiel 7 werden 10 g (51 mmol) 1-Ethyl-4-imidazolsulfonsäurechlorid aus Beispiel C-2 mit 7,5 ml (51 mmol) 3-Morpholinopropylamin in 200 ml Acetonitril umgesetzt und entsprechend aufgearbeitet. Das so erhaltene Hydrochlorid wird in Methanol gelöst, und mittels einer äquivalenten Menge methanolischer Natriummethylatlösung in die freie Base überführt. Das nach dem Abdampfen im Vakuum verbliebene Öl kristallisiert nach Zugabe von einer ethanolischen äquimolaren Menge Fumarsäure als Hydrogenfumarat mit dem Schmelzpunkt 148 - 149°C.

### Beispiel 38

### N-(1,3-Dimorpholino-2-propyl)-1-methyl-4-imidazolsulfonamid

4 g (17,5 mmol) 1,3-Dimorpholino-2-propylamin und 3,5 g (17,5 mmol) MSTFA [N-Methyl-N-(trimethylsilyl)trifluoracetamid] werden unter Argon vereinigt und 17 Stunden bei Raumtemperatur gerührt. Dann wird die klare Lösung bei 40°C/0.1 Torr im Vakuum eingeengt. Das erhaltene Öl wird mit einer Lösung von 3,16 g (17,5 mmol) 1-Methyl-4-imidazolsulfonsäurechlorid in 20 ml absolutem Dichlormethan bei 20°C unter Rühren versetzt. Nach weiterem Rühren für 6 Stunden bei 20°C wird das Lösungsmittel bei 20°C/18 Torr im Vakuum abdestilliert. Das verbleibende Öl kristallisiert aus Isopropanol. Nach Entfärbung mit Aktivkohle ergibt Umkristallisieren aus Methanol/Isopropanol die Titelverbindung mit dem Schmelzpunkt 191 - 192°C.
Ausbeute: 3,9 g (60 % d.Th.)

### Beispiel 39

### N-(3-N -Ethyl-N -isopropylaminopropyl)-1-methyl-4-imidazolsulfonsäureamid

Zu einer Lösung von 13 g (0,06 mol) 1-Trimethylsilylamino-N-ethyl-N-isopropyl-3-propylamin in 100 ml Acetonitril wird bei Raumtemperatur die Lösung von 10,8 g (0,06 mol) 1-Methyl-4-imidazolsulfonsäurechlorid aus Beispiel C-1 zugetropft und 6 Stunden weitergerührt. Der nach dem Eindampfen im Vakuum verbleibende Rückstand wird aus Isopropanol umkristallisiert, um die Titelverbindung mit dem Schmelzpunkt von 145 - 146°C zu ergeben.
Ausbeute: 5,7 g (29,2 % d.Th.)

### Beispiel 40

### N-[4-(4-Hydroxyphenyl)-piperazino]-5-chlor-1-methyl-4-imidazolsulfonsäureamid

Zu 16 g (90 mmol) 4-(4-Hydroxyphenyl)-piperazin in 250 ml Chloroform wird bei Raumtemperatur eine Lösung von 20 g (93 mmol) 5-Chlor-1-methyl-4-imidazolsulfonsäurechlorid in 250 ml Chloroform zugetropft. Danach werden langsam 41 ml (0,3 mol) Triethylamin zugetropft. Wenn nach 6 Stunden Rühren bei Raumtemperatur dünnschichtchromatographisch kein Säurechlorid mehr vorhanden ist, wird der gebildete Niederschlag abfiltriert, mehrfach mit Wasser gewaschen, getrocknet und aus Acetonitril umkristallisiert. Die Titelverbindung schmilzt bei 249 - 250°C.
Ausbeute: 22 g (66,3 % d.Th.)

### Beispiel 41

### 1-(1-Methyl-5-chlor-4-imidazolsulfonyl)-4-[4-(1-methyl-5-chlor-4-imidazolsulfonyloxy)-phenyl]-piperazin

Die Mutterlaugen von Beispiel 40 werden im Vakuum eingedampft. Der Rückstand wird mit Wasser gewaschen und aus Wasser/Ethanol umkristallisiert. Die so erhaltene Titelverbindung schmilzt bei 196 - 197°C.
Ausbeute: 3,5 g (21,1 % d.Th.)

### Beispiel 42

### 4-(1-Methyl-4-imidazolsulfonyl)-tetrahydro-4H-1,4-thiazin

Zu einer Lösung von 1,51 ml (15 mmol) Thiomorpholin in 50 ml Acetonitril werden 2 g (11 mmol) 1-Methyl-4-imidazolsulfonsäurechlorid aus Beispiel C-1, gelöst in 20 ml Acetonitril, unter Rühren bei Raumtemperatur zugetropft. Das Reaktionsgemisch wird 6 Stunden nachgerührt, filtriert und im Vakuum eingedampft. Der verbleibende Rückstand wird aus Isopropanol umkristallisiert, um die Titelverbindung mit Schmelzpunkt 154 - 155°C zu ergeben. Ausbeute: 1,37 g (50 % d.Th.)

In analoger Weise wie in Beispiel 42 beschrieben, erhält man:

| Bsp. | Bezeichnung | Schmelzpunkt °C (Lösungsmittel für die Umkristallisation) |
|---|---|---|
| 43 | 1-[3-(1-Methyl-5-imidazolsulfonyl)-aminopropyl]-2-pyrrolidinon aus N-(3-Aminopropyl)-2-pyrrolidinon und 1-Methyl-4-imidazolsulfonsäurechlorid | 125 - 126 (i-PrOH) |
| 44 | N-(3-Methoxypropyl)-1-methyl-4-imidazolsulfonsäureamid aus 3-Methoxypropylamin (2 Mol pro Mol Säurechlorid) und 1-Methyl-4-imidazolsulfonsäurechlorid | 95 - 96 (i-PrOH) |
| 45 | N-(4-Hydroxyphenethyl)-1-methyl-4-imidazolsulfonsäureamid aus Tyramin und 1-Methyl-4-imidazolsulfonsäurechlorid in Dichlormethan anstelle von Acetonitril | 207 - 208 (H₂O) |
| 46 | N-(4-Hydroxyphenethyl)-5-chlor-1-methyl-4-imidazolsulfonsäureamid aus Tyramin und 5-Chlor-1-methyl-4-imidazolsulfonsäurechlorid in Dichlormethan anstelle von Acetonitril | 170 (H₂O) |
| 47 | 1,6-Bis-(5-chlor-1-methyl-4 imidazolsulfonamido)-hexan aus Hexamethylendiamin und 5-Chlor-1-methyl-4-imidazolsulfonsäurechlorid | 209 - 210 (H₂O/MeOH) |
| 48 | N-(2-Cyanoethyl)-1-methyl-4-imidazolsulfonsäureamid aus 3-Aminopropionitril und 1-Methyl-4-imidazolsulfonsäurechlorid | 122 - 124 (EtOH) |
| 49 | N-(5-Cyanopentyl)-1-methyl-4-imidazolsulfonsäureamid aus 6-Aminocapronitril und 1-Methyl-4-imidazolsulfonsäurechlorid | 92 - 94 (H₂O) |

### Beispiel 50

### N-(3-Propargyl)-1-methyl-4-imidazolsulfonsäureamid

Zu einer vorgelegten, auf - 20°C gekühlten Lösung von 1,14 ml (16,6 mmol) Propargylamin in 30 ml Dichlormethan wird unter weiterer Kühlung und unter Rühren eine Lösung von 3 g (16,6 mmol) 1-Methyl-4-imidazolsulfonsäurechlorid aus Beispiel C-1 in 40 ml Dichlormethan zugetropft. Danach läßt man die Reaktionslösung sich langsam auf Raumtemperatur erwärmen. Der dabei ausfallende Niederschlag wird abgesaugt und aus Isopropanol umkristallisiert, um die Titelverbindung vom Schmelzpunkt 145°C zu bilden. Ausbeute: 1,5 g (45,3 % d.Th.)

### Beispiel 51

### 4-[2-(1-Methyl-4-imidazolsulfonyl)-aminoethyl]-phenoxyessigsäure

6,3 g (32 mmol) 4-(2-Aminoethyl)-phenoxyessigsäure werden zu einer Lösung von 9,7 g (79 mmol) Kaliumcarbonat in 50 ml Wasser gegeben und 5 Minuten gerührt. Zu dieser Suspension wird langsam eine Suspension von 5,8 g (32 mmol) 1-Methyl-4-imidazolsulfonsäurechlorid aus Beispiel C-1 in 20 ml Wasser zugefügt. Es wird auf 80°C erwärmt und bei dieser Temperatur 2 Stunden gerührt. Nach dem Abkühlen auf Raumtemperatur wird die Reaktionslösung mit 2 N Salzsäure auf pH 4 angesäuert. Der ausgefallene Niederschlag wird abgetrennt, mehrmals mit Wasser gewaschen und aus verdünnter Essigsäure umkristallisiert, um die Titelverbindung mit dem Schmelzpunkt 203 - 204°C zu ergeben.
Ausbeute: 6 g (55,2 % d.Th.)

### Beispiel 52

### 4-[2-(5-Chlor-1-methyl-4-imidazolsulfonyl)-aminoethyl]-phenoxyessigsäure

In analoger Weise wie in Beispiel 51 beschrieben, erhält man in 29 %iger Ausbeute die Titelverbindung aus 5-Chlor-1-methyl-4-imidazolsulfonsäurechlorid und 4-(2-Aminoethyl)-phenoxyessigsäure. Die aus Wasser umkristallisierte Verbindung schmilzt bei 174 - 175°C.

### Beispiel 53

### N-(3-Morpholino-1-propyl)-1-methyl-5-imidazolsulfonamid Hydrochlorid

1 g (2,8 mmol) N-(3-Morpholinopropyl)-4-chlor-1-methyl-5-imidazolsulfonamid aus Beispiel 31 wird in 130 ml 25 %igem wässrigen Ethanol gelöst, mit 0,3 g Pd/C-Katalysator versetzt und unter Schütteln bei einem Anfangsdruck von 3,45 bar bis zum Ende der Wasserstoffaufnahme hydriert. Der Katalysator wird abfiltriert. Das Filtrat wird im Vakuum eingeengt und der Rückstand aus Ethanol umkristallisiert. Man erhält die Titelverbindung mit dem Schmelzpunkt 205 - 206°C in einer Ausbeute von 0,8 g (88 % d.Th.).

### Beispiel 54

### 1,6-Bis-(1-methyl-4-imidazolsulfonamido)-hexan

13 g (27 mmol) 1,6-Bis-(5-chlor-1-methyl-4-imidazolsulfonamido)-hexan aus Beispiel 47 werden in 250 ml 1 N Natronlauge über 3 g 10 %igem Pd/C-Katalysator unter Schütteln bei einem Anfangsdruck von 3,45 bar bis zum Ende der Wasserstoffaufnahme hydriert. Nach Abtrennen des Katalysators wird das Filtrat im Vakuum eingedampft. Der Rückstand wurde aus Wasser/Methanol umkristallisiert, um die Titelverbindung als farblose Kristalle mit dem Schmelzpunkt 153 - 154°C zu ergeben Ausbeute: 6,1 g (55 % d.Th.)

### Beispiel 55

### N-[3-(1-Piperazinyl)-propyl]-1-methyl-4-imidazolsulfonamid Hydrochlorid

3 g (7,2 mmol) N-[3-(4-Benzyl-1-piperazinyl)-1-propyl]-1-methyl-4-imidazolsulfonamid Hydrochlorid aus Beispiel 26 werden in einer Lösung von 30 ml Ethanol in 150 ml 25 %iger Ammoniumhydroxid-Lösung unter Schütteln, bei 3,45 bar Anfangsdruck und Raumtemperatur bis zur Beendigung der Wasserstoffaufnahme in Gegenwart von 1 g Pd/C- Katalysator hydriert. Nach Einengen unter vermindertem Druck wird der Rückstand aus Ethanol umkristallisiert, um die gewünschte Verbindung mit dem Schmelzpunkt 174 - 175°C zu ergeben. Ausbeute: 1,4 g (59 % d.Th.)

### Beispiel 56

### 4-Methyl-4-[3-(1-methyl-4-imidazolsulfamoyl)-1-propyl]-morpholiniumjodid

2 g (6,9 mmol) N-(3-Morpholinopropyl)-1-methyl-4-imidazolsulfonamid aus Beispiel 7 werden bei Raumtemperatur in einer Lösung von 0,48 ml (7,6 mmol) Jodmethan in 50 ml Acetonitril 8 Stunden gerührt. Der gebildete Niederschlag wird abgetrennt, gut mit Acetonitril gewaschen und getrocknet, um die Titelverbindung mit dem Schmelzpunkt 204 - 205°C zu ergeben.
Ausbeute: 2,5 g (84 % d.Th.)

### Beispiel 57

### 4-(1-Methyl-4-imidazolsulfonyl)-tetrahydro-4H-1,4-thiazin-1,1-dioxid

1 g (4 mmol) 4-(1-Methyl-4-imidazolsulfonyl)-tetrahydro-4H-1,4-thiazin aus Beispiel 42 werden in 10 ml Chloroform aufgenommen und tropfenweise bei 0 - 5°C mit einer Lösung von 1,39 g (8 mmol) m-Chlorperoxybenzoesäure versetzt. Nach dem Erwärmen auf Raumtemperatur wird 2 Stunden nachgerührt, wobei das Reaktionsprodukt ausfällt, das abgesaugt und aus Wasser umkristallisiert wird. Die so erhaltene Titelverbindung schmilzt bei 179 - 180°C.
Ausbeute: 0,3 g (26,6 % d.Th.)

### Beispiel 58

### 4-[4-(5-Chlor-1-methyl-4-imidazolsulfonyl)-piperazinyl]-phenoxyessigsäureethylester

2 g (5,6 mmol) N-[4-(4-Hydroxyphenyl)-piperazino]-5-chlor-1-methyl-4-imidazolsulfonsäureamid aus Beispiel 40 werden in 70 ml Ethanol aufgenommen, mit 0,22 g (5,5 mmol) Natriumhydroxid versetzt, 30 Minuten gerührt, und tropfenweise mit 0,86 g (6 mmol) Bromessigsäureethylester versetzt. Wenn nach 6-stündigem Rühren bei Raumtemperatur die Umsetzung nach DC noch unvollständig ist, setzt man nochmals 0,42 g (2,5 mmol) Bromessigsäureethylester und 0,12 g (3 mmol) Natriumhydroxid zu, erhitzt auf 50°C und rührt etwa 10 Stunden nach. Nach dem Eindampfen im Vakuum wird der Rückstand in Dichlormethan aufgenommen und mit 2 N NaOH gewaschen. Die organische Phase wird nach dem Trocknen über Natriumsulfat im Vakuum eingedampft. Der kristalline Rückstand läßt sich aus Isopropanol umkristallisieren. Die so erhaltene Titelverbindung schmilzt bei 148 - 149°C.
Ausbeute: 0,8 g (32 % d.Th.)

### Beispiel 59

### N-(6-Aminohexyl)-1-methyl-4-imidazolsulfonsäureamid Dihydrochlorid

2,6 g(10 mmol) N-(5-Cyanopentylamino)-1-methyl-4-imidazol-sulfonsäureamid aus Beispiel 49 werden in 50 ml etwa 5 N ethanolischer Ammoniak-Lösung gelöst, mit 1 g Raney-Nickel versetzt und unter Schütteln bei einem Anfangsdruck von 3,45 bar bis zum Ende der Wasserstoffaufnahme hydriert. Der Katalysator wird abfiltriert. Das Filtrat wird im Vakuum eingeengt. Das zurückbleibende Öl wird in absolutem Ethanol gelöst. Auf Zusatz von ethanolischer Salzsäure fällt die Titelverbindung als kristallines Salz, das nach Abtrennung und Trocknen einen Schmelzpunkt von 215 - 223°C hat, aus.
Ausbeute: 2,3 g (69 % d.Th.)

### Beispiel 60

### N-(3-Aminopropyl)-1-methyl-4-imidazolsulfonsäureamid Hydrochlorid

In analoger Weise die in Beispiel 59 beschrieben, erhält man die Titelverbindung aus N-(3-Cyanoethylamino)-1-methyl-4-imidazolsulfonsäureamid aus Beispiel 48 mit dem Schmelzpunkt 168 - 169°C in etwa 55 %iger Ausbeute.

### Beispiel 61

### N-(3-Thiomorpholinopropyl)-1-methyl-4-imidazolsulfonsäureamid S-oxid Hydrochlorid

Eine Lösung von 3,5 g (10 mmol) N-(3-Thiomorpholinopropyl)-1-methyl-4-imidazolsulfonsäureamid Hydrochlorid aus Beispiel 13 in 30 ml 50 %igem wäßrigem Methanol wird bei -5°C zu einer Lösung von 1,9 g (9 mmol) Natriumjodat in 25 ml Wasser zugetropft. Ein zwischenzeitlich gebildeter Niederschlag geht nach weiteren 20 Minuten wieder in Lösung. Nach Stehen über Nacht wird die Reaktionslösung mit überschüssigem Natriumbicarbonat versetzt, im Vakuum zur Trockne eingedampft und durch Säulenchromatographie an Kieselgel mit Dichlormethan:Methanol 9:1 bis 0:10 gereinigt. Das eluierte Öl überführt man mit ethanolischer Salzsäure ins Hydrochlorid und die so erhaltene Titelverbindung wird bis zum Schmelzpunkt von 186°C aus Ethanol/Methanol umkristallisiert.
Ausbeute: 1,2 g (32 % d.Th.)

### Beispiel 62

### N-(3-Methylamino-1-propyl)-1-methyl-4-imidazolsulfonamid Hydrochlorid

15 g (41,7 mmol) N-[3-(N -Benzyl-N -methylamino)-1-propyl)-1-methyl-4-imidazolsulfonamid Hydrochlorid aus Beispiel 28 werden in einer Lösung von 100 ml 25 %iger Ammoniaklösung und 100 ml Ethanol in Gegenwart von 2 g 10 %igem Pd/C-Katalysator hydriert. Nach Ende der Wasserstoffaufnahme und Abfiltrieren vom Katalysator, wird das Filtrat im Vakuum eingedampft. Der Rückstand wird aus Ethanol umkristallisiert, um die Titelverbindung mit dem Schmelzpunkt 169 - 170°C zu ergeben.
Ausbeute: 2,3 g (20,5 % d.Th.)

### C) Zwischenprodukte (Imidazolsulfonsäurechloride)

### Beispiel 1

### 1-Methyl-4-imidazolsulfonsäurechlorid

1-Methylimidazol (250 g, 3,05 mol) wird derart zu Chlorschwefelsäure (600 ml, 9,03 mol) tropfenweise zugegeben, daß eine Innentemperatur von 30°C nicht überschritten wird, ohne den gebildeten Chlorwasserstoff abzusaugen. Nach beendeter Zugabe wird das Reaktionsgemisch 6 h bei 150°C gerührt. Bei 60°C wird mit Thionylchlorid (340 ml, 4,66 mol) versetzt und anschließend 6 h bei 100°C Badtemperatur erhitzt. Nach dem Abkühlen auf Raumtemperatur wird das dickflüssige Reaktionsgemisch auf soviel Eis gegossen, daß am Ende ca 7.5 l Wasser-Eis-Gemisch bleibt. Der ausgefallene Niederschlag wird abgenutscht und kurz lufttrocken gesaugt. Dann wird entweder in dünner Schicht im Vakuumtrockenschrank bei 50°C und 15 Torr getrocknet, oder es wird bevorzugt mit Dichlormethan aufgenommen, über Natriumsulfat getrocknet und im Vakuum vom Lösungsmittel befreit. Ausbeute: Es wurden 176 g (32 % d.Th.) farblose Kristalle erhalten (Schmp.: 89 - 90°C). Zur Entfernung der als Nebenprodukt gebildeten isomeren 5-Imidazolsulfonsäure aus der Mutterlauge wird diese weitgehend im Rotationsverdampfer bei 15 Torr im Vakuum eingeengt. Bei Zugabe von Ethanol kristallisiert ein Imidazolsulfonsäuregemisch aus, das aus Ethanol umkristallisiert werden kann.

### Beispiel 2

### 1-Ethyl-4-imidazolsulfonsäurechlorid

In analoger Weise wie in Beispiel C-1 beschrieben, erhält man die Titelverbindung in etwa 24,7 %iger Ausbeute mit dem Schmelzpunkt 34 - 35°C aus 1-Ethylimidazol mittels Chlorsulfonsäure und Thionylchlorid.

### Beispiel 3

### 1,2-Dimethyl-4-imidazolsulfonsäurechlorid

In analoger Weise wie in Beispiel C-1 beschrieben, erhält man die Titelverbindung in 30 %iger Ausbeute aus 1,2-Dimethylimidazol mittels Chlorsulfonsäure und Thionylchlorid. Sie läßt sich aus Toluol/Cyclohexan umkristallisieren und hat dann einen Schmelzpunkt von 90 - 91°C.

### Beispiel 4

### 1-Propyl-4-imidazolsulfonsäurechlorid und 1-Propyl-5-imidazolsulfonsäurechlorid

In analoger Weise wie in Beispiel C-1 beschrieben, erhält man in etwa 57 %iger Ausbeute ein Gemisch der Titelverbindungen aus 1-Propylimidazol mittels Chlorsulfonsäure und Thionylchlorid, das zweckmäßigerweise in Form ihrer Derivate getrennt wird.

### Beispiel 5

### 1-Butyl-4-imidazolsulfonsäurechlorid und 1-Butyl-5-imidazolsulfonsäurechlorid

In analoger Weise wie in Beispiel C-1 beschrieben, erhält man in etwa 17 %iger Ausbeute ein Gemisch der Titelverbindungen aus 1-Butylimidazol mittels Chlorsulfonsäure und Thionylchlorid, das zweckmäßigerweise in Form ihrer Derivate getrennt wird.

Die Verbindungen der Formel I gemäß den Beispielen aus den Abschnitten A und B sind in der folgenden Tabelle 1 zusammengestellt; wenn in den Beispielen die Salze hergestellt wurden, ist dies auch in der Tabelle berücksichtigt. In der Tabelle sind weiterhin auch die Verfahrensvarianten angegeben, nach denen die Verbindungen in den betreffenden Beispielen hergestellt wurden.

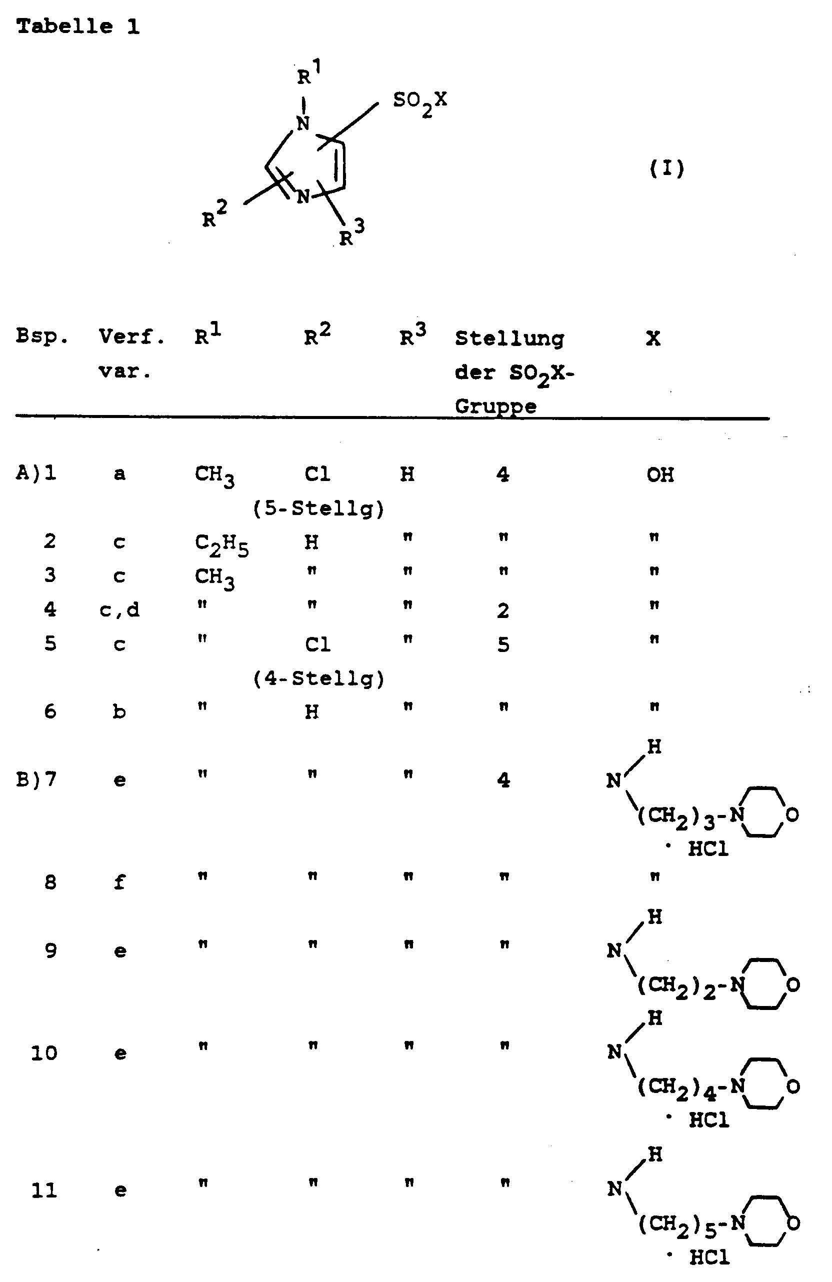

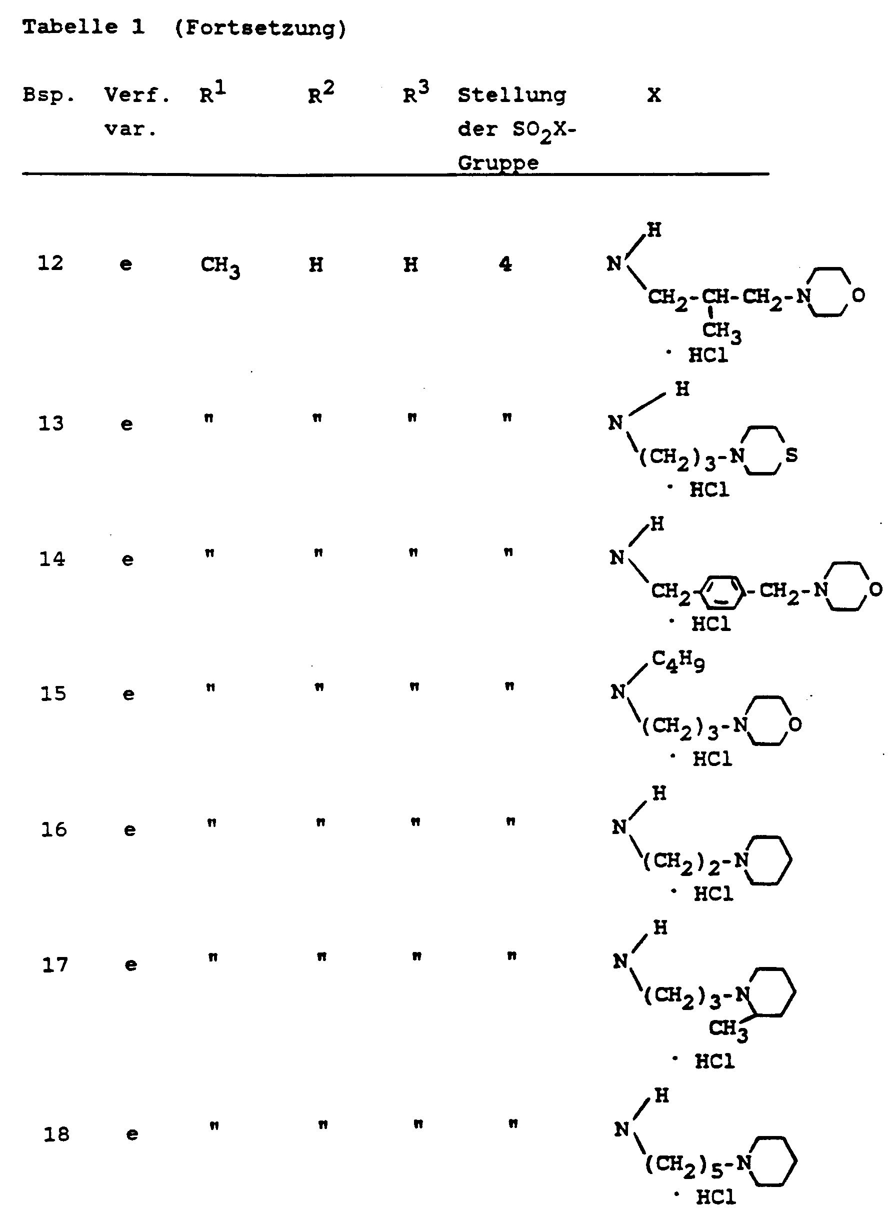

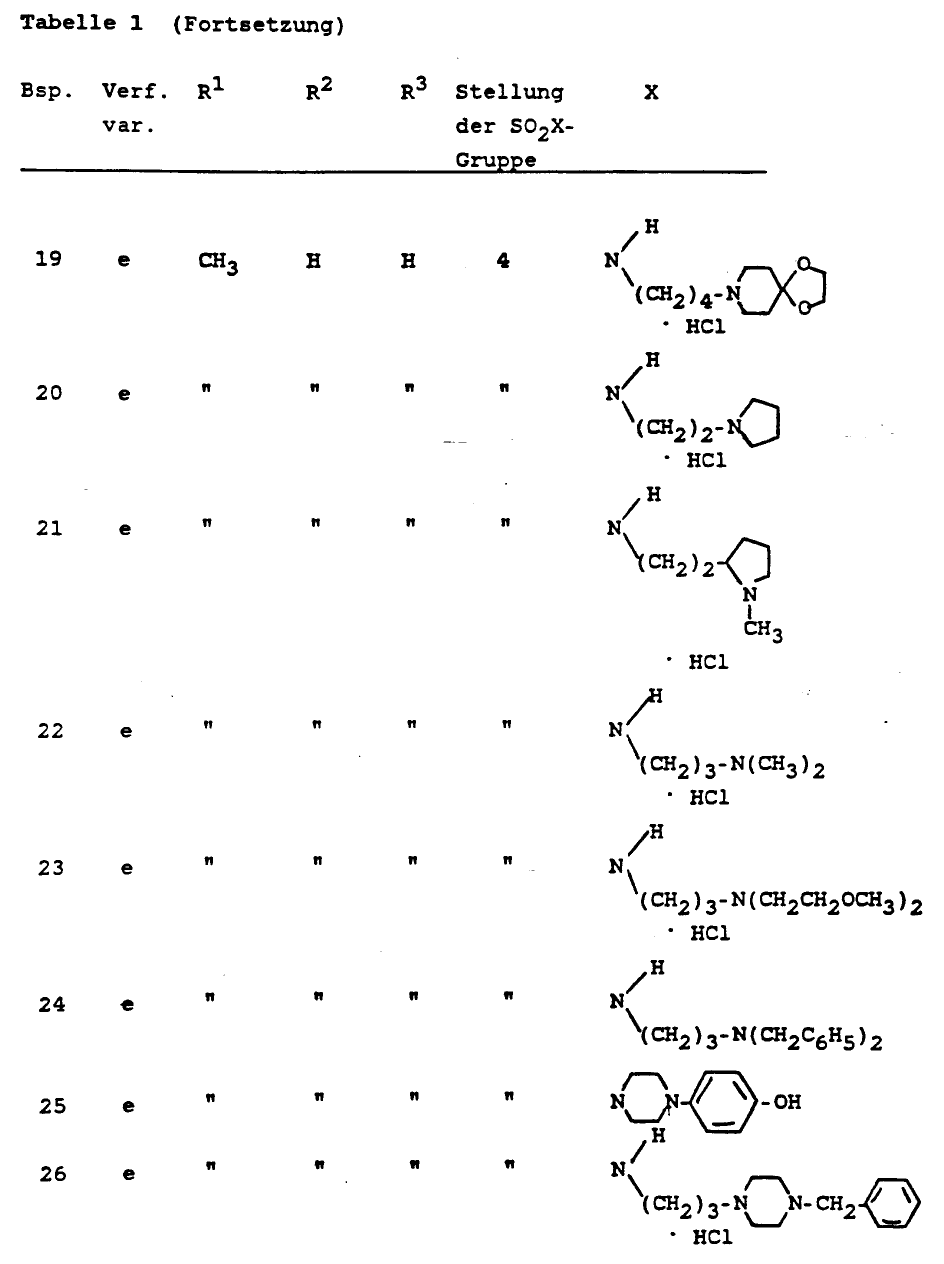

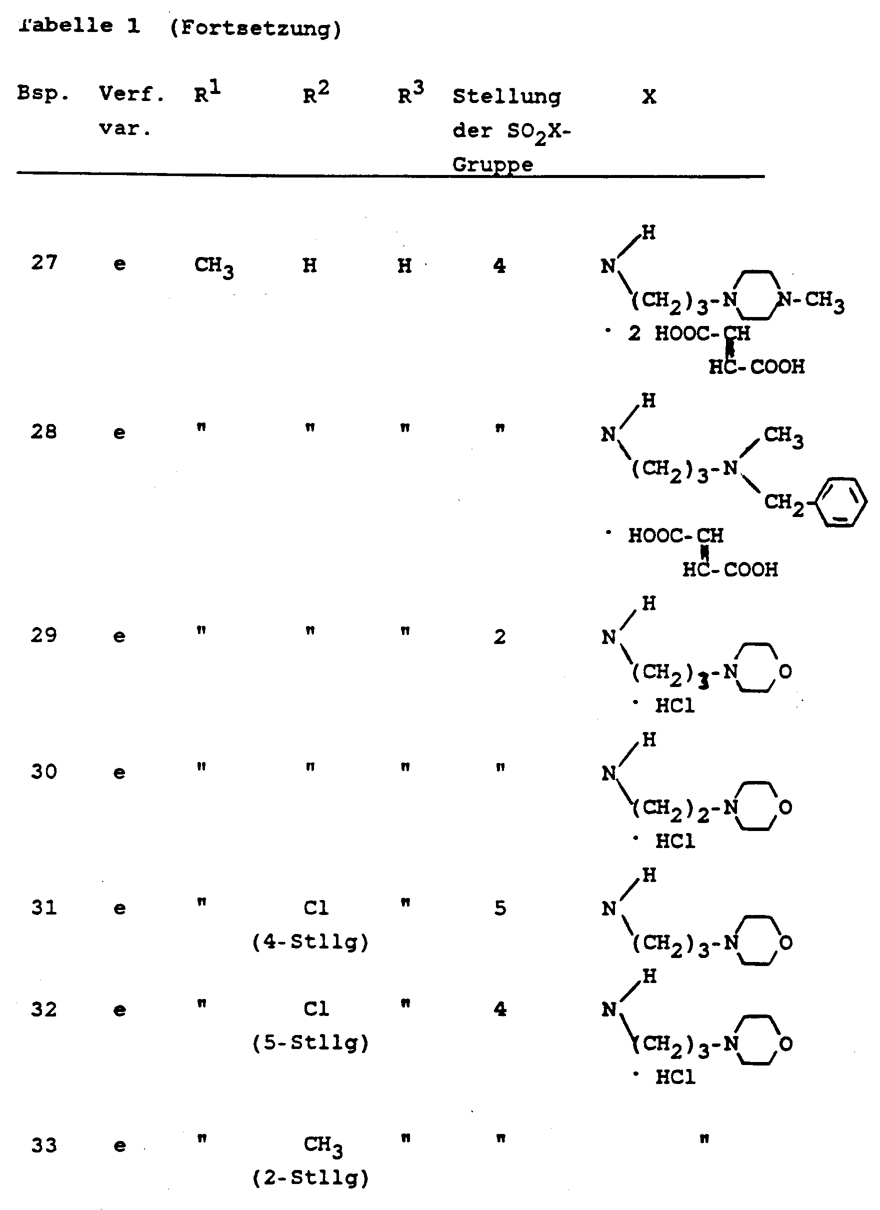

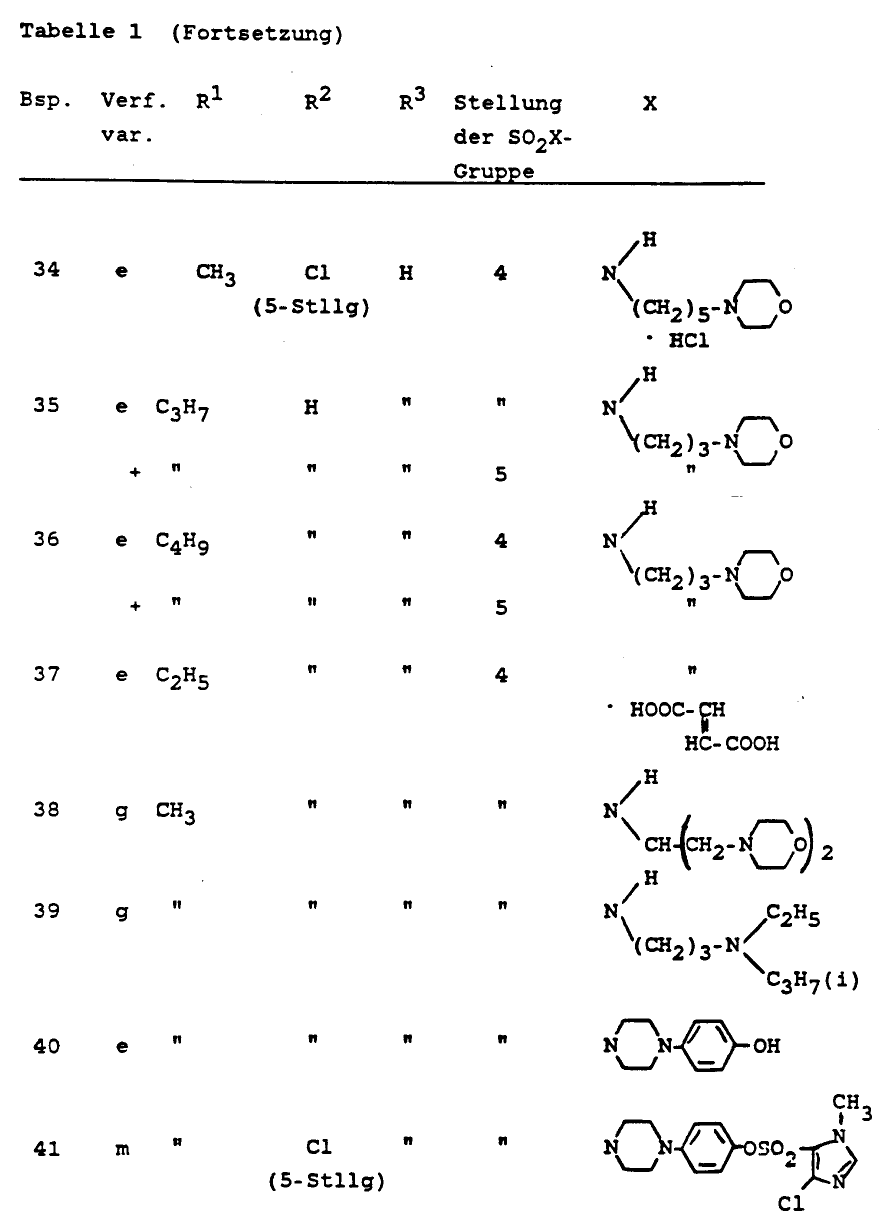

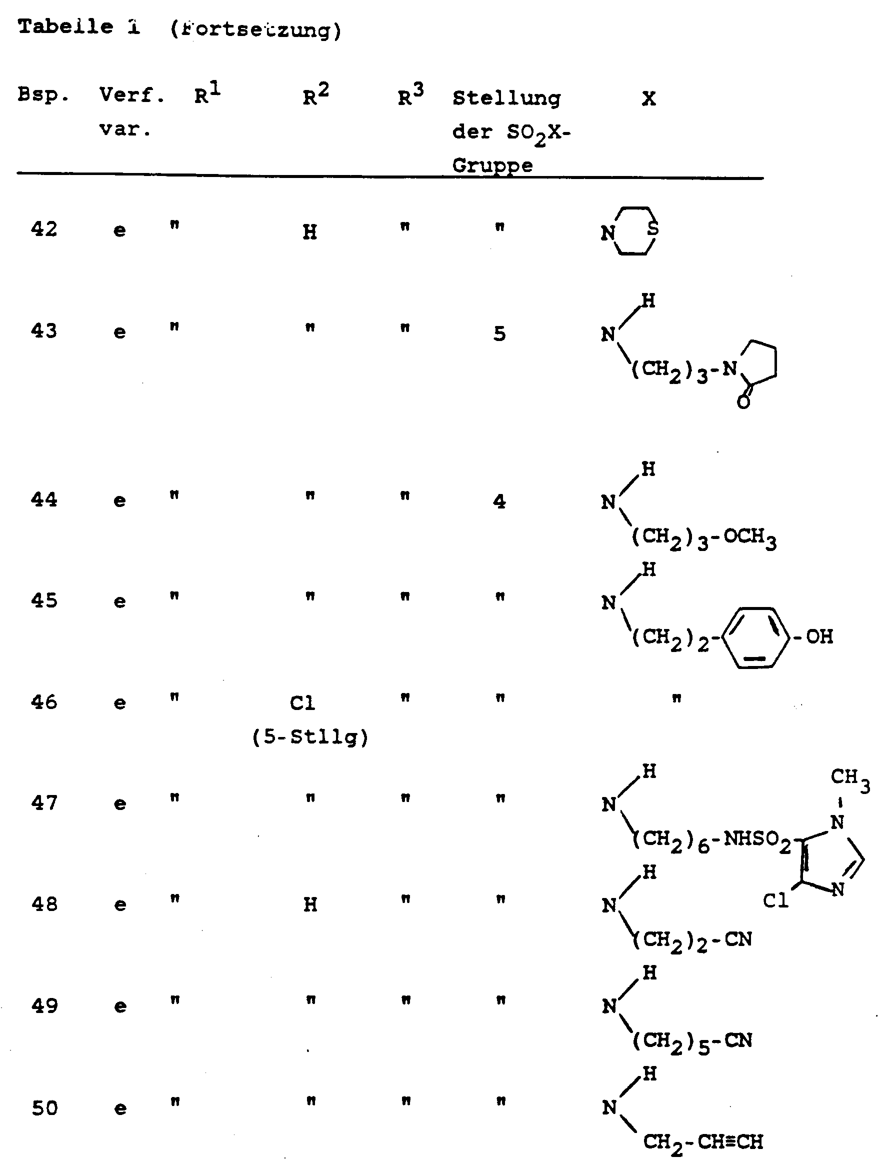

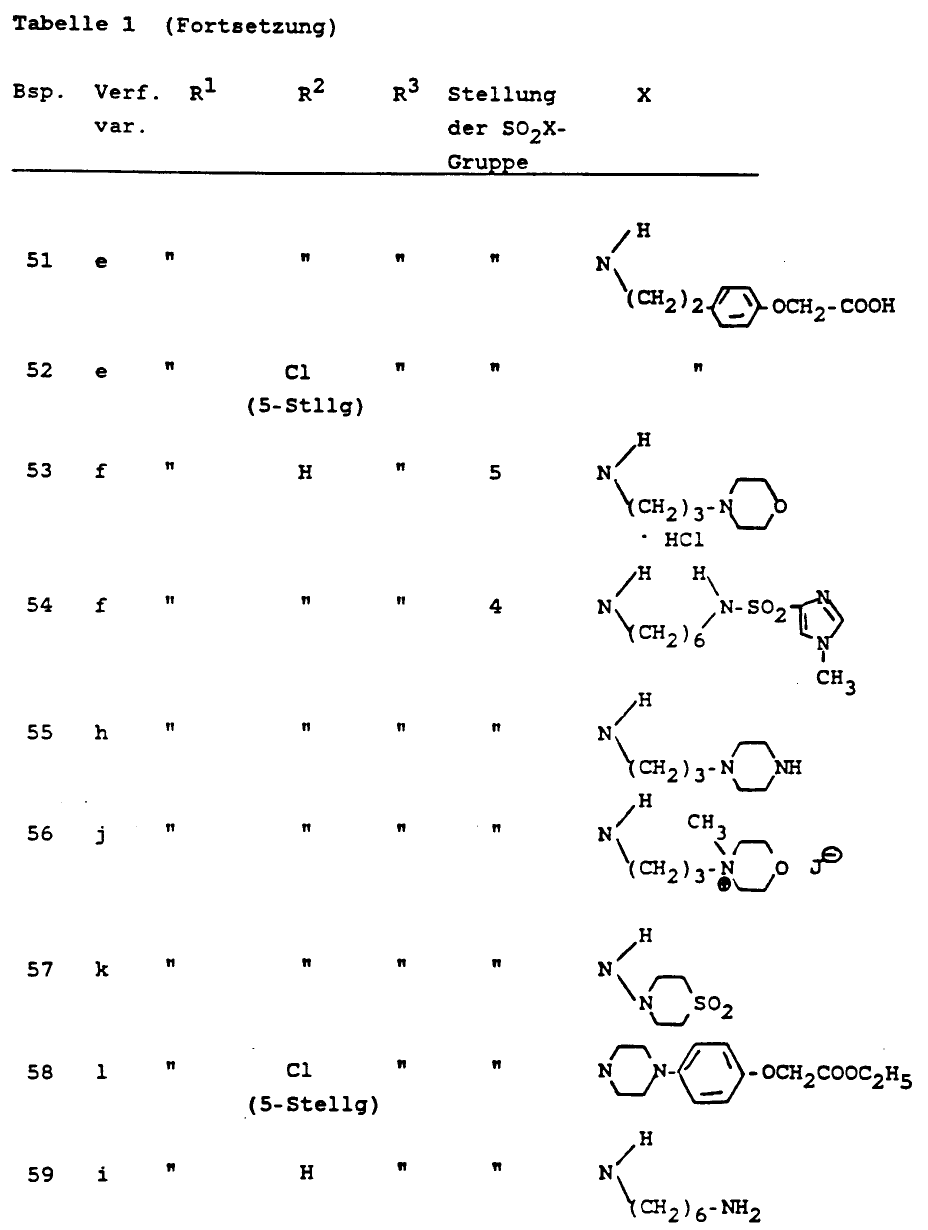

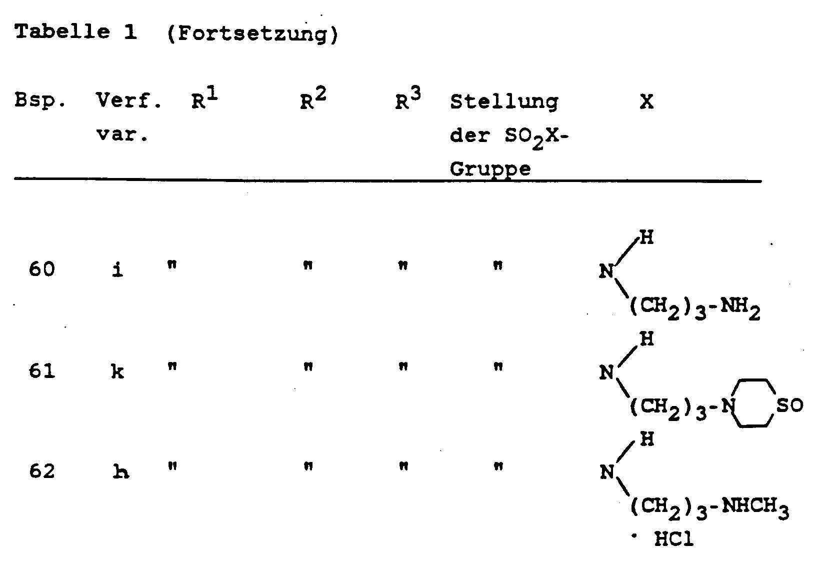

### Pharmakologische Prüfung und Ergebnisse

### 1) Wirkung auf die Kontraktilität des Skelettmuskels nach chronischer Ischämie

In den letzten Jahren hat sich in den Vorstellungen über die Pathophysiologie der chronischen peripheren arteriellen Verschlußkrankheit ein beachtlicher Wandel vollzogen, als sich das wissenschaftliche Interesse in zunehmendem Maße von der Makrozirkulation hin zur Mikrozirkulation verlagerte. Störungen in der Mikrozirkulation manifestieren sich demzufolge in einer Unterversorgung mit Substraten mit daraus resultierender Gewebsischämie, die wiederum zu einer Beeinträchtigung der Funktion der betroffenen Extremität führt. Die logische Folge davon ist, daß das Zielorgan Skelettmuskel immer mehr in den Vordergrund rückt. Das bedeutet, daß das therapeutische Ziel einer jeden medikamentösen Therapie die Verbesserung oder - im Idealfall - die Wiederherstellung der normalen Leistungsfähigkeit sein muß. Die klinische Wirksamkeit wird ja auch folgerichtig am Menschen mit Hilfe der schmerzfreien Gehstrecke auf dem Laufband ermittelt.

Die Prüfung der erfindungsgemäßen Verbindungen auf ihre die Funktion verbessernde Wirkung erfolgte daher anhand von Messungen der Kontraktionskraft im ischämischen Skelettmuskel mit der unten beschriebenen Versuchsanordnung, wobei das Standardtherapeutikum Pentoxifyllin als Vergleichspräparat in die Untersuchungen miteinbezogen wurde (siehe auch Okyayuz-Baklouti, I., in: Muscle Ischaemia, Functional and Metabolic Aspects, eds I. Okyayuz-Baklouti und O. Hudlicka, Dr. C. Wolf und Sohn, München, p 103-126, 1988; Okyayuz-Baklouti, I., European J. of Pharmacology 166: 75-86, 1989).

Als Versuchstiere dienten männliche Wistar - Ratten mit einem Körpergewicht von 380 bis 410 g. In Hexobarbital Narkose (^{(R)}Evipan - Natrium, 200 mg/kg KG (= Körpergewicht) i.p.) wurde den Tieren eine einseitige Ligatur der rechten Femoralarterie in der Leiste gesetzt. Nach Einstreuen von Penizillin - Sulfonamid - Puder zur antibiotischen Wundversorgung wurde die kleine Operationswunde verschlossen und die Tiere wurden bis zum völligen Erwachen ständig observiert. Eine Woche später begann die Substanzapplikation durch orale Gabe mit einer Schlundsonde (6 mg/kg KG, Carboxymethylcellulose-Natrium-Suspension) und wurde für 7 Tage weitergeführt (Einmalgabe pro Tag, ca 7h30 bis 8h30). Die Kontraktionskraft wurde 24 h nach der letzten Substanzgabe gemessen, um akute Effekte auszuschließen, und zwar nach folgendem Versuchsprotokoll:

Die Tiere wurden mit ^{(R)}Nembutal (Pentobarbital - Natrium, 35 mg/kg KG i.p.) narkotisiert, die Muskeln der betreffenden Extremität freigelegt (Gastrocnemius - Plantaris- Soleus - Gruppe) und die Sehne wurde an einen Kraftaufnehmer (Rhema Z6, Firma Rhema, Hofheim) mit einer Vorlast von 50 g angebunden. Eine Superfusion mit physiologischer Kochsalzlösung (37°C) diente zur Vermeidung von Austrocknung und Abkühlung. Der mittlere arterielle Blutdruck wurde via Statham (= Blutdruckmeßgerät) über eine kanülierte Karotisarterie kontinuierlich zur Kontrolle des physiologischen Status der Tiere während des Versuchs aufgezeichnet. Alle Tiere atmeten spontan durch einen eingeführten Trachealtubus.

Nach diesen Vorbereitungen wurde der Muskel durch direkte elektrische Stimulation ( 2.5 mA, 2 Hz) zur Kontraktion gebracht (Stimulator I, Firma Hugo Sachs, Bundesrepublik Deutschland). Als Meßparameter für die Präparatewirkung diente die absolute Kontraktionskraft in Gramm zu verschiedenen Zeiten der Stimulation. Die initiale Kontraktilität des chronisch - ischämischen Skelettmuskels unterscheidet sich dabei nur unerheblich (Streubreite der Versuche) von der des normalen Muskels. Da aber der unterversorgte Muskel schneller ermüdet, fällt die Kontraktionskraft während des hier gewählten Stimulationsintervals von 5 Minuten signifikant schneller und stärker ab als beim normalen nichtischämischen Muskel. Wenn man nun die maximale Kontraktionskraft am Anfang der Stimulation durch die verbliebene Restkraft nach 5 minütiger ermüdender Stimulation dividiert, kann man einen "Ermüdungs - Index EI" für den betreffenden Muskel errechnen; dabei ist die Ermüdbarkeit um so größer, je größer numerisch EI ist. So bewegt sich der EI für den normalen nur mit dem Vehikel behandelten Muskel je nach Versuch zwischen 1.78 und 3.29 (s. Tabelle 2). Die Ermüdbarkeit des ischämischen Muskels ist um 40 bis 60 % höher.

In Tabelle 2 wurde der EI des normalen Muskels mit dem EI des ischämischen behandelten Muskels verglichen, da dies am eindeutigsten eine eventuelle Verbesserung der Funktion in Richtung Normalisierung widerspiegelt. Dies bedeutet, daß je kleiner der numerische Wert der prozentualen Änderung ist, desto effektiver ist das jeweilige Präparat , d.h. eine prozentuale Änderung mit z.B. negativem Vorzeichen bedeutet sogar eine niedrigere Ermüdbarkeit im Vergleich zum nichtischämischen unbehandelten Muskel. Für jedes Prüfpräparat wurden 4 bis 6 Einzelversuche durchgeführt

### 2) Antithrombotische Aktivität

Ein wichtiger Faktor in der Genese und dem Verlauf von peripheren arteriellen Verschlußkrankheiten und anderen für diese Substanzgruppe in Anspruch genommenen Indikationen sind thrombotische Ereignisse. So wurden die erfindungsgemäßen Verbindungen auf Hemmung von Laser - induzierter Thrombose getestet (vgl. dazu: Seiffge, D. und Kremer, E., Thromb. Res. 42, 331-341, 1986):
Diese Untersuchungen erfolgten an weiblichen Sprague - Dawley - Ratten mit einem Körpergewicht von ca. 200 g. Die Tiere wurden mit 0.1 mg Atropinsulfat s.c. prämediziert und mit 100 mg Ketaminhydrochlorid und 4 mg Xylazin pro kg KG i.p. anästhetisiert. Zur Untersuchung dienten Arteriolen und Venolen des mit entgastem Paraffinöl überschichteten Mesenteriums mit einem Durchmesser von ca. 13 µm. Der Strahl des 4W Argon-Lasers (Firma Spectra-Physics, Darmstadt) wurde mittels einer Strahladaptions - und Justieranlage koaxial in den invertierten Strahlengang eines Mikroskops (ICM 405, LD ^{(R)}Epiplan 40/o.60, Zeiss, Oberkochen) eingebracht. Die benutzte Wellenlänge betrug 514.5 nm mit einer Leistung oberhalb des Objektivs von 30 mW. Die Expositionszeit pro Einzelschuß dauerte 1/15 sec. Alle Meßvorgänge wurden per Videokamera (^{(R)}Trinicon - Röhre, Sony, Köln) aufgenommen und auf einem Recorder (^{(R)}Sony U-matics 3/4" gespeichert. Die Testsubstanzen wurden den Versuchstieren in verschiedenen Dosierungen oral 1 Stunde, bei i.v. Gabe 10 min vor Versuchsbeginn verabreicht; Kontrolltiere erhielten die gleiche Menge des Placebos. Die Applikation der Substanzen erfolgte als Einzelgabe einmal am Tag bzw einmal am Tag über mehrere Tage. Zur Auswertung wurde die Zahl der Laserschüsse gezählt, die benötigt wird, um eine wandständige Thrombose von einer Mindestgröße des halben Gefäßdurchmessers zu erzeugen. Dies bedeutet je größer die Anzahl der Laserschüsse desto wirksamer sind die Präparate in diesem Test. In Tabelle 3 ist die prozentuale Hemmung der Thrombose angegeben.

### 3) Akute Toxizität

Die Bestimmung der LD50 - Bereiche erfolgte standardgemäß über die innerhalb von 7 Tagen bei NMRI - Mäusen nach einmaliger intravenöser (i.v.) oder intraperitonealer (i.p.) Gabe auftretende Mortalität (NMRI = NIH Medical Research Institute). Die Werte sind ebenfalls in Tabelle 3 zusammengefaßt.

### 4) Zusätzliche spezielle Versuche

Die eindeutige Überlegenheit der erfindungsgemäßen Verbindungen insbesondere gegenüber dem am häufigsten zur Therapie peripherer Durchblutungsstörungen therapeutisch eingesetzten Präparat, dem Pentoxifyllin, ließ sich auch in weiteren speziellen Versuchen eindrucksvoll bestätigen.

Ein wichtiger Vorteil der erfindungsgemäßen Substanzen beispielhaft der Verbindung aus Beispiel 7 ist es, daß sie die Thrombose in hyperlipidämischen, spontan hypertensiven und damit zu Schlaganfall neigenden Ratten und in atherosklerotischen Kaninchen hemmen. So hemmt die Substanz aus Beispiel 7 die Laser - induzierte Thrombose nach täglicher Verabreichung von 10 bzw. 30 mg/kg für 7 Tage in hyperlipidämischen Hochdruckratten um 18 bzw. 32 % und in atherosklerotischen Kaninchen nach täglicher Verabreichung von 30 mg/kg für 14 Tage um 36 %.

Weiterhin hemmen die erfindungsgemäßen Substanzen nicht nur die Laser - induzierte Thrombusbildung, sondern darüber hinaus hemmen sie, besonders die Substanz aus Beispiel 7, auch die photochemisch induzierte Thrombose. In diesem Modell wurden Ratten wie oben unter 2) beschrieben anästhetisiert. Die Untersuchungen erfolgten an Mesenterialarteriolen mit einem Durchmesser von 11 bis 50 µm. Die Induktion einer Thrombose wurde in Modifikation entsprechend einer literaturbekannten Methode durchgeführt (Herrmann, K.H., Microvasc. Res. 26, 238 - 249, 1983) und beruht auf der photochemischen Freisetzung von Singulet - Sauerstoff, welcher lokal zur Endothelläsion führt. Die zu untersuchenden Tiere erhielten eine intravenöse Injektion von 0.3 ml einer 10 %igen Lösung von Fluoresceinisothiocyanat - Dextran 70 (FITC - Dextran 70 s, Firma Sigma, München). Die Arteriole wurde unter dem Mikroskop aufgesucht und im Beobachtungsfeld zentriert. Danach wurde das FITC - Dextran im Blutgefäß mit einer speziellen Lampen - und Filtereinrichtung angeregt ( Anregung 490 nm, Emission 510 nm). Zur Auswertung der Thrombusbildung wurde die Zeit genommen, welche von dem Zeitpunkt der Anregung bis zur Bildung eines ersten wandständigen Thrombus verstreicht. In jedem Tier wurden 5 Gefäße auf diese Weise untersucht. Es stellte sich dabei heraus, daß die Thrombose durch die Verbindung aus Beispiel 7 dosisabhängig und statistisch signifikant gehemmt wurde (1, 3, 10 mg / kg p.o.: 69, 75, 130%). Im Vergleich dazu bewirkte Pentoxifyllin nur eine Hemmung von 29, 18, 68% bei 1, 3 und 10 mg/kg p.o. Nach i.v. Verabreichung von der Verbindung aus Beispiel 7 von 3 bzw 10 mg/kg wurde die Thrombose um 36 % bzw 56 % gehemmt; bei i.v. Verabreichung von Pentoxifyllin von 3 bzw 10 mg/kg um 3% bzw 38 %.

Mit Hilfe der Laser - Doppler - Flowmetrie (LDF) kann auf nicht-invasive und kontinuierliche Weise der Erythrozytenflux (Anzahl der unter dem Laserstrahl vorbeifließenden Zellen x ihrer Geschwindigkeit) im Kapillarbett des Skelettmuskels der Ratte gemessen werden (Laser - Doppler - Flowmeter Perimed PF2, Firma Perimed, Schweden). Diese Technik gibt allerdings keine absoluten Werte der Durchblutung sondern qualitative Änderungen in Volt an, wobei das erhaltene Signal aber linear mit dem Flux korreliert. Das Gerät wurde wie folgt eingestellt: 12 kHz, gain 10, Zeitkonstante 1.5 sec, 37°C. Männlichen Wistar - Ratten mit einem Körpergewicht von 380 - 430 g wurden die rechte Femoralarterie freigelegt und über einem kleinen Muskelareal (tibialis anterior) die Haut und das Bindegewebe wegpräpariert. Etwa 1 mm über diesem Areal wurde die Sonde plaziert. Sobald sich die Kurve stabilisiert hatte, wurde die Femoralarterie mit Hilfe einer Klemme okkludiert, worauf die LD - Kurve in dem von diesem Gefäß versorgten Muskel rasch abfiel, dann infolge der spontanen Eröffnung von Kollateralgefäßen wieder geringfügig anstieg und sich schließlich auf einem gegenüber dem Ausgangswert stark erniedrigten Niveau einpendelte ( Restdurchblutung im akut ischämischen Muskel ca 25 %). Zu diesem Zeitpunkt wurden die Prüfsubstanzen in wäßriger Lösung intravenös infundiert (0.03 und 0.6 mg/kg/min). Als Meßparameter für die Substanzwirkung diente der maximale prozentuale Anstieg des Erythrozytenfluxes nach Substanzgabe während der Okklusion (siehe auch Okyayuz-Baklouti, I., European J. of Pharmacology, 166: 75 - 86, 1989). Es stellte sich dabei heraus, daß die Durchblutung in der Mikrozirkulation beispielhaft nach Infusion der Verbindung aus Beispiel 7 stark und dosisabhängig anstieg (+ 53.3 % in der niedrigen und + 80.6 % in der höheren Dosierung). Im Vergleich dazu bewirkte Pentoxifyllin einen Anstieg um 24.6 % in der niedrigen und um 33.1 % in der höheren Dosierung. Pro Präparat und Dosis wurden 3 - 7 Tiere eingesetzt.

Die Kontraktilität am akut ischämischen Muskel wurde in einer ähnlichen Versuchsanordnung, wie unter 1) beschrieben, gemessen. Der zunächst normal durchblutete Muskel wurde durch direkte elektrische Stimulation (1.2 Hz, 2.5 mA) zur isometrischen Kontraktion gebracht. Danach wurde die rechte Femoralarterie mittels einer Klemme für 5 min okkludiert. Die Kontraktionskraft fällt dabei aufgrund der ungenügenden Substratversorgung signifikant ab (Infusion des Vehikels). Das Gefäß wurde dann wiedereröffnet und die Ausgangskontraktilität vor der ersten Okklusion wurde erreicht. Während der nun folgenden zweiten Okklusion wurden die zu testenden Präparate intravenös in wäßriger Lösung über die Jugularvene appliziert. Pro Präparat und Dosis wurden 4 - 8 Tiere eingesetzt. Der Abfall der Kontraktionskraft während der Okklusion mit und ohne Substanzgabe wurde verglichen und die Prozentänderung wurde zur Beurteilung der Substanzwirkung herangezogen. Auch in diesem Test fielen die erfindungsgemäßen Substanzen beispielhaft die Verbindung aus Beispiel 7 durch hervorragende Effekte auf. So war die Kontraktionskraft nach einer Dosis von 0.03 mg/kg/min um +17.2%, und nach einer Dosis von 0.3 mg/kg/min um +25.2 % verbessert, während Pentoxifyllin auch in diesem Funktionstest nur marginale Wirkung zeigte.

So zeigen die erfindungsgemäßen Verbindungen hervorragende Wirkungen auf die Leistungsfähigkeit des ischämischen Muskels sowohl während chronischer als auch während akuter Mangeldurchblutung, sowie günstige Effekte auf die kapilläre Durchströmung gepaart mit einer ausgezeichneten Hemmung der intravaskulären Thrombosierung.

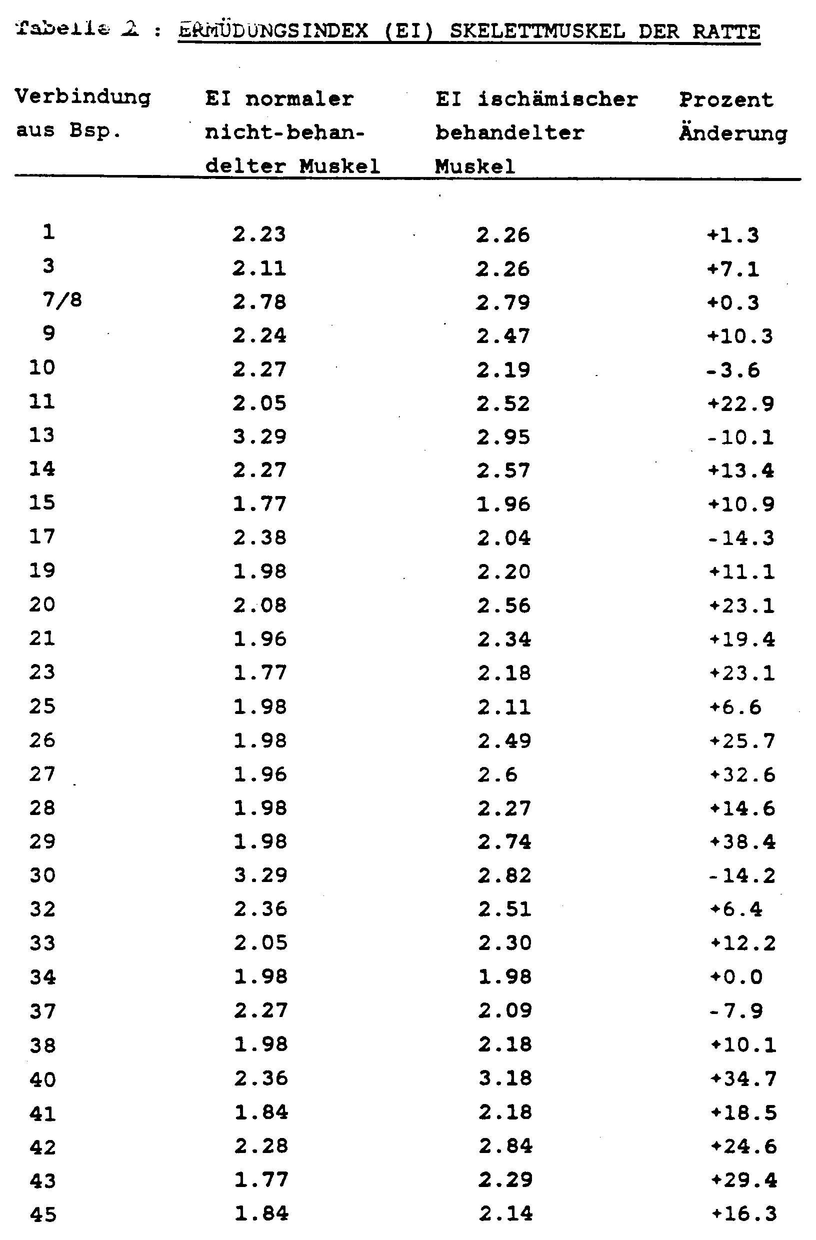

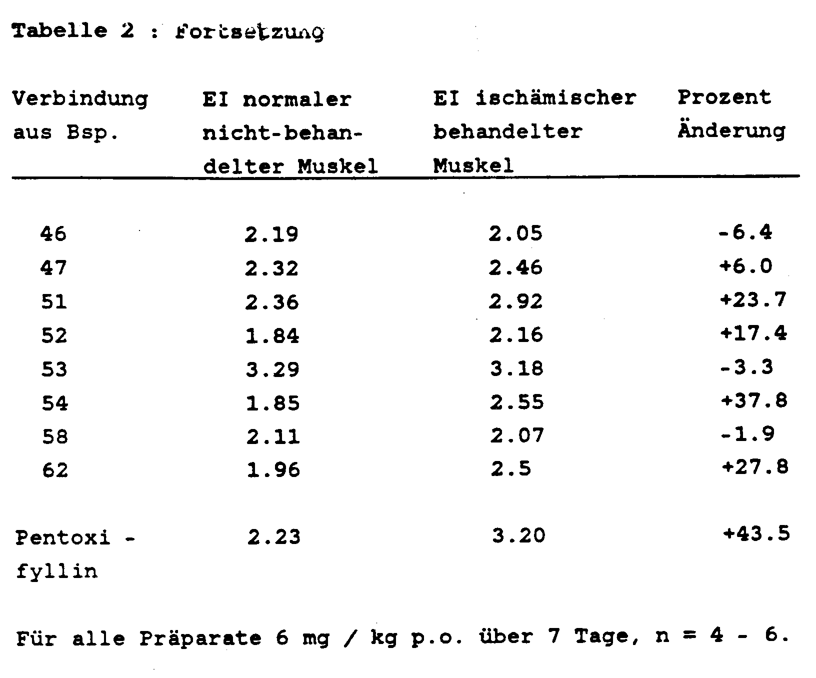

**Tabelle 3**

| HEMMUNG DER LASER - INDUZIERTEN THROMBOSE IN MESENTERIALARTERIOLEN DER RATTE UND TOXIZITÄT | | | |
|---|---|---|---|
| Verbindung | Dosis (mg/kg p.o.) | Prozentuale Hemmung vs Kontrolle | Toxizität (LD₅₀-Bereich) mg/kg |
| 1 | 10 | 13 | > 200 i.v. |
| 3 | 20 | 23 | |
| 7/8 | 10 | 36 | > 100 i.v. |
| 10 | 10 | 38 | > 100 i.v. |
| 12 | 20 | 24 | > 100 i.v. |
| 13 | 20 | 14 | |
| 14 | 20 | 13 | > 100 i.v. |
| 21 | 20 | 24 | |
| 25 | 10 | 22 | > 300 i.p. |
| 26 | 10 | 24 | |
| 27 | 20 | 23 | > 100 i.v. |
| 29 | 10 | 21 | > 100 i.v. |
| 30 | 10 | 45 | > 100 i.v. |
| 32 | 10 | 21 | > 200 i.v. |
| 33 | 10 | 18 | |
| 34 | 10 | 15 | > 100 i.v. |
| 38 | 10 | 21 | |
| 40 | 10 | 17 | > 1200 i.p. |
| 43 | 20 | 15 | |
| 46 | 10 | 29 | > 1200 i.p. |
| 46 | 30 | 29 | |
| 53 | 10 | 18 | > 100 i.v. |
| 54 | 10 | 17 | > 1200 i.p. |
| 56 | 10 | 24 | > 100 i.v. |
| 57 | 10 | 19 | |

## Patentansprüche (Patentansprüche für folgende(n) Vertragsstaat(en): AT, BE, CH, DE, DK, FR, GB, IT, LI, LU, NL, SE)

1. Imidazolverbindungen der Formel I 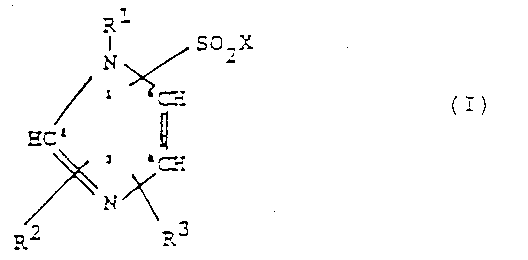 in welcher
R¹ ist (C₁-C₅)-Alkyl,
R², R³ sind gleich oder verschieden und jeweils
H
Halogen (F, Cl, Br, J) oder (C₁-C₃)-Alkyl,
X ist OH
oder eine Aminogruppe der Formel II 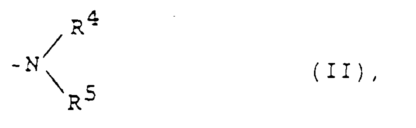 worin
R⁴ is H oder (C₁-C₇)-Alkyl,
gegebenenfalls substituiert mit CN, NH₂ oder COOH,
R⁵ ist (C₁-C₈)-Alkylrest,
bei dem - wenn er mehr als 1 C-Atom besitzt - zwischen 2 C-Atomen auch ein Phenylrest stehen kann
und dessen (aliphatische) C-Atome substituiert sind mit 1 oder mehreren der folgenden Gruppen: OH,
(C₁-C₃)-Alkoxy,
Phenyl, gegebenenfalls substituiert mit 1 - 3 OH-, (C₁-C₃)-Alkylgruppen, (C₁-C₃)-Alkoxy-COOH, und/oder (C₁-C₃)-Alkoxy-(C₁-C₄)-Alkyl
COOH,
COO(C₁-C₃)-Alkyl,
CONH₂,
CN
(C₂-C₅)-Alkinyl,
NH₂,
NHR⁶
worin R⁶ gleich oder verschiedene
N(R⁶)₂
Reste der Art (C₁-C₄)-Alkyl,
N^{⊕}(R⁶)₃
(C₂-C₆)-Alkoxyalkyl und Phenylalkyl
mit 1 - 3 C-Atomen im Alkylteil
bedeutet,
NH-CO-(C₁-C₆)-Alkyl, 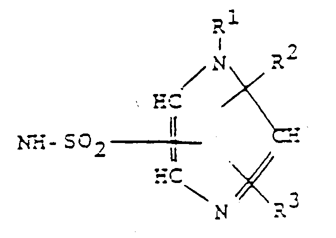
(Bedeutung von R¹, R² und R³ siehe vorstehend),
monocyclische 5- bis 7-gliedrige gesättigte oder ungesättigte heterocyclische Reste mit 1 N-Atom sowie gegebenenfalls noch einem zusätzlichen N-, O- oder S-Atom im Ring, aus der Gruppe Thiomorpholin, Piperazin, Morpholin, Imidazol, Pyrrolidin und Piperidin,
gegebenenfalls
substituiert mit
(C₁-C₃)-Alkyl,
Phenyl,
Phenylalkyl mit 1 - 3 C-Atomen im Alkylteil,
OH, und/oder Oxo (= O), einschließlich der offenen und cyclischen Ketalformen mit 2 - 6 C-Atomen im Ketalteil,
und worin das Ring-S-Atom - falls vorhanden - auch zur Sulfoxid(SO)- oder Sulfon(SO₂)-Form oxidiert sein kann
oder worin
R⁴ und R⁵ zusammen mit dem Amid-N-Atom, an das sie gebunden sind, einen gesättigten 5- bis 7-gliedrigen heterocyclischen Ring bilden, aus der Gruppe Piperazin und Thiomorpholin,
und der heterocyclische Ring ansonsten substituiert sein kann mit folgenden Gruppen:
(C₁-C₃)-Alkoxy,
Phenylalkyl mit 1 - 4 C-Atomen im Alkylteil,
Phenyl, gegebenenfalls substituiert mit 1 oder mehreren der Gruppe:
(C₁-C₃)-Alkyl,
OH,
(C₁-C₃)-Alkoxy,
(C₁-C₃)-Alkoxy-COOH,
(C₁-C₃)-Alkoxy-COO(C₁-C₄)alkyl, 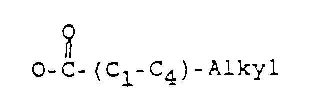
O-SO₂-C₆H₅
O-SO₂-C₆H₄CH₃, 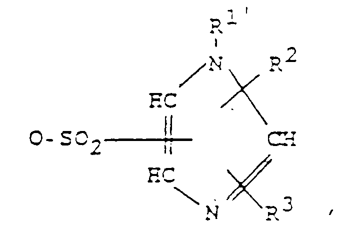
worin R^{1'}
die gleiche Bedeutung wie R¹ besitzt und zusätzlich noch H sein kann, und R² und R³
die vorerwähnte Bedeutung besitzen und das Ring-S-Atom - falls vorhanden - auch zur Sulfoxid(SO)- oder Sulfon(SO₂)-Form oxidiert sein kann, sowie deren physiologisch verträglichen Salze.

2. Imidazolverbindungen gemäß Anspruch 1, dadurch gekennzeichnet, daß in Formel I mindestens eines der folgenden Merkmale vorliegt:
a) R¹ = CH₃ oder C₂H₅,
b) R², R³ = gleich oder verschieden und jeweils H, Cl oder CH₃, und
c) der -SO₂X-Rest sich in 2- oder 4-Stellung des Imidazolrings befindet.

3. Imidazolverbindungen nach Anspruch 1 oder 2, dadurch gekennzeichnet, daß der Rest 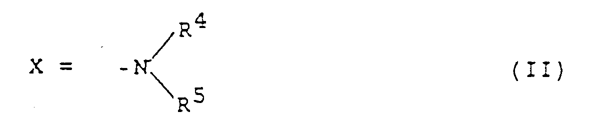 mit R⁴ =
H und
R⁵ =
(C₂-C₅)-Alkylrest, in welchem gegebenenfalls zwischen 2 C-Atomen ein Phenylrest steht und dessen (aliphatische) C-Atome mit insgesamt 1 oder 2 der folgenden Gruppen
substituiert sind:
Hydroxyphenyl C₆H₄OH
CN
(C₂-C₃)-Alkinyl
NH₂ 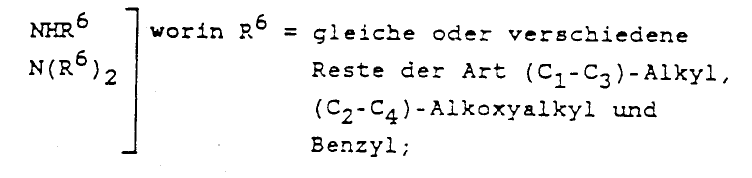 monocyclischer 5- bis 6-gliedriger gesättigter heterocyclischer Rest aus der Gruppe: 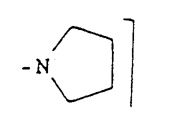 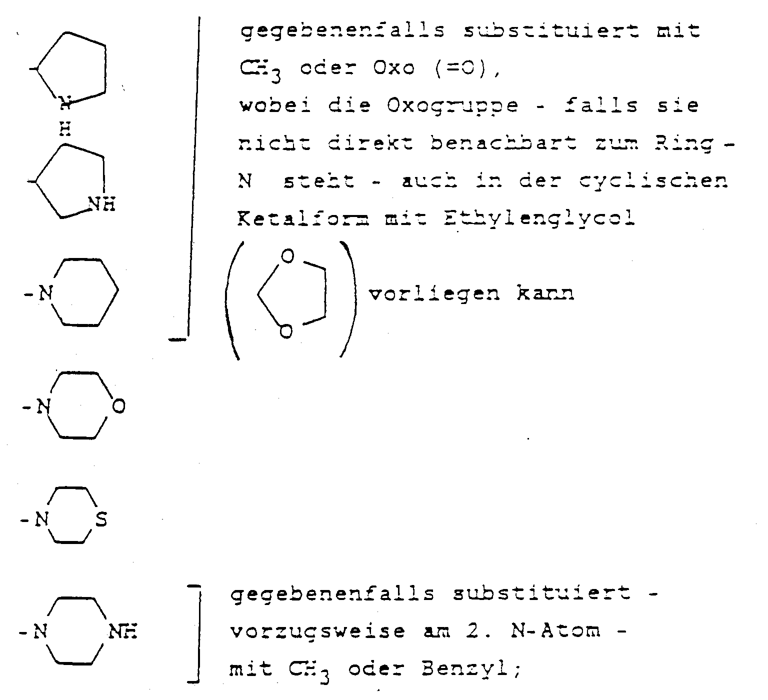 oder R⁴ oder R⁵ zusammen mit dem Amid-N-Atom, an das sie gebunden sind, einen gesättigten 6-gliedrigen heterocyclischen Ring bilden der Art 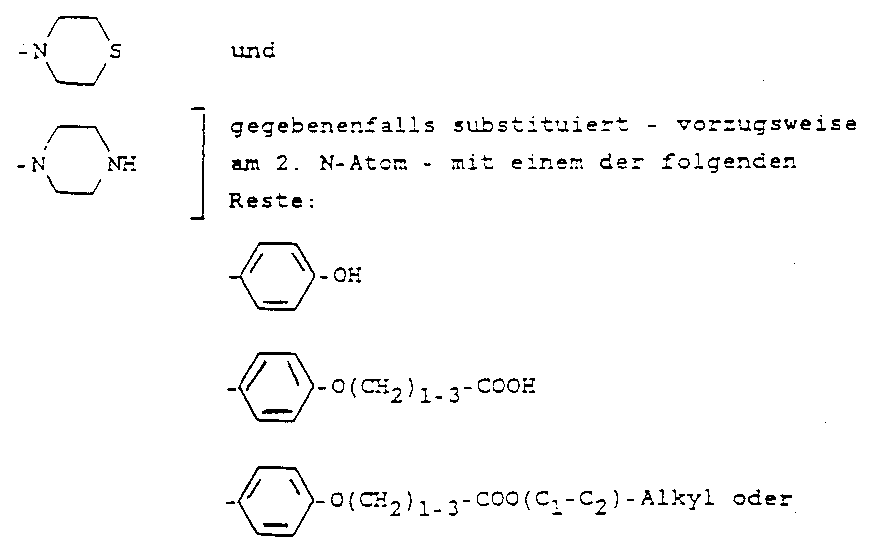 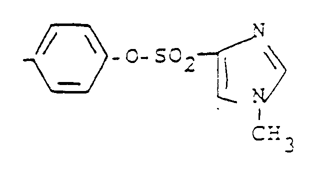 sowie deren physiologisch verträglichen Salze

4. N-(2-Morpholinoethyl)-1-methyl-2-imidazolsulfonsäureamid = Verbindung der Formel I gemäß Anspruch 1, worin
R¹ = CH₃
R² = R³ = H,
SO₂X-Gruppe in 2-Position und 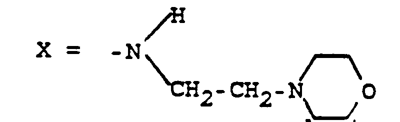 sowie deren physiologisch verträglichen Salze.

5. N-(3-Morpholinopropyl)-1-methyl-4-imidazolsulfonsäureamid = Verbindung der Formel I gemäß Anspruch 1, worin
R¹ = CH₃,
R² = R³ = H,
SO₂X-Gruppe in 4-Position und 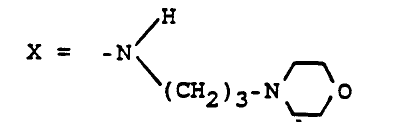 sowie deren physiologisch verträglichen Salze.

6. Verfahren zur Herstellung der Imidazolverbindungen der Formel I gemäß der Definition in einem oder mehreren der Ansprüche 1 - 5 und gegebenenfalls ihrer physiologisch verträglichen Salze, dadurch gekennzeichnet, daß man
a) ein Imidazolderivat der allgemeinen Formel III 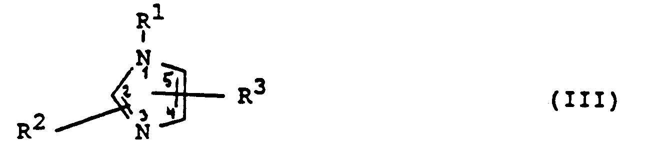 in der R¹, R² und R³ die bei Formel I genannte Bedeutung haben und eine der Stellungen 4 und 5 unsubstituiert ist, durch Sulfonierung in die Verbindungen der Formel Ia umwandelt, 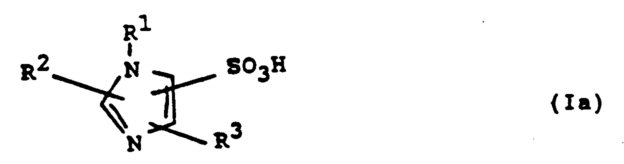 worin R¹, R² und R³ ebenfalls die bei Formel I genannte Bedeutung besitzen,
oder
b) ein Imidazolderivat der allgemeinen Formel IV 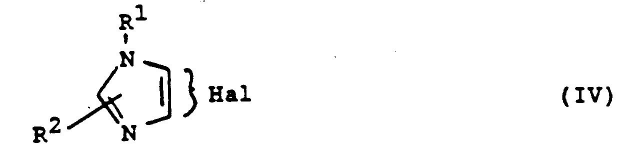 in der R¹ und R² die bei Formel I genannte Bedeutung haben, eine der Stellungen 4 und 5 einen Halogensubstituenten Hal (Cl, Br, J) trägt und die andere unsubstituiert ist, durch Sulfonierung und anschließende hydrogenolytische Enthalogenierung in eine Verbindung der Formel Ib umwandelt 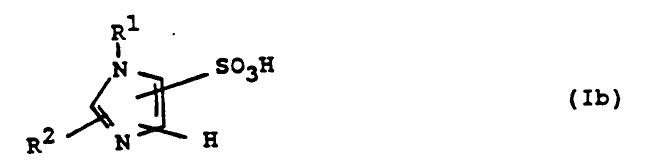 (Bedeutung von R¹ und R² wie bei Formel I)
oder
c) ein Imidazolderivat der allgemeinen Formel V 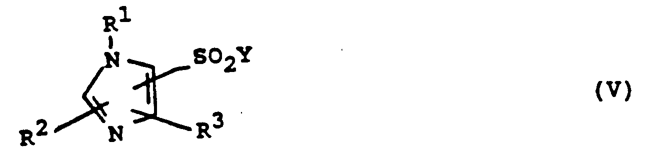 in der R¹, R² und R³ die bei Formel I genannte Bedeutung haben und Y für Halogen, vorzugsweise Chlor, steht, zu den Imidazolsulfonsäuren der Formel Ia mit den dort genannten Bedeutungen für R¹, R² und R³ hydrolysiert,
oder
d) ein Imidazolderivat der allgemeinen Formel VI oder VI' 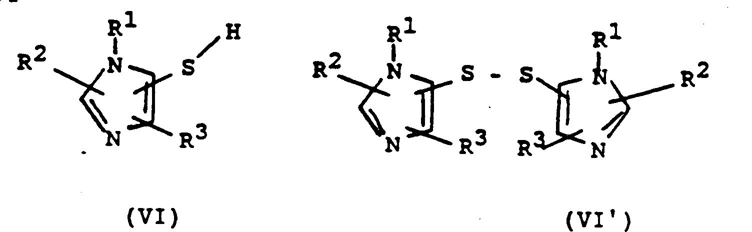 in der R¹, R² und R³ die bei Formel I genannte Bedeutung haben, zu der entsprechenden Imidazolsulfonsäure (Formel Ia) oxidiert oder
e) ein Imidazolsulfonsäurehalogenid der allgemeinen Formel V (siehe Variante c), mit einem Amin der Formel H-II 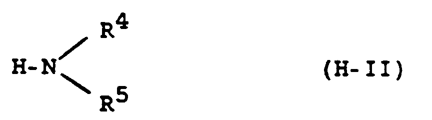 in der R⁴ und R⁵ die gleiche Bedeutung wie in Formel II (siehe Anspruch 1) haben, zu den Sulfonamiden der Formel Ic umsetzt 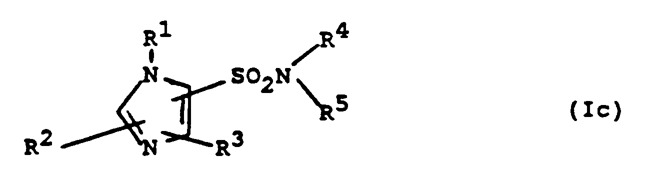 worin R¹ bis R⁵ die bei den Formeln I und II genannten Bedeutungen besitzen,
oder
f) Imidazolderivate der allgemeinen Formel VII 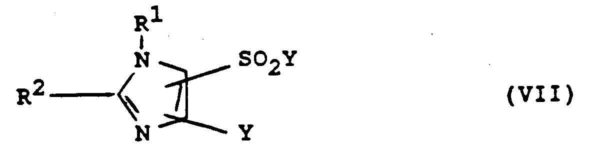 worin R¹ und R² die bei Formel I genannte Bedeutung haben und Y gleiche oder verschiedene Halogenatome (Cl, Br, J) bedeutet, mit Aminen der Formel H-II umsetzt und anschließend der hydrogenolytischen Enthalogenierung unterwirft, um die in 4- oder 5-Stellung unsubstituierten Sulfonamide der Formel Id mit den in Anspruch 1 für R¹ und R² angegebenen Bedeutungen zu erhalten 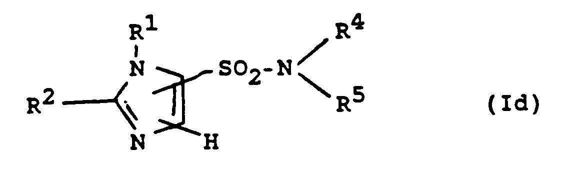 (Bedeutung von R¹, R², R⁴ und R⁵ wie in Formeln I und II),
oder
g) ein Imidazolsulfonylhalogenid der allgemeinen Formel V (siehe Variante c) mit einem Trialkylsilylamin der Formel VIII 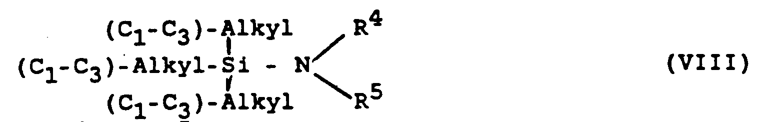 wobei R⁴ und R⁵ die bei Formel II genannte Bedeutung haben, und bevorzugter (C₁-C₃)-Alkylrest der Methylrest ist, zur Umsetzung bringt,um die Sulfonamide der Formel Ic (siehe Variante e) zu erhalten,
oder
h) zur Herstellung von Verbindungen der Formel I - worin X = -N(R⁴)R⁵ (Formel II) und R⁴ mindestens 1 NH₂-Gruppe und/oder R⁵ mindestens eine primäre oder sekundäre Aminogruppe trägt, Amine der Formel
H-N(R⁴)R⁵,
die an ihren Resten R⁴ und/oder R⁵ mindestens eine N-geschützte, - bevorzugt N-benzylierte - entsprechende Aminogruppe tragen, mit Imidazolsulfonylhalogeniden der Formel V (siehe Variante c) umsetzt und die entstandenen Sulfonamide anschließend von der Schutzgruppe befreit,
oder
i) zur Herstellung von Verbindungen der allgemeinen Formel I, worin X = -N(R⁴)R⁵ (Formel II) und wenigstens einer der Reste R⁴ und R⁵ mindestens 1 primäre Aminogruppe trägt, Imidazolsulfonylhalogenide der Formel V (siehe Variante c) mit entsprechenden Aminonitrilen umsetzt und die so erhaltenen Sulfonylaminonitrile zu den Aminoverbindungen reduziert,
oder
j) zur Herstellung von Imidazolverbindungen der allgemeinen Formel I, worin X = -N(R⁴)R⁵ und R⁵ mindestens eine quartäre Aminogruppe -N^{⊕}(R⁶)₃ trägt, in der die Reste R⁶ gleich oder verschieden sein können, eine Imidazolverbindung der Formel I, worin X = -N(R⁴)R⁵ und R⁵ mindestens eine tertiäre Aminogruppe -N(R⁶)₂ trägt, mit einem Alkylierungsmittel alkyliert,
oder
k) zur Herstellung von Imidazolverbindungen der allgemeinen Formel I, worin X = -N(R⁴)R⁵ und R⁵ oder R⁴ und R⁵ zusammen eine oder mehrere Sulfoxid- oder Sulfongruppen enthalten, Imidazolverbindungen der allgemeinen Formel I, worin X = -N(R⁴)R⁵ und R⁵ oder R⁴ und R⁵ zusammen eine oder mehrere Sulfidgruppen enthalten, zu den entsprechenden Sulfoxiden oder Sulfonen oxidiert,
oder
l) zur Herstellung von Imidazolverbindungen der Formel I, worin X = -N(R⁴)R⁵ und R⁵ oder R⁴ und R⁵ zusammen eine Phenolethergruppe enthalten, Imidazolverbindungen der allgemeinen Formel I, in der X = -N(R⁴)R⁵ und R⁵ oder R⁴ und R⁵ zusammen mindestens einen - mit einer oder mehreren phenolischen Hydroxylgruppen substituierten - Arylrest tragen, mit Alkylierungsreagenzien, bevorzugt ω-Halogenfettsäurederivaten, zu den entsprechenden Phenolethern alkyliert, die im Falle von ω-Fettsäureestern weiterhin noch der Hydrolyse oder Aminolyse zu den entsprechenden Carbonsäuren bzw. Carbonsäureamiden unterworfen werden können,
oder
m) zur Herstellung von Imidazolverbindungen der Formel I, worin X = -N(R⁴)R⁵ und R⁵ oder R⁴ und R⁵ zusammen eine Phenolestergruppe enthalten, die gleichen Ausgangs-Imidazolverbindungen wie für Variante l mit Acylierungsagenzien - vorzugsweise mit (C₁-C₄)-Alkylcarbonsäurechloriden, Benzol- und Toluolsulfonsäurechloriden sowie Imidazolsulfonsäurehalogeniden der allgemeinen Formel V (siehe Variante c), in der R¹, R² und R³ die gleiche Bedeutung wie in Formel I besitzen und R¹ zusätzlich noch Wasserstoff bedeuten kann - zu den entsprechenden Phenolestern umsetzt,
und die bei allen Verfahrensvarianten gebildeten Verbindungen der Formel I - sofern diese nicht bereits in Form von physiologisch verträglichen Salzen anfallen - gegebenenfalls noch in solche umwandelt.

7. Verbindungen der Formel I sowie ihrer physiologisch verträglichen Salze gemäß einem oder mehreren der Ansprüche 1 - 5 oder wie erhalten nach dem Verfahren gemäß Anspruch 6 zur Anwendung als Heilmittel.

8. Arzneimittel, gekennzeichnet durch einen Gehalt an mindestens einer Verbindung der Formel I gemäß der Definition in einem oder mehreren der Ansprüche 1 - 5 und/oder mindestens einem von deren physiologisch verträglichen Salzen und/oder mindestens einer nach dem Verfahren gemäß Anspruch 6 hergestellten Verbindung.

9. Arzneimittel nach Anspruch 8, dadurch gekennzeichnet, daß sie zur Prophylaxe und/oder Therapie von Durchblutungsstörungen, insbesondere von Störungen der Mikrozirkulation, sowie der daraus resultierenden Erkrankungen bestimmt sind.

10. Verfahren zur Herstellung der Arzneimittel gemäß Anspruch 8 oder 9, dadurch gekennzeichnet, daß man mindestens eine Verbindung der Formel I gemäß der Definition in einem oder mehreren der Ansprüche 1 - 5 und/oder mindestens eines von deren physiologisch verträglichen Salzen und/oder mindestens eine nach dem Verfahren gemäß Anspruch 6 erhaltenen Verbindung mit üblichen Träger- sowie gegebenenfalls Zusatz- und Hilfsstoffen in eine geeignete Darreichungsform bringt.

11. Verwendung der Verbindungen der Formel I gemäß der Definition in einem oder mehreren der Ansprüche 1 - 5 und/oder mindestens einer nach dem Verfahren gemäß Anspruch 6 erhaltenen Verbindung zur Prophylaxe und/oder Therapie von Durchblutungsstörungen, insbesondere von Störungen der Mikrozirkulation, sowie der daraus resultierenden Erkrankungen.

12. 1-Methyl-,
1,2-Dimethyl- und
1-Ethyl-4-imidazolsulfonsäurechlorid.

13. Verfahren zur Herstellung der in Anspruch 11 genannten Verbindungen, dadurch gekennzeichnet, daß man
a) 1-Methyl- bzw. 1,2-dimethyl- bzw. 1-Ethyl-imidazol mit Chlorsulfonsäure ClSO₃H, gegebenenfalls unter nachträglichem Zusatz von SOCl₂, zur Reaktion bringt, oder daß man
b) 1-Methyl- bzw. 1,2-Methyl- bzw. 1-Ethyl-4-mercaptoimidazol mit Cl₂ oxidierend chloriert.

## Patentansprüche (Patentansprüche für folgende(n) Vertragsstaat(en): ES, GR)

1. Verfahren zur Herstellung der Imidazolverbindungen der Formel I 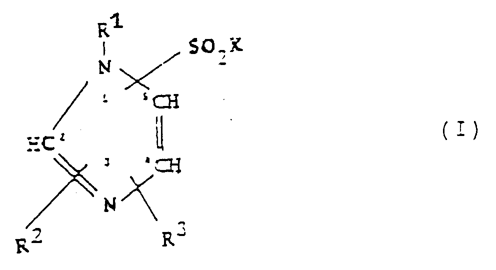 in welcher
R¹ ist (C₁-C₆)-Alkyl,
R², R³ sind gleich oder verschieden und jeweils H,
Halogen (F, Cl, Br, J) oder (C₁-C₃)-Alkyl,
X ist OH oder eine Aminogruppe der Formel II 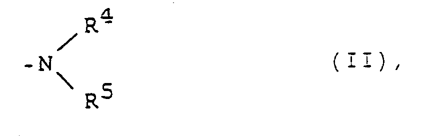
worin
R⁴ ist H oder
(C₁-C₇)-Alkyl,
gegebenenfalls substituiert mit CN, NH₂ oder COOH,
R⁵ ist (C₁-C₈)-Alkylrest,
bei dem - wenn er mehr als 1 C-Atom besitzt - zwischen 2 C-Atomen auch ein Phenylenrest stehen kann
und dessen (aliphatische) C-Atome substituiert sind mit 1 oder mehreren der folgenden Gruppen: OH,
(C₁-C₃)-Alkoxy,
Phenyl, gegebenenfalls substituiert mit 1 - 3 OH-, (C₁-C₃)-Alkylgruppen, (C₁-C₃)-Alkoxy-COOH, und/oder (C₁-C₃)-Alkoxy-(C₁-C₄)-Alkyl
COOH,
COO(C₁-C₃)-Alkyl,
CONH₂,
CN
(C₂-C₅)-Alkinyl,
NH₂, 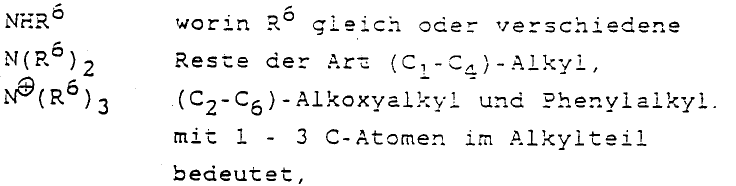
NH-CO-(C₁-C₆)-Alkyl, 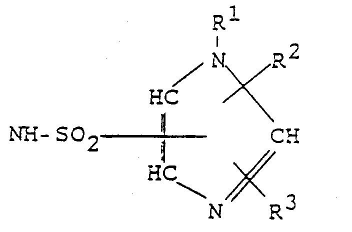 (Bedeutung von R¹, R² und R³ siehe vorstehend), monocyclische 5- bis 7-gliedrige gesättigte oder ungesättigte heterocyclische Reste mit 1 N-Atom sowie gegebenenfalls noch einem zusätzlichen N-, O- oder S-atom im Ring aus der Gruppe Thiomorpholin, Piperazin, Morpholin, Imidazol, Pyrrolidin und Piperidin,
gegebenenfalls
substituiert mit (C₁-C₃)-Alkyl,
Phenyl,
Phenylalkyl mit 1 - 3 C-Atomen
im Alkylteil,
OH, und/oder
Oxo (=O), einschließlich der
offenen und cyclischen
Ketalformen mit 2 - 6 C-Atomen
im Ketalteil,
und worin das Ring-S-Atom - falls vorhanden - auch zur Sulfoxid(SO)- oder Sulfon(SO₂)-Form oxidiert sein kann
oder worin
R⁴ und R⁵ zusammen mit dem Amid-N-Atom, an das sie gebunden sind, einen 5- bis 7-gliedrigen heterocyclischen Ring aus der Gruppe Piperazin und Thiomorpholin bilden
und der heterocyclische Ring ansonsten substituiert sein kann mit folgenden Gruppen:
(C₁-C₃)-Alkoxy,
Phenylalkyl mit 1 - 4 C-Atomen im Alkylteil,
Phenyl, gegebenenfalls substituiert mit 1 oder mehreren der Gruppen:
(C₁-C₃)-Alkyl,
OH,
(C₁-C₃)-Alkoxy,
(C₁-C₃)-Alkoxy-COOH,
(C₁-C₃)-Alkoxy-COO(C₁-C₄)alkyl, 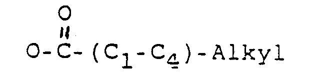 O-SO₂-C₆H₅
O-SO₂-C₆H₄CH₃, 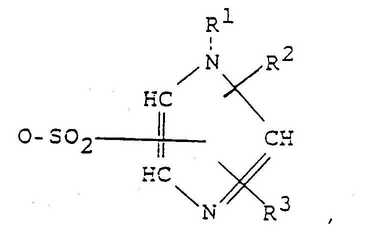 worin R¹' die gleiche Bedeutung wie R¹ besitzt und zusätzlich noch H sein kann, und
R² und R³ die vorerwähnte Bedeutung besitzen
und das Ring-S-Atom - falls vorhanden - auch zur Sulfoxid(SO)- oder Sulfon(SO₂)-Form oxidiert sein kann, sowie deren physiologisch verträglichen Salze, dadurch gekennzeichnet, daß man
a) ein Imidazolderivat der allgemeinen Formel III 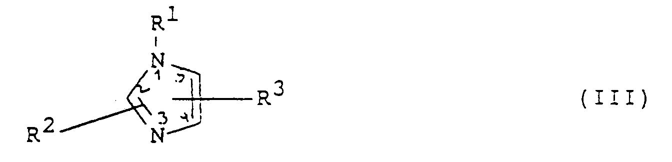 in der R¹, R² und R³ die bei Formel I genannte Bedeutung haben und eine der Stellungen 4 und 5 unsubstituiert ist, durch Sulfonierung in die Verbindungen der Formel Ia umwandelt, 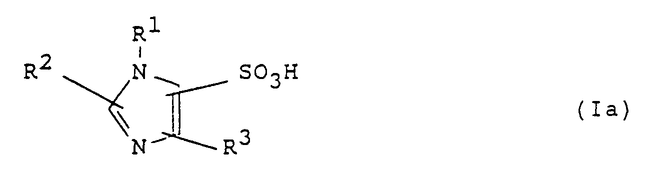 worin R¹, R² und R³ ebenfalls die bei Formel I genannte Bedeutung besitzen,
oder
b) ein Imidazolderivat der allgemeinen Formel IV 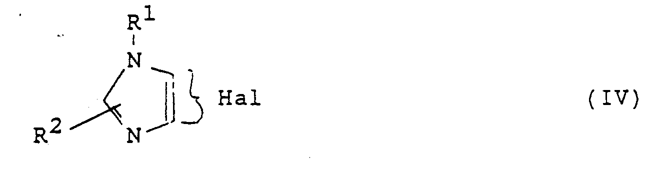 in der R¹ und R² die bei Formel I genannte Bedeutung haben, eine der Stellungen 4 und 5 einen Halogensubstituenten Hal (Cl, Br, J) trägt und die andere unsubstituiert ist, durch Sulfonierung und anschließende hydrogenolytische Enthalogenierung in eine Verbindung der Formel Ib umwandelt 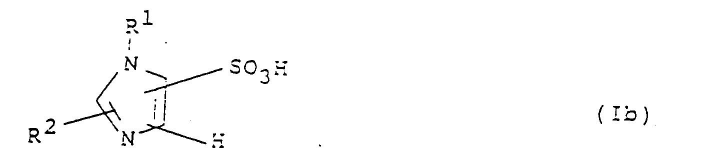 (Bedeutung von R¹ und R² wie bei Formel I)
oder
c) ein Imidazolderivat der allgemeinen Formel V 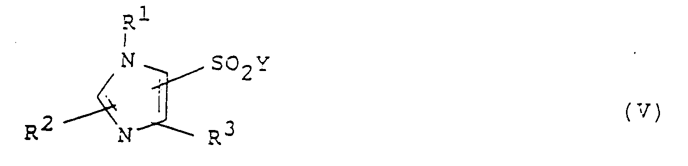 in der R¹, R² und R³ die bei Formel I genannte Bedeutung haben und Y für Halogen steht, zu den Imidazolsulfonsäuren der Formel Ia mit den dort genannten Bedeutungen für R¹, R² und R³ hydrolysiert, oder
d) ein Imidazolderivat der allgemeinen Formel VI oder VI' 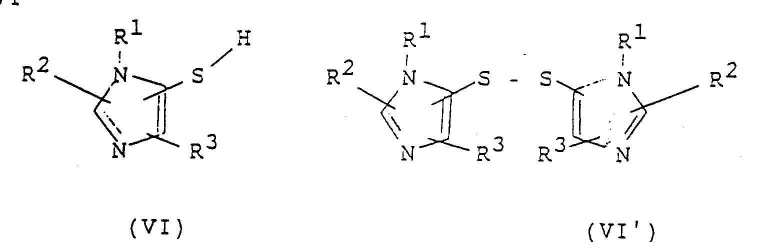 in der R¹, R² und R³ die bei Formel I genannte Bedeutung haben, zu der entsprechenden Imidazolsulfonsäure (Formel Ia) oxidiert
oder
e) ein Imidazolsulfonsäurehalogenid der allgemeinen Formel V (siehe Variante c), mit einem Amin der Formel H-II 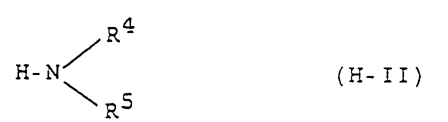 in der R⁴ und R⁵ die gleiche Bedeutung wie in Formel II haben, zu den Sulfonamiden der Formel Ic umsetzt 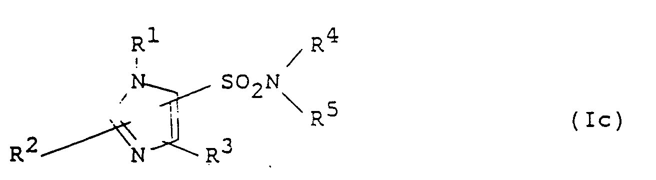 worin R¹ bis R⁵ die bei den Formeln I und II genannten Bedeutungen besitzen,
oder
f) Imidazolderivate der allgemeinen Formel VII 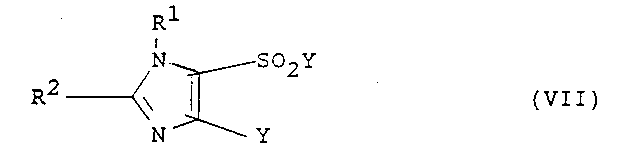 worin R¹ und R² die bei Formel I genannte Bedeutung haben und Y gleiche oder verschiedene Halogenatome (Cl, Br, J) bedeutet, mit Aminen der Formel H-II umsetzt und anschließend der hydrogenolytischen Enthalogenierung unterwirft, um die in 4- oder 5-Stellung unsubstituierten Sulfonamide der Formel Id mit den in Anspruch 1 für R¹ und R² angegebenen Bedeutungen zu erhalten 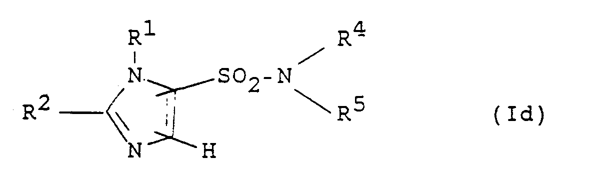 (Bedeutung von R¹, R², R⁴ und R⁵ wie in Formeln I und II),
oder
g) ein Imidazolsulfonylhalogenid der allgemeinen Formel V (siehe Variante c) mit einem Trialkylsilylamin der Formel VIII 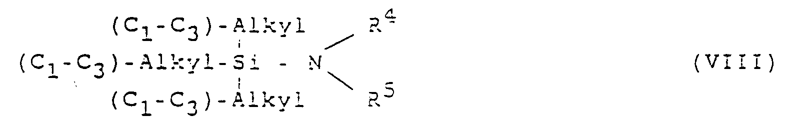 wobei R⁴ und R⁵ die bei Formel II genannte Bedeutung haben, und bevorzugter (C₁-C₃)-Alkylrest der Methylrest ist, zur Umsetzung bringt um die Sulfonamide der Formel Ic (siehe Variante e) zu erhalten,
oder
h) zur Herstellung von Verbindungen der Formel I - worin X = -N(R⁴)R⁵ (Formel II) und R⁴ mindestens 1 NH₂-Gruppe und/oder R⁵ mindestens eine primäre oder sekundäre Aminogruppe trägt, Amine der Formel
H-N(R⁴)R⁵,
die an ihren Resten R⁴ und/oder R⁵ mindestens eine N-geschützte
entsprechende Aminogruppe tragen, mit Imidazolsulfonylhalogeniden der Formel V (siehe Variante c) umsetzt und die entstandenen Sulfonamide anschließend von der Schutzgruppe befreit,
oder
i) zur Herstellung von Verbindungen der allgemeinen Formel I, worin X = -N(R⁴)R⁵ (Formel II) und wenigstens einer der Reste R⁴ und R⁵ mindestens 1 primäre Aminogruppe trägt, Imidazolsulfonylhalogenide der Formel V (siehe Variante c) mit entsprechenden Aminonitrilen umsetzt und die so erhaltenen Sulfonylaminonitrile zu den Aminoverbindungen reduziert,
oder
j) zur Herstellung von Imidazolverbindungen der allgemeinen Formel I, worin X = -N(R⁴)R⁵ und R⁵ mindestens eine quartäre Aminogruppe -N^{⊕}(R⁶)₃ trägt, in der die Reste R⁶ gleich oder verschieden sein können, eine Imidazolverbindung der Formel I, worin X = -N(R⁴)R⁵ und R⁵ mindestens eine tertiäre Aminogruppe -N(R⁶)₂ trägt, mit einem Alkylierungsmittel alkyliert,
oder
k) zur Herstellung von Imidazolverbindungen der allgemeinen Formel I, worin X = -N(R⁴)R⁵ und R⁵ oder R⁴ und R⁵ zusammen eine oder mehrere Sulfoxid- oder Sulfongruppen enthalten, Imidazolverbindungen der allgemeinen Formel I, worin X = -N(R⁴)R⁵ und R⁵ oder R⁴ + R⁵ zusammen eine oder mehrere Sulfidgruppen enthalten, zu den entsprechenden Sulfoxiden oder Sulfonen oxidiert,
oder
l) zur Herstellung von Imidazolverbindungen der Formel I, worin X = -N(R⁴)R⁵ und R⁵ oder R⁴ und R⁵ zusammen eine Phenolethergruppe enthalten, Imidazolverbindungen der allgemeinen Formel I, in der X = -N(R⁴)R⁵ und R⁵ oder R⁴ + R⁵ zusammen mindestens einen - mit einer oder mehreren phenolischen Hydroxylgruppen substituierten - Arylrest tragen, mit Alkylierungsreagentien, bevorzugt ω-Halogenfettsäurederivaten, zu den entsprechenden Phenolethern alkyliert, die im Falle von ω-Fettsäureestern weiterhin noch der Hydrolyse oder Aminolyse zu den entsprechenden Carbonsäuren bzw. Carbonsäureamiden unterworfen werden können,
oder
m) zur Herstellung von Imidazolverbindungen der Formel I, worin X = -N(R⁴)R⁵ und R⁵ oder R⁴ + R⁵ zusammen eine Phenolestergruppe enthalten, die gleichen Ausgangs-Imidazolverbindungen wie für Variante l mit Acylierungsagentien wie (C₁-C₄)-Alkylcarbonsäurechloriden, Benzol- und Toluolsulfonsäurechloriden sowie Imidazolsulfonsäurehalogeniden der allgemeinen Formel V (siehe Variante c), in der R¹, R² und R³ die gleiche Bedeutung wie in Formel I besitzen und R¹ zusätzlich noch Wasserstoff bedeuten kann - zu den entsprechenden Phenolestern umsetzt,
und die bei allen Verfahrensvarianten gebildeten Verbindungen der Formel I - sofern diese nicht bereits in Form von physiologisch verträglichen Salzen anfallen - gegebenenfalls noch in solche umwandelt.

2. Verfahren nach Anspruch 1, dadurch gekennzeichnet, daß man eine Verbindung der Formel I in der mindestens eines der folgenden Merkmale vorliegt:
a) R¹ = CH₃ oder C₂H₅,
b) R², R³ = gleich oder verschieden und jeweils H, Cl oder CH₃,
und
c) der -SO₂X-Rest sich in 2- oder 4-Stellung des Imidazolrings befindet, herstellt.

3. Verfahren nach Anspruch 1 oder 2, dadurch gekennzeichnet, daß man eine Verbindung der Formel I worin der Rest 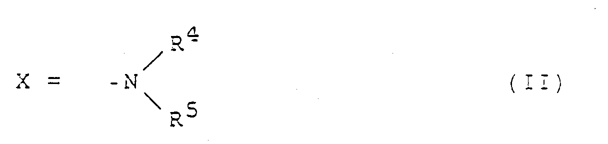 mit
R⁴ = H und
R⁵ = (C₂-C₅)-Alkylrest, in welchem gegebenenfalls zwischen 2 C-Atomen ein Phenylenrest steht und dessen (aliphatische) C-Atome mit insgesamt 1 oder 2 der folgenden
Gruppen substituiert sind:
Hydroxyphenyl C₆H₄OH
CN
(C₂-C₃)-Alkinyl
NH₂ 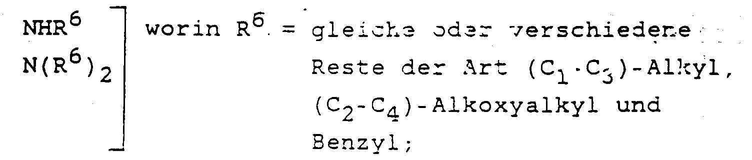 monocyclischer 5- bis 6-gliedriger gesättigter heterocyclischer Rest aus der Gruppe: 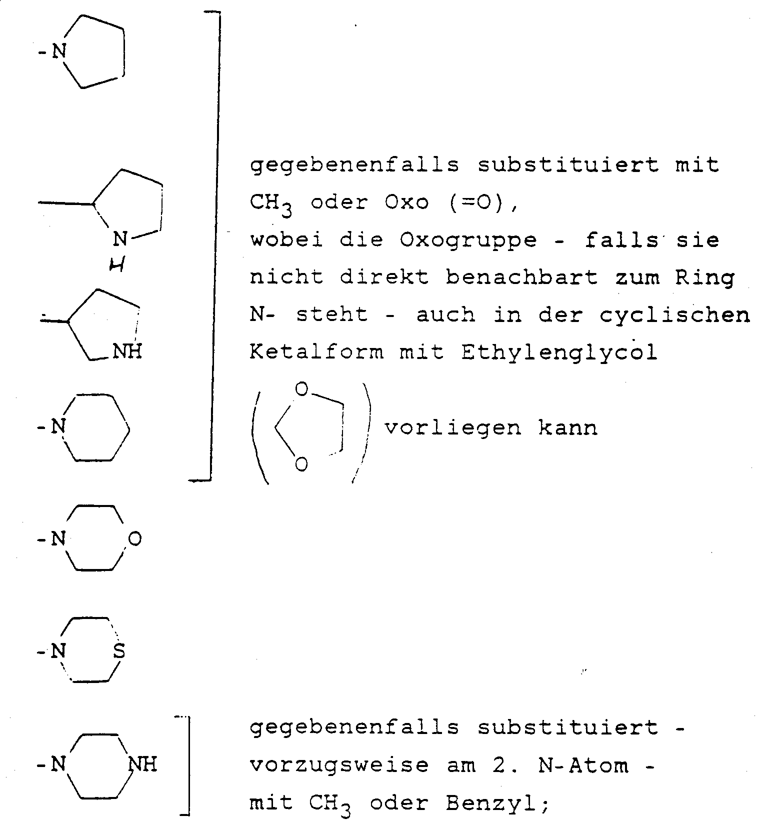
oder R⁴ und R⁵ zusammen mit dem Amid-N-Atom, an das sie gebunden sind, einen gesättigten 6-gliedrigen heterocyclischen Ring bilden der Art 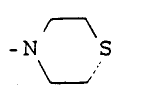 und 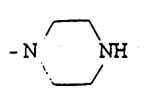
gegebenenfalls substituiert - vorzugsweise am 2. N-Atom - mit einem der folgenden Reste: 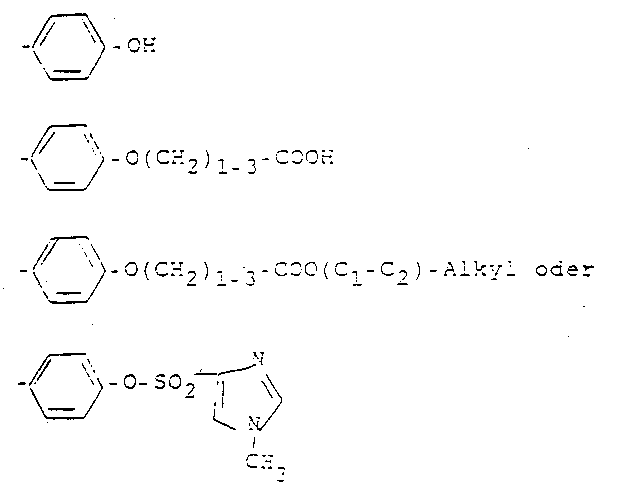 bedeutet, sowie deren physiologisch verträglichen Salze, herstellt.

4. Verfahren nach Anspruch 1, dadurch gekennzeichnet, daß man die Verbindung der Formel I gemäß Anspruch 1, worin
R¹ = CH₃
R² = R³ = H,
SO₂X-Gruppe in 2-Position und 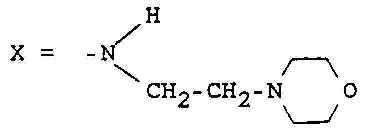 bedeutet, sowie deren physiologisch verträglichen
Salze, herstellt.

5. Verfahren nach Anspruch 1, dadurch gekennzeichnet, daß man die Verbindung der Formel I gemäß Anspruch 1, worin
R¹ = CH₃,
R² = R³ = H,
SO₂X-Gruppe in 4-Position und 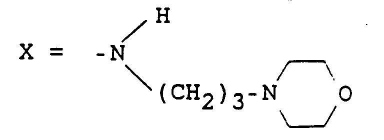 bedeutet, sowie deren physiologisch verträglichen Salze, herstellt.

6. Verwendung von Verbindungen der Formel I sowie ihrer physiologisch verträglichen Salze erhalten gemäß einem oder mehreren der Ansprüche 1 - 5 als Heilmittel.

7. Verwendung von mindestens einer Verbindung der Formel I erhalten gemäß der Definition in einem oder mehreren der Ansprüche 1 - 5 und/oder mindestens einem von deren physiologisch verträglichen Salzen zur Herstellung von Arzneimitteln.

8. Verwendung von mindestens einer Verbindung der Formel I erhalten nach einem oder mehreren der Ansprüche 1 bis 5 und/oder mindestens einem von deren physiologisch verträglichen Salzen zur Herstellung von Arzneimitteln zur Prophylaxe und/oder Therapie von Durchblutungsstörungen, insbesondere von Störungen der Mikrozirkulation, sowie der daraus resultierenden Erkrankungen.

9. Verfahren zur Herstellung der Arzneimittel gemäß Anspruch 7 oder 8, dadurch gekennzeichnet, daß man mindestens eine Verbindung der Formel I erhalten gemäß der Definition in einem oder mehreren der Ansprüche 1 - 5 und/oder mindestens eines von deren physiologisch verträglichen Salzen mit üblichen Träger- sowie gegebenenfalls Zusatz- und Hilfsstoffen in eine geeignete Darreichungsform bringt.

10. Verwendung der Verbindungen der Formel I erhalten gemäß der Definition in einem oder mehreren der Ansprüche 1 - 5 zur Prophylaxe und/oder Therapie von Durchblutungsstörungen, insbesondere von Störungen der Mikrozirkulation, sowie der daraus resultierenden Erkrankungen.

11. Verfahren zur Herstellung von
1-Methyl-,
1,2-Dimethyl- und
1-Ethyl-4-imidazolsulfonsäurechlorid,
dadurch gekennzeichnet, daß man
a) 1-Methyl- bzw. 1,2-dimethyl- bzw. 1-Ethyl-imidazol mit Chlorsulfonsäure ClSO₃H, gegebenenfalls unter nachträglichem Zusatz von SOCl₂, zur Reaktion bringt, oder daß man
b) 1-Methyl- bzw. 1,2-Methyl- bzw. 1-Ethyl-4-mercaptoimidazol mit Cl₂ oxidierend chloriert.

## Claims (Claims for the following Contracting State(s): AT, BE, CH, DE, DK, FR, GB, IT, LI, LU, NL, SE)

1. An imidazole compound of the formula I 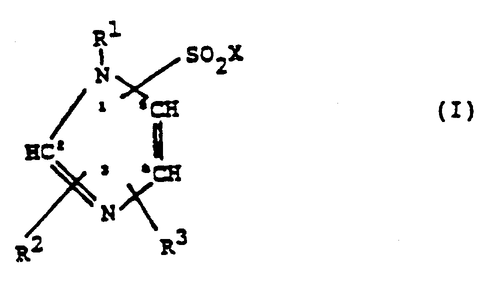 in which
R¹ is (C₁-C₆)-alkyl,
R² and R³ are identical or different and in each case are H,
halogen (F, Cl, Br or I) or (C₁-C₃)-alkyl,
X is OH
or an amino group of the formula II 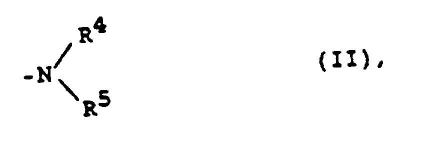 in which
R⁴ is H or (C₁-C₇)- alkyl, optionally substituted by CN, NH₂ or COOH,
R⁵ is a (C₁-C₈)-, alkyl radical, in which - if it has more than 1 carbon atom - there can also be a phenylene radical between 2 carbon atoms
and its (aliphatic) carbon atoms are substituted by 1 or more of the following groups:
OH,
(C₁-C₃)-alkoxy,
phenyl, optionally substituted by 1 - 3 OH or (C₁-C₃)-alkyl groups, (C₁-C₃)-alkoxy-COOH, and/or (-C-₁-C₃)-alkoxy-(C₁-C₄)-alkyl
COOH,
COO(C₁-C₃)-alkyl,
CONH₂,
CN,
(C₂-C₅)-alkynyl,
NH₂, 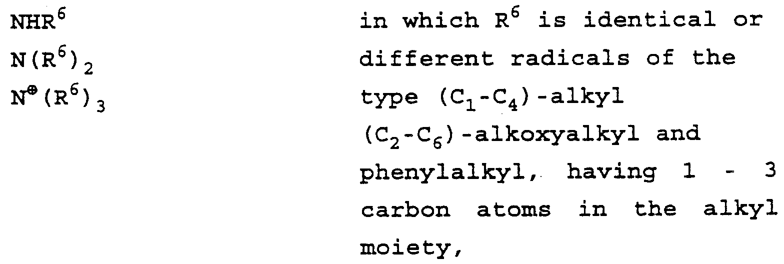 NH-CO-(C₁-C₆)-alkyl, 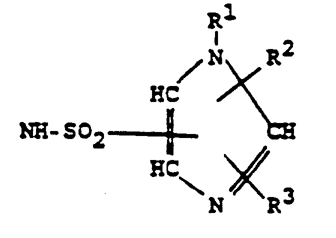 (meaning of R¹, R² and R³ see above),
monocyclic 5- to 7-membered saturated or unsatured heterocyclic radicals having 1 nitrogen atom and optionally also an additional nitrogen, oxygen or sulfur atom in the ring, from the group consisting of thiomorphine, piperazine, morpholine, imidazole, pyrrolidine and piperidine,
optionally substituted by
(C₁-C₃)-alkyl,
phenyl,
phenylalkyl having 1-3 carbon atoms in the alkyl moiety, OH, and/or
oxo (= O), including the open and cyclic ketal forms having 2 - 6 carbon atoms in the ketal moiety,
and in which the ring sulfur atom - if present - can also be oxidized to the sulfoxide (SO) or sulfone (SO₂) form,
or in which
R⁴ and R⁵, together with the amide nitrogen atom to which they are bonded, form a 5- to 7-membered heterocyclic ring from the group consisting of piperazine and thiomorpholine,
and the heterocyclic ring can otherwise be substituted by the following groups:
(C₁-C₃)-alkoxy,
phenylalkyl having 1 - 4 carbon atoms in the alkyl moiety,
phenyl, optionally substituted by 1 or more of the groups:
(C₁-C₃)-alkyl,
OH,
(C₁-C₃)-alkoxy,
(C₁-C₃)-alkoxy-COOH,
(C₁-C₃)-alkoxy-COO(C₁-C₄)alkyl, 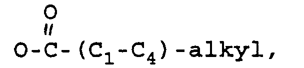 O-SO₂-C₆H₅
O-SO₂-C₆H₄CH₃, 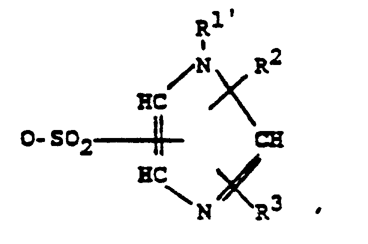 in which R^{1'} has the same meaning as R¹ and can additionally also be H, and
R² and R³ have the abovementioned meaning,
and the ring sulfur atom - if present - can also be oxidized to the sulfoxide (SO) or sulfone (SO₂) form, and its physiologically tolerable salts.

2. An imidazole compound as claimed in claim 1, wherein in formula I at least one of the following features is present:
a) R¹ is CH₃ or C₂H₅,
b) R² and R³ are identical or different and in each case are H, Cl or CH₃, and
c) the -SO₂X radical is situated in the 2- or 4-position of the imidazole ring.

3. An imidazole compound as claimed in claim 1 or 2, wherein the radical 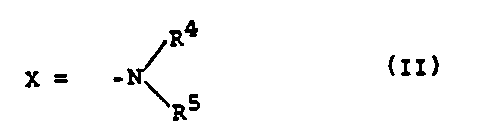 where
R⁴ is H and
R⁵ is a (C₂-C₅)-alkyl radical in which there is optionally a phenylene radical between 2 carbon atoms and its (aliphatic) carbon atoms are substituted by a total of 1 or 2 of the following groups:
hydroxyphenyl C₆H₄OH
CN
(C₂-C₃)-alkynyl
NH₂ 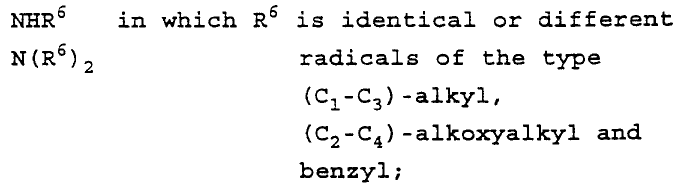 a monocylic 5- to 6-membered saturated heterocyclic radical from the group consisting of: 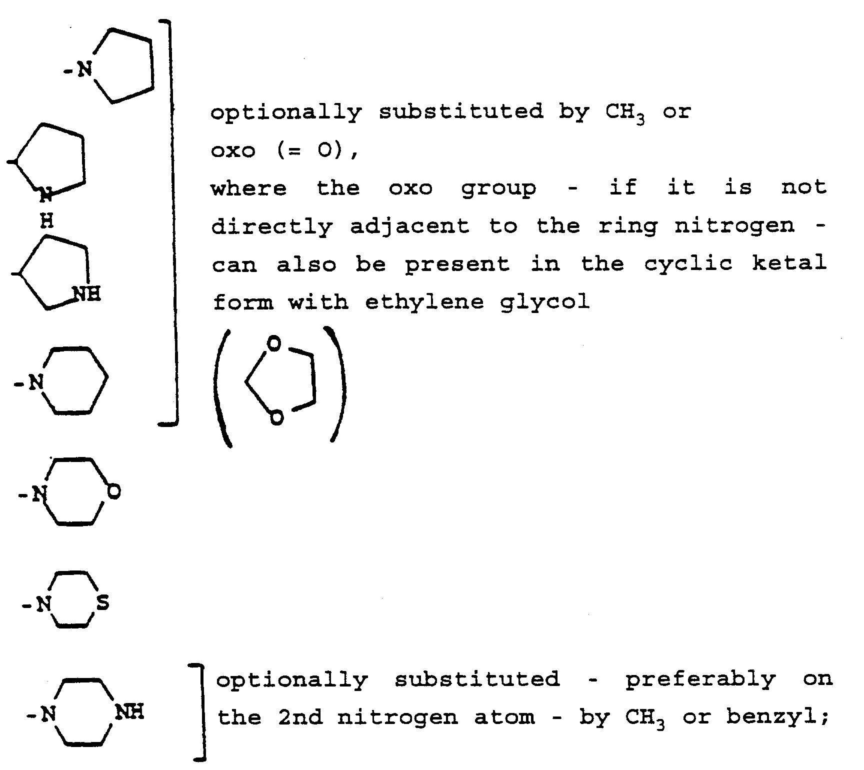 or R⁴ and R⁵, together with the amide nitrogen atom to which they are bonded, form a saturated 6-membered heterocyclic ring of the type 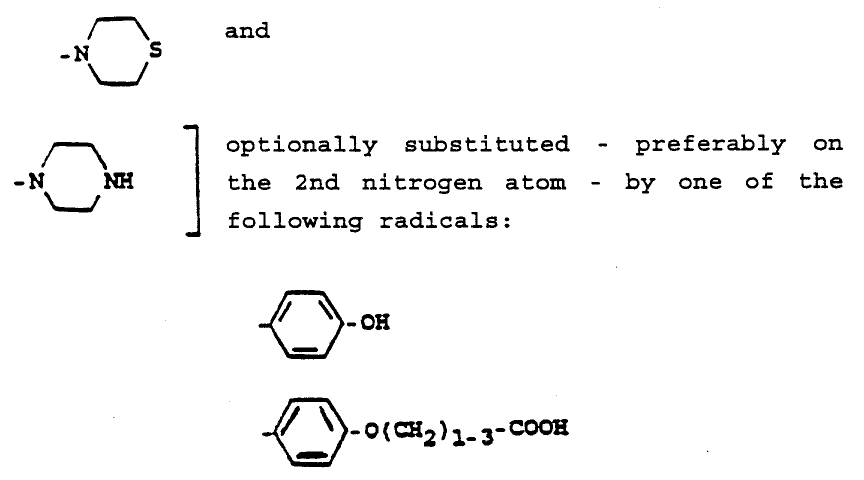 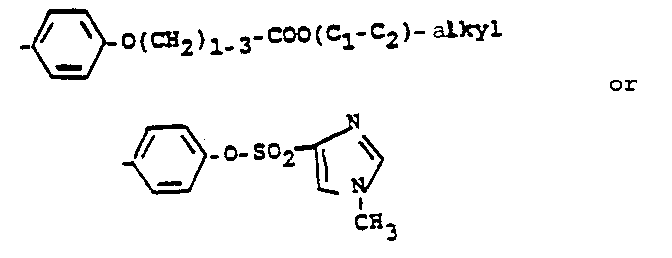 and its physiologically tolerable salts.

4. N-(2-Morpholinoethyl)-1-methyl-2-imidazolesulfonamide = compound of the formula I as claimed in claim 1 in which
R¹ = CH₃,
R² = R³ = H,
the SO₂X group is in the 2-position and 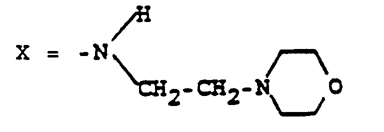 and its physiologically tolerable salts.

5. N-(3-Morpholinopropyl)-1-methyl-4-imidazolesulfonamide = compound of the formula I as claimed in claim 1 in which
R¹ = CH₃,
R² = R³ = H,
the SO₂X group is in the 4-position and 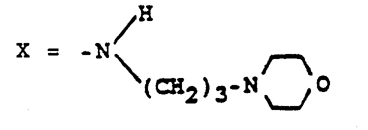 and its physiologically tolerable salts.

6. A process for the preparation of an imidazole compound of the formula I as claimed in the definition in one or more of claims 1 - 5 and, if desired, of its physiologically tolerable salts, which comprises
a) converting an imidazole derivative of the general formula III 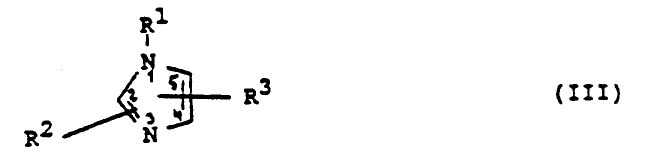 in which R¹, R² and R³ have the meaning mentioned in formula I, and one of the positions 4 or 5 is unsubstituted, by sulfonation into the compounds of the formula Ia 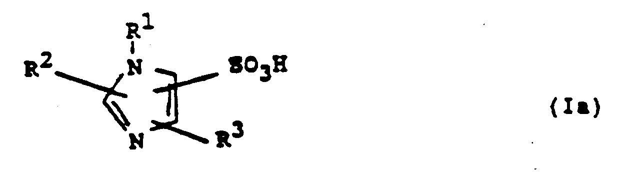 in which R¹, R² and R³ likewise have the meaning mentioned in formula I,
or
b) converting an imidazole derivative of the formula IV 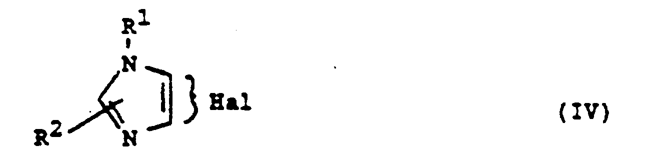 in which R¹ and R² have the meaning mentioned in formula I, one of the positions 4 or 5 carries a halogen substituent Hal (Cl, Br or I) and the other is unsubstituted, by sulfonation and subsequent hydrogenolytic dehalogenation into a compound of the formula Ib 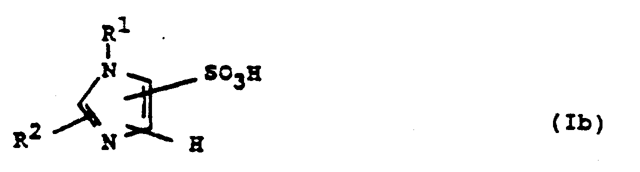 (meaning of R¹ and R² as in formula I)
or
c) hydrolyzing an imidazole derivative of the formula V 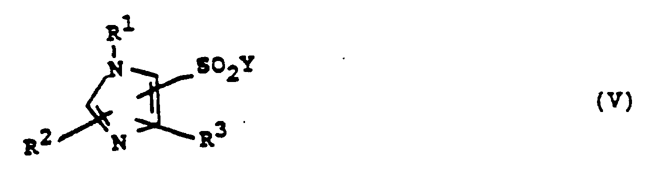 in which R¹, R² and R³ have the meaning mentioned in formula I and Y is halogen, preferably chlorine, to give the imidazolesulfonic acids of the formula Ia with the meanings for R¹, R² and R³ mentioned there,
or
d) oxidizing an imidazole derivative of the formula VI or VI' 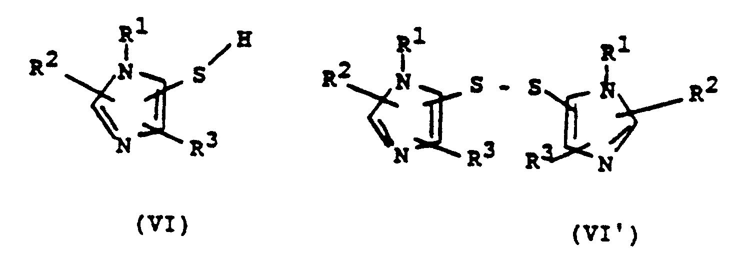 in which R¹, R² and R³ have the meaning mentioned in formula I, to give the corresponding imidazolesulfonic acid (formula Ia),
or
e) reacting an imidazolesulfonyl halide of the formula V (see variant c) with an amine of the formula H-II 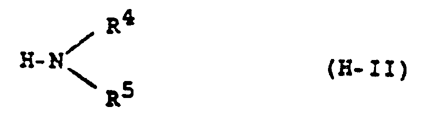 in which R⁴ and R⁵ have the same meaning as in formula II (see claim 1), to give the sulfonamides of the formula Ic 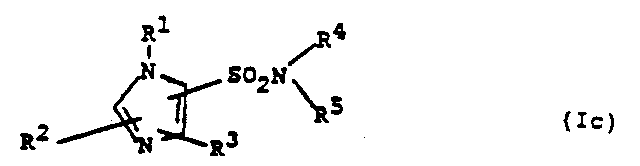 in which R¹ to R⁵ have the meanings mentioned in the formulae I and II,
or
f) reacting imidazole derivatives of the formula VII 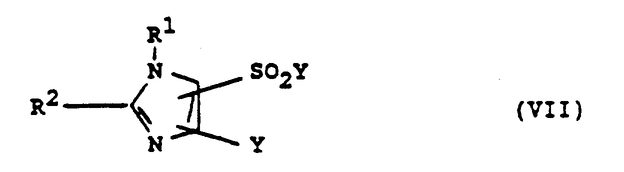 in which R¹ and R² have the meaning mentioned in formula I and Y is identical or different halogen atoms (Cl, Br or I), with amines of the formula H-II and then subjecting the products to hydrogenolytic dehalogenation in order to obtain the sulfonamides unsubstituted in the 4- or 5-position of the formula Id having the meanings for R¹ and R² given in claim 1 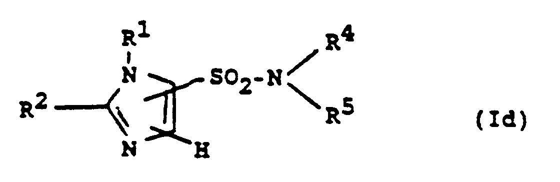 (meaning of R¹, R², R⁴ and R⁵ as in formulae I and II),
or
g) reacting an imidazolesulfonyl halide of the formula V (see variant c) with a trialkylsilylamine of the formula VIII 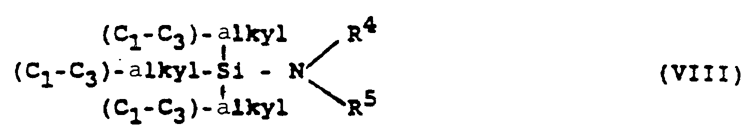 where R⁴ and R⁵ have the meaning mentioned in formula II and a preferred (C₁-C₃)-alkyl radical is the methyl radical, in order to obtain the sulfonamides of the formula Ic (see variant e),
or
h) for the preparation of compounds of the formula I -in which X = -N(R⁴)R⁵ (formula II) and R⁴ carries at least 1 NH₂ group and/or R⁵ at least one primary or secondary amino group, reacting amines of the formula
H-N(R⁴)R⁵
which carry on their radicals R⁴ and/or R⁵ at least one N-protected - preferably N-benzylated - appropriate amino group, with imidazolesulfonyl halides of the formula V (see variant c) and subsequently setting the resulting sulfonamides free from the protecting group,
or
i) for the preparation of compounds of the formula I in which X = -N(R⁴)R⁵ (formula II) and at least one of the radicals R⁴ and R⁵ carries at least 1 primary amino group, reacting imidazolesulfonyl halides of the formula V (see variant c) with appropriate aminonitriles and reducing the sulfonylaminonitriles thus obtained to the amino compounds,
or
j) for the preparation of imidazole compounds of the formula I in which X = -N(R⁴)R⁵ and R⁵ carries at least one quaternary amino group - N^{⊕}(R⁶)₃, in which the radicals R⁶ can be identical or different, alkylating an imidazole compound of the formula I in which X = -N(R⁴)R⁵ and R⁵ carries at least one tertiary amino group -N(R⁶)₂, with an alkylating agent,
or
k) for the preparation of imidazole compounds of the formula I in which X=-N(R⁴)R⁵ and R⁵, or R⁴ and R⁵ together, contain one or more sulfoxide or sulfone groups, oxidizing imidazole compounds of the formula I in which X = -N(R⁴)R⁵ and R⁵, or R⁴ and R⁵ together, contain one or more sulfide groups, to give the corresponding sulfoxides or sulfones,
or
l) for the preparation of imidazole compounds of the formula I in which X = -N(R⁴)R⁵ and R⁵, or R⁴ and R⁵ together, contain a phenol ether group, alkylating imidazole compounds of the formula I in which X = -N(R⁴)R⁵ and R⁵, or R⁴ and R⁵ together, carry at least one aryl radical - substituted by one or more phenolic hydroxyl groups - with alkylating reagents, preferably ω-halo fatty acid derivatives, to give the corresponding phenol ethers which in the case of ω-fatty acid esters can furthermore be additionally subjected to hydrolysis or aminolysis to give the corresponding carboxylic acids or carboxamides,
or
m) for the preparation of imidazole compounds of the formula I in which X = -N(R⁴)R⁵ and R⁵, or R⁴ and R⁵ together, contain a phenol ester group, reacting the same starting imidazole compounds as for variant l with acylating agents - preferably with (C₁-C₄)-alkylcarbonyl chlorides, benzene- and toluenesulfonyl chlorides and also imidazolesulfonyl halides of the formula V (see variant c) in which R¹, R² and R³ have the same meaning as in formula I and R¹ can additionally also be hydrogen - to give the corresponding phenol esters,
and, if desired, additionally converting the compounds of the formula I formed in all process variants - if these are not already obtained in the form of physiologically tolerable salts - into these.

7. A compound of the formula I and its physiologically tolerable salts as claimed in one or more of claims 1 - 5 or as obtained by the process as claimed in claim 6 for use as a medicine.

8. A pharmaceutical which contains at least one compound of the formula I as claimed in the definition in one or more of claims 1 - 5 and/or at least one of its physiologically tolerable salts and/or at least one compound prepared by the process as claimed in claim 6.

9. A pharmaceutical as claimed in claim 8, which is intended for the prophylaxis and/or treatment of circulatory disturbances, in particular of disturbances of the microcirculation, and of the disorders resulting therefrom.

10. A process for the production of the pharmaceutical as claimed in claim 8 or 9, which comprises bringing at least one compound of the formula I as claimed in the definition in one or more of claims 1 - 5 and/or at least one of its physiologically tolerable salts and/or at least one compound obtained by the process as claimed in claim 6 into a suitable administration form using customary excipients and, if appropriate, additives and auxiliaries.

11. The use of a compound of the formula I as claimed in the definition in one or more of claims 1 - 5 and/or at least one compound obtained by the process as claimed in claim 6 for the prophylaxis and/or treatment of circulatory disturbances, in particular of disturbances of the microcirculation, and of the disorders resulting therefrom.

12. 1-Methyl-,
1,2-dimethyl- and
1-ethyl-4-imidazolesulfonyl chloride.

13. A process for the preparation of the compounds mentioned in claim 11, which comprises
a) reacting 1-methyl- or 1,2-dimethyl- or 1-ethylimidazole with chlorosulfonic acid ClSO₃H, if appropriate with subsequent addition of SOCl₂, or
b) subjecting 1-methyl- or 1,2-dimethyl- or 1-ethyl-4-mercaptoimidazole to oxidative chlorination with Cl₂.

## Claims (Claims for the following Contracting State(s): ES, GR)

1. A process for the preparation of an imidazole compound of the formula I 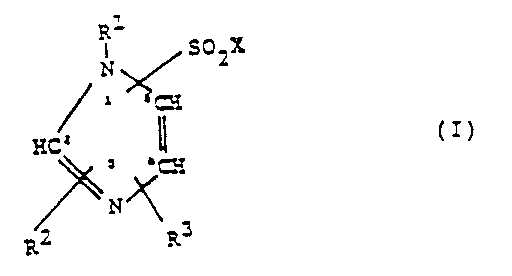 in which
R¹ is (C₁-C₆)-alkyl,
R² and R³ are identical or different and in each case are H,
halogen (F, Cl, Br or I), or (C₁-C₃)-alkyl,
X is OH
or an amino group of the formula II 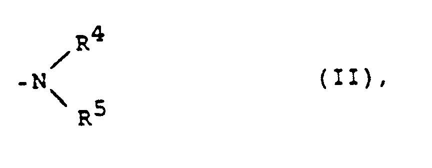 in which
R⁴ is H or
(C₁-C₇)-, alkyl,
optionally substituted by CN, NH₂ or COOH,
R⁵ is a (C₁-C₈)-alkyl radical, in which - if it has more than 1 carbon atom - there can also be a phenylene radical between 2 carbon atoms and its (aliphatic) carbon atoms are substituted by 1 or more of the following groups:
OH,
(C₁-C₃)-alkoxy,
phenyl, optionally substituted by 1 - 3 OH or (C₁-C₃)-alkyl groups, (C₁-C₃)-alkoxy-COOH,
and/or (C₁-C₃)-alkoxy-(C₁-C₄)-alkyl
COOH,
COO(C₁-C₃)-alkyl,
CONH₂,
CN,
(C₂-C₅)-alkynyl,
NH₂, 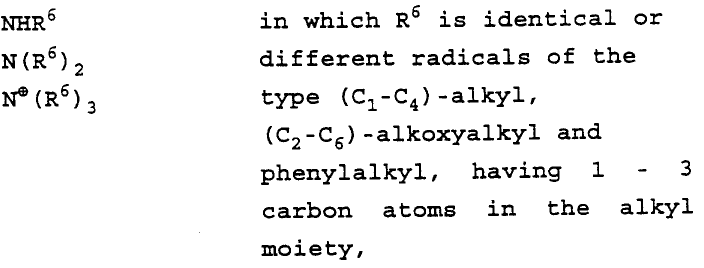 NH-CO-(C₁-C₆)-alkyl, 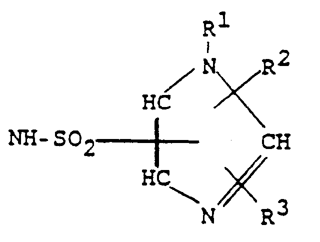 (meaning of R¹, R² and R³ see above),
monocyclic 5- to 7-membered saturated or unsatured heterocyclic radicals having 1 nitrogen atom and optionally also an additional nitrogen, oxygen or sulfur atom in the ring from the group consisting of thiomorpholine, piperazine, morpholine, imidazole, pyrrolidine and piperidine,
optionally substituted by (C₁-C₃)-alkyl,
phenyl,
phenylalkyl having 1 - 3 carbon atoms in the alkyl moiety, OH, and/or
oxo (= O), including the open and cyclic ketal forms having 2 - 6 carbon atoms in the ketal moiety,
and in which the ring sulfur atom - if present - can also be oxidized to the sulfoxide (SO) or sulfone (SO₂) form,
or in which
R⁴ and R⁵, together with the amide nitrogen atom to which they are bonded, form a 5- to 7-membered heterocyclic ring from the group consisting of piperazine and thiomorpholine
and the heterocyclic ring can otherwise be substituted by the following groups:
(C₁-C₃)-alkoxy,
phenylalkyl having 1 - 4 carbon atoms in the alkyl moiety,
phenyl, optionally substituted by 1 or more of the groups:
(C₁-C₃)-alkyl,
OH,
(C₁-C₃)-alkoxy,
(C₁-C₃)-alkoxy-COOH,
(C₁-C₃)-alkoxy-COO(C₁-C₄)alkyl, 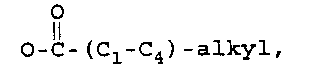 O-SO₂-C₆H₅
O-SO₂-C₆H₄CH₃, 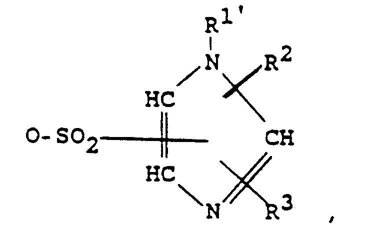 in which R^{1'} has the same meaning as R¹ and can additionally also be H, and
R² and R³ have the abovementioned meaning, and the ring sulfur atom - if present - can also be oxidized to the sulfoxide (SO) or sulfone (SO₂) form, and its physiologically tolerable salts,
which comprises
a) converting an imidazole derivative of the formula III 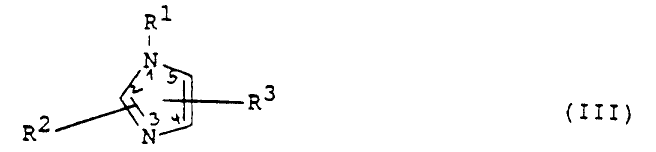 in which R¹, R² and R³ have the meaning mentioned in formula I, and one of the positions 4 or 5 is unsubstituted, by sulfonation into the compounds of the formula Ia 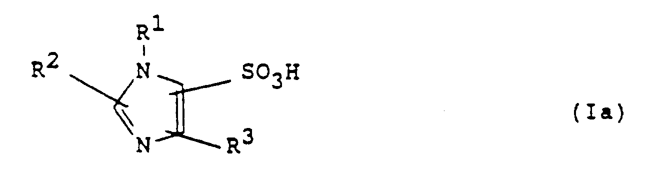 in which R¹, R² and R³ likewise have the meaning mentioned in formula I,
or
b) converting an imidazole derivative of the formula IV 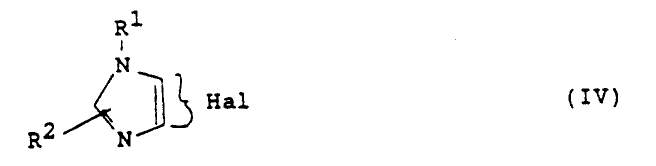 in which R¹ and R² have the meaning mentioned in formula I, one of the positions 4 or 5 carries a halogen substituent Hal (Cl, Br or I) and the other is unsubstituted, by sulfonation and subsequent hydrogenolytic dehalogenation into a compound of the formula Ib 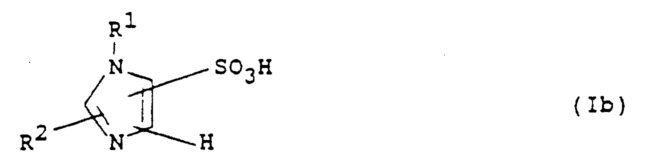 (meaning of R¹ and R² as in formula I)
or
c) hydrolyzing an imidazole derivative of the formula V 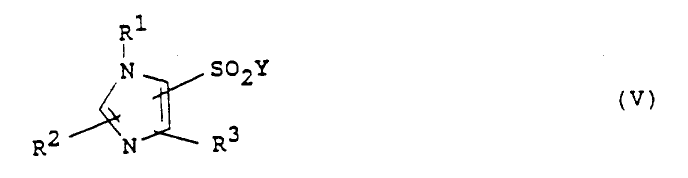 in which R¹, R² and R³ have the meaning mentioned in formula I and Y is halogen, to give the imidazolesulfonic acids of the formula Ia with the meanings for R¹, R² and R³ mentioned there,
or
d) oxidizing an imidazole derivative of the formula VI or VI' 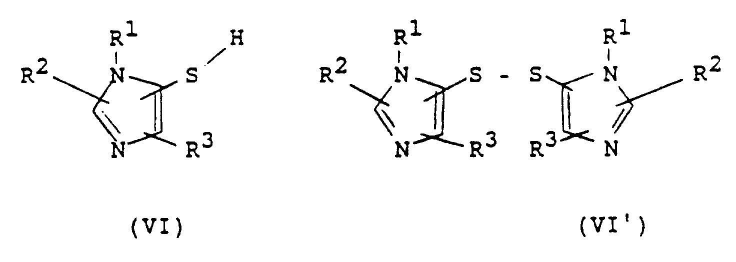 in which R¹, R² and R³ have the meaning mentioned in formula I, to give the corresponding imidazolesulfonic acid (formula Ia),
or
e) reacting an imidazolesulfonyl halide of the formula V (see variant c) with an amine of the formula H-II 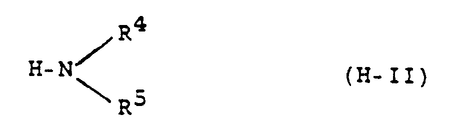 in which R⁴ and R⁵ have the same meaning as in formula II, to give the sulfonamides of the formula Ic 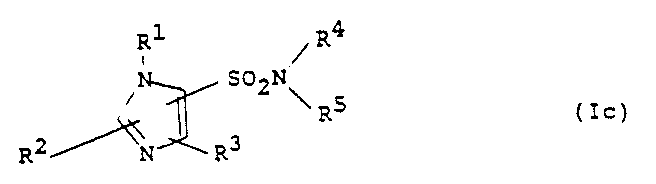 in which R¹ to R⁵ have the meanings mentioned in the formulae I and II,
or
f) reacting imidazole derivatives of the formula VII 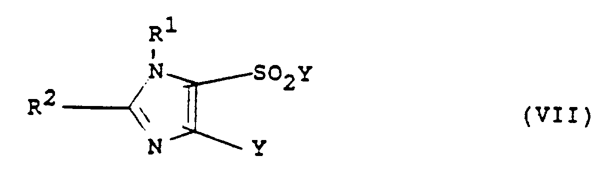 in which R¹ and R² have the meaning mentioned in formula I and Y is identical or different halogen atoms (Cl, Br or I), with amines of the formula H-II and then subjecting the products to hydrogenolytic dehalogenation, in order to obtain the sulfonamides unsubstituted in the 4- or 5-position of the formula Id having the meanings for R¹ and R² given in claim 1 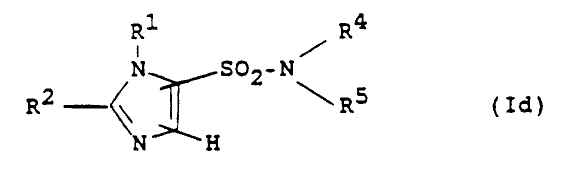 (meaning of R¹, R², R⁴ and R⁵ as in formulae I and II),
or
g) reacting an imidazolesulfonyl halide of the formula V (see variant c) with a trialkylsilylamine of the formula VIII 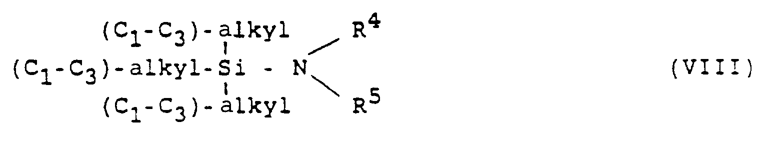 where R⁴ and R⁵ have the meaning mentioned in formula II and a preferred (C₁-C₃)-alkyl radical is the methyl radical, in order to obtain the sulfonamides of the formula Ic (see variant e),
or
h) for the preparation of compounds of the formula I -in which X = -N(R⁴)R⁵ (formula II) and R⁴ carries at least 1 NH₂ group and/or R⁵ at least one primary or secondary amino group, reacting amines of the formula
H-N(R⁴)R⁵
which carry on their radicals R⁴ and/or R⁵ at least one N-protected appropriate amino group, with imidazolesulfonyl halides of the formula V (see variant c) and subsequently setting the resulting sulfonamides free from the protecting group,
or
i) for the preparation of compounds of the formula I in which X = -N(R⁴)R⁵ (formula II) and at least one of the radicals R⁴ and R⁵ carries at least 1 primary amino group, reacting imidazolesulfonyl halides of the formula V (see variant c) with appropriate aminonitriles and reducing the sulfonyl-aminonitriles thus obtained to the amino compounds,
or
j) for the preparation of imidazole compounds of the formula I in which X = -N(R⁴)R⁵ and R⁵ carries at least one quaternary amino group -N^{⊕}(R⁶)₃, in which the radicals R⁶ can be identical or different, alkylating an imidazole compound of the formula I in which X = -N(R⁴)R⁵ and R⁵ carries at least one tertiary amino group -N(R⁶)₂, with an alkylating agent,
or
k) for the preparation of imidazole compounds of the formula I in which X=-N(R⁴)R⁵ and R⁵, or R⁴ and R⁵ together, contain one or more sulfoxide or sulfone groups, oxidizing imidazole compounds of the formula I in which X = -N(R⁴)R⁵ and R⁵, or R⁴ and R⁵ together, contain one or more sulfide groups, to give the corresponding sulfoxides or sulfones,
or
l) for the preparation of imidazole compounds of the formula I in which X = -N(R⁴)R⁵ and R⁵, or R⁴ and R⁵ together, contain a phenol ether group, alkylating imidazole compounds of the formula I in which X = -N(R⁴)R⁵ and R⁵, or R⁴ and R⁵ together, carry at least one aryl radical - substituted by one or more phenolic hydroxyl groups - with alkylating reagents, preferably ω-halo fatty acid derivatives, to give the corresponding phenol ethers which in the case of ω-fatty acid esters can furthermore be additionally subjected to hydrolysis or aminolysis to give the corresponding carboxylic acids or carboxamides,
or
m) for the preparation of imidazole compounds of the formula I in which X = -N(R⁴)R⁵ and R⁵, or R⁴ and R⁵ together, contain a phenol ester group, reacting the same starting imidazole compounds as for variant l with acylating agents such as (C₁-C₄)-alkylcarbonyl chlorides, benzene- and toluenesulfonyl chlorides and also imidazolesulfonyl halides of the formula V (see variant c) in which R¹, R² and R³ have the same meaning as in formula I and R¹ can additionally also be hydrogen to give the corresponding phenol esters,
and, if desired, additionally converting the compounds of the formula I formed in all process variants - if these are not already obtained in the form of physiologically tolerable salts - into these.

2. The process as claimed in claim 1, wherein a compound of the formula I is prepared in which at least one of the following features is present:
a) R¹ is CH₃ or C₂H₅,
b) R² and R³ are identical or different and in each case are H, Cl or CH₃, and
c) the -SO₂X radical is situated in the 2- or 4-position of the imidazole ring.

3. The process as claimed in claim 1 or 2, wherein a compound of the formula I is prepared in which the radical 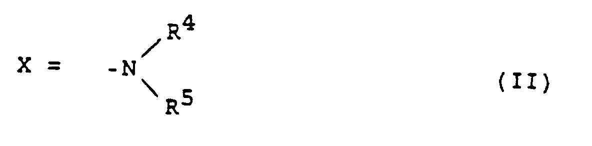 where
R⁴ is H and
R⁵ is a (C₂-C₅)-alkyl radical in which there is optionally a phenylene radical between 2 carbon atoms and its (aliphatic) carbon atoms are substituted by a total of 1 or 2 of the following groups:
hydroxyphenyl C₆H₄OH
CN
(C₂-C₃)-alkynyl
NH₂ 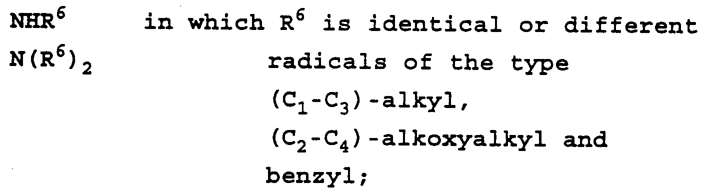 a monocylic 5- to 6-membered saturated heterocyclic radical from the group consisting of: 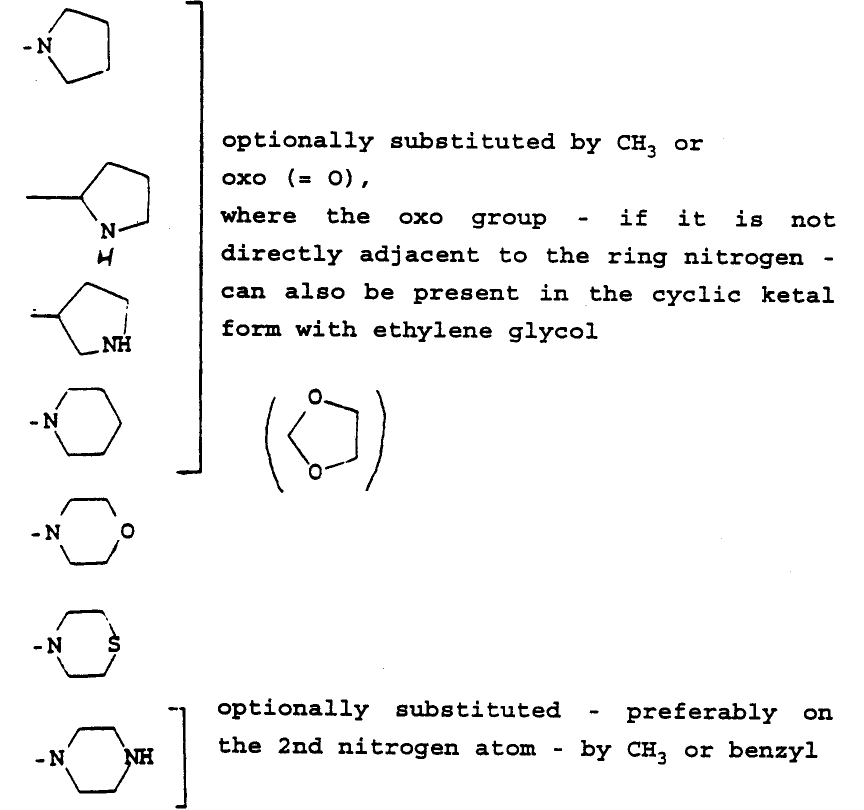 or R⁴ and R⁵, together with the amide nitrogen atom to which they are bonded, form a saturated 6-membered heterocyclic ring of the type
and 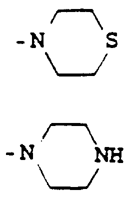 optionally substituted - preferably on the 2nd nitrogen atom - by one of the following radicals: 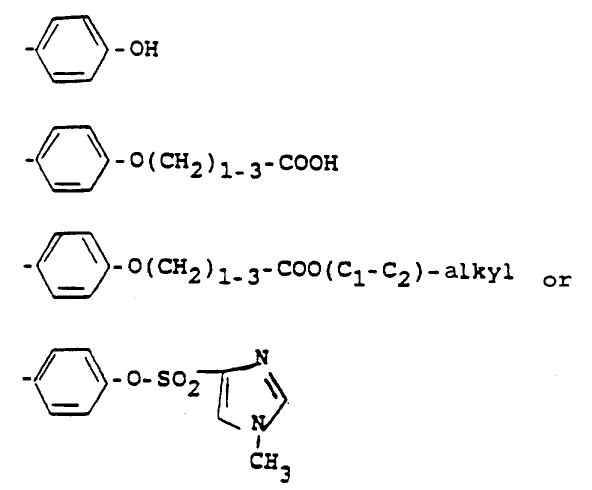 and its physiologically tolerable salts.

4. The process as claimed in claim 1, wherein the compound of the formula I as claimed in claim 1 is prepared in which
R¹ = CH₃,
R² = R³ = H,
the SO₂X group is in the 2-position and 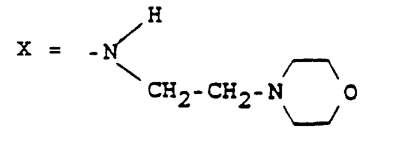 and its physiologically tolerable salts.

5. The process as claimed in claim 1, wherein the compound of the formula I as claimed in claim 1 is prepared in which
R¹ = CH₃,
R² = R³ = H,
the SO₂X group is in the 4-position and 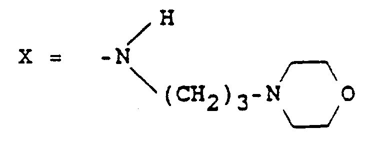 and its physiologically tolerable salts.

6. The use of a compound of the formula I and its physiologically tolerable salts obtained as claimed in one or more of claims 1 - 5 as a medicine.

7. The use of at least one compound of the formula I obtained as claimed in the definition in one or more of claims 1 - 5 and/or at least one of its physiologically tolerable salts for the production of pharmaceuticals.

8. The use of at least one compound of the formula I obtained as claimed in one or more of claims 1 to 5 and/or at least one of its physiologically tolerable salts for the production of pharmaceuticals for the prophylaxis and/or treatment of circulatory disturbances, in particular of disturbances of the microcirculation, and of the disorders resulting therefrom.

9. A process for the production of the pharmaceuticals as claimed in claim 7 or 8, which comprises bringing at least one compound of the formula I obtained as claimed in the definition in one or more of claims 1 - 5 and/or at least one of its physiologically tolerable salts into a suitable administration form using customary excipients and, if appropriate, additives and auxiliaries.

10. The use of the compounds of the formula I obtained as claimed in the definition in one or more of claims 1 -5 for the prophylaxis and/or treatment of circulatory disturbances, in particular of disturbances of the microcirculation, and of the disorders resulting therefrom.

11. A process for the preparation of
1-methyl-,
1,2-dimethyl- and
1-ethyl-4-imidazolesulfonyl chloride,
which comprises
a) reacting 1-methyl- or 1,2-dimethyl- or 1-ethylimidazole with chlorosulfonic acid ClSO₃H, if appropriate with subsequent addition of SOCl₂, or
b) subjecting 1-methyl- or 1,2-dimethyl- or 1-ethyl-4-mercaptoimidazole to oxidative chlorination with Cl₂.

## Revendications (Revendications pour l'(les) Etat(s) contractant(s) suivant(s): AT, BE, CH, DE, DK, FR, GB, IT, LI, LU, NL, SE)

1. Composés de type imidazole de formule I 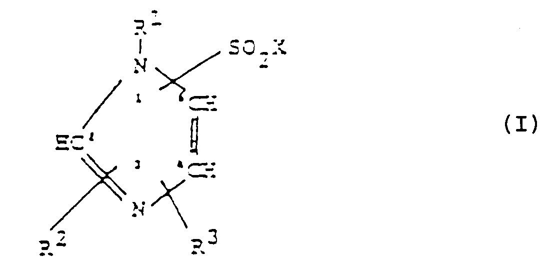 dans laquelle
R¹ est un groupe alkyle en C₁-C₆,
R² et R³ sont identiques ou différents et représentent chacun
H,
un atome d'halogène (F, Cl, Br, I) ou un groupe alkyle en C₁-C₃,
X est OH,
ou un groupe amino de formule II 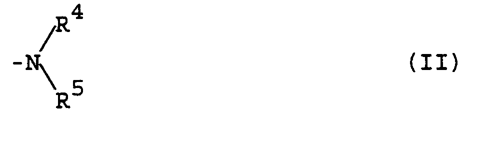 dans laquelle
R⁴ est H ou un groupe alkyle en C₁-C₇ éventuellement substitué par CN, NH₂ ou COOH,
R⁵ est un groupe alkyle en C₁-C₈ dans lequel - lorsqu'il a plus de 1 atome de carbone - un radical phénylène peut également être présent entre 2 atomes de carbone, et dont les atomes de carbone (aliphatiques) sont substitués par un ou plusieurs des groupes suivants: OH,
alcoxy en C₁-C₃,
phényle éventuellement substitué par 1-3 groupes OH, alkyle en C₁-C₃, alcoxy(C₁-C₃)-COOH et/ou alcoxy(C₁-C₃)-alkyle(C₁-C₄),
COOH,
COO-alkyle(C₁-C₃),
CONH₂,
CN,
alcynyle en C₂-C₅,
NH₂, 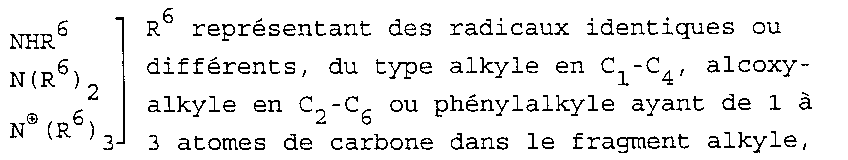 NH-CO-alkyle(C₁-C₆) 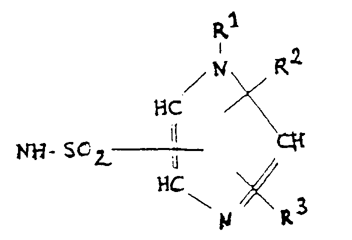 (significations de R¹, R² et R³: voir plus haut),
des radicaux hétérocycliques monocycliques à 5-7 chaînons, saturés ou insaturés, comportant dans le cycle un atome de N ainsi qu'éventuellement encore un atome de N, O ou S supplémentaire, choisis parmi les cycles thiomorpholine, pipérazine, morpholine, imidazole, pyrrolidine et pipéridine,
éventuellement substitués par
un groupe alkyle en C₁-C₃,
phényle,
phénylalkyle ayant de 1 à 3 atomes de carbone dans le fragment alkyle,
OH et/ou oxo (=O), y compris les formes cétal ouvertes ou cycliques comportant 2-6 atomes de carbone dans le fragment cétal,
et dans lesquels l'atome de S du cycle - s'il est présent - peut également être oxydé en la forme sulfoxyde (SO) ou sulfone (SO₂),
ou dans laquelle
R⁴ et R⁵ forment, conjointement avec l'atome de N de la fonction amide auquel ils sont liés, un cycle hétérocyclique saturé à 5-7 chaînons, choisi parmi les cycles pipérazine et thiomorpholine,
et le cycle hétérocyclique peut par ailleurs être substitué par les groupes suivants:
alcoxy en C₁-C₃,
phénylalkyle ayant de 1 à 4 atomes de carbone dans le fragment alkyle,
phényle éventuellement substitué par un ou plusieurs groupes choisis parmi les suivants:
alkyle en C₁-C₃,
OH,
alcoxy en C₁-C₃,
alcoxy(C₁-C₃)-COOH,
alcoxy(C₁-C₃)-COO-alkyle(C₁-C₄), 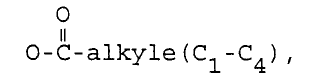 O-SO₂-C₆H₅,
O-SO₂-C₆H₄CH₃, 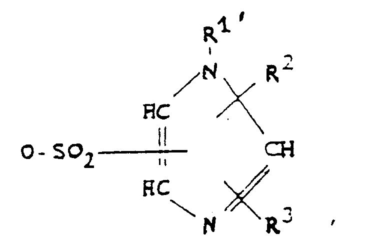 dans lequel
R^{1'} a la même signification que R¹ et en plus peut encore être H, et
R² et R³ ont les significations indiquées précédemment,
et l'atome de S du cycle - s'il est présent - peut également être oxydé en la forme sulfoxyde (SO) ou sulfone (SO₂),
et leurs sels physiologiquement acceptables.

2. Composés de type imidazole selon la revendication 1, caractérisés en ce que, dans la formule I, est présente au moins l'une des caractéristiques suivantes:
a) R¹ = CH₃ ou C₂H₅,
b) R², R³ sont identiques ou différents et représentent chacun H, Cl ou CH₃, et
c) le radical -SO₂X se trouve en position 2 ou 4 du cycle imidazole.

3. Composés de type imidazole selon la revendication 1 ou 2, caractérisés en ce que le radical 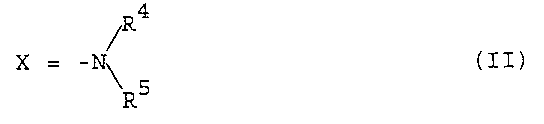 dans lequel
R⁴ = H et
R⁵ représente un radical alkyle en C₂-C₅ dans lequel éventuellement un radical phénylène est présent entre 2 atomes de carbone, et dont des atomes de carbone (aliphatiques) sont substitués par au total 1 ou 2 des groupes suivants:
hydroxyphényle C₆H₄OH,
CN,
alcynyle en C₂-C₃,
NH₂, 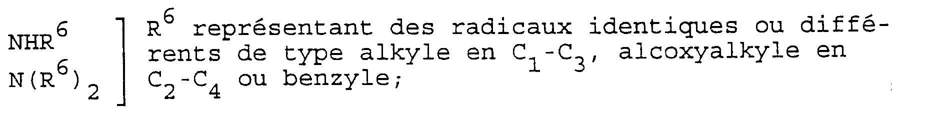 un radical monocyclique hétérocyclique saturé à 5-6 chaînons, choisi parmi: 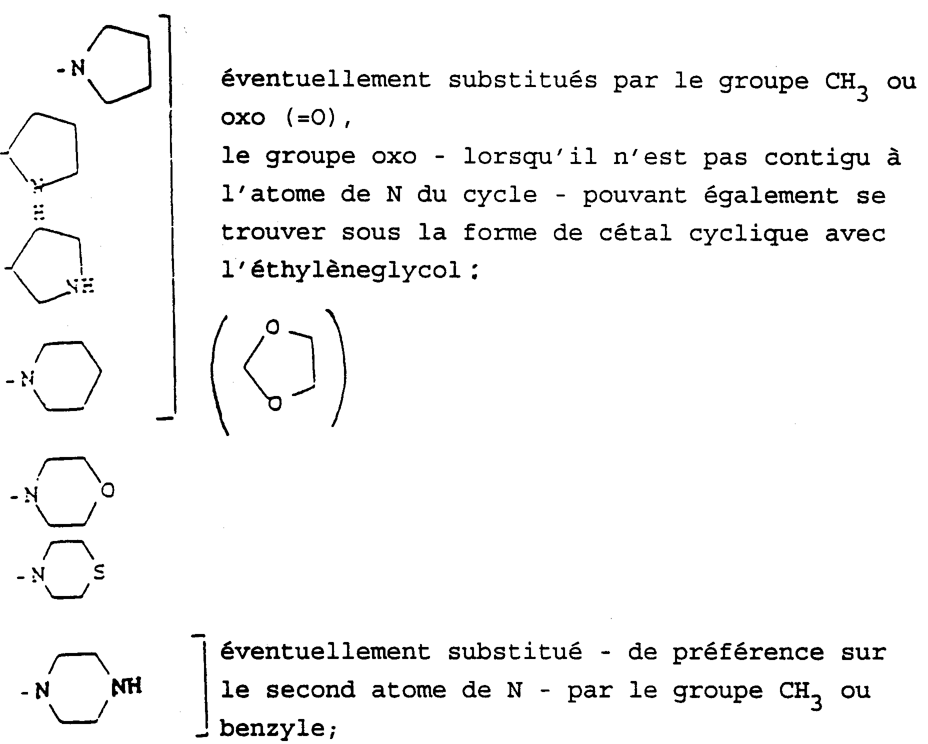 ou R⁴ et R⁵ forment ensemble, avec l'atome de N de la fonction amide auquel ils sont liés, un cycle hétérocyclique saturé à 6 chaînons, du type 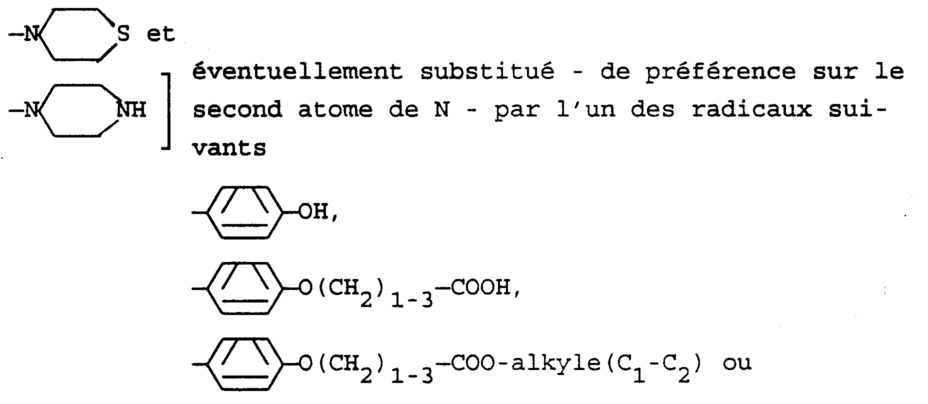 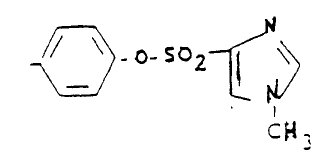 et leurs sels physiologiquement acceptables.

4. N-(2-morpholinoéthyl)-1-méthyl-2-imidazolsulfonamide = composé de formule I selon la revendication 1, dans lequel R¹ = CH₃,
R² = R³ = H,
le groupe SO₂X est en position 2 et 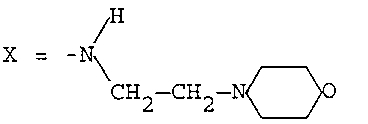 et ses sels physiologiquement acceptables.

5. N-(3-morpholinopropyl)-1-méthyl-4-imidazolsulfonamide = composé de formule I selon la revendication 1, dans lequel
R¹ = CH₃,
R² = R³ = H,
le groupe SO₂X est en position 4 et 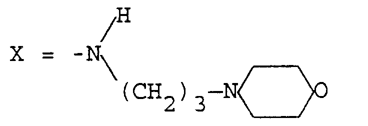 et ses sels physiologiquement acceptables.

6. Procédé pour la préparation des composés de type imidazole de formule I selon la définition donnée dans une ou plusieurs des revendications 1 à 5, et éventuellement de leurs sels physiologiquement acceptables, caractérisé en ce que
a) on convertit par sulfonation un dérivé d'imidazole de formule générale III 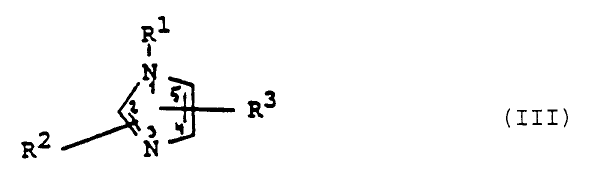 dans laquelle R¹, R² et R³ ont les significations données à propos de la formule I, et l'une des positions 4 et 5 est non substituée, en les composés de formule Ia 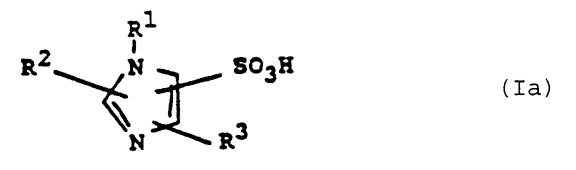 dans laquelle R¹, R² et R³ ont également les significations données à propos de la formule I, ou
b) on convertit un dérivé d'imidazole de formule générale IV 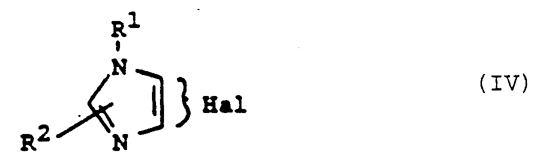 dans laquelle R¹ et R² ont les significations données à propos de la formule I, l'une des positions 4 et 5 porte un substituant halogène Hal (Cl, Br, I) et l'autre n'est pas substitué, par sulfonation et déshalogénation par hydrogénolyse subséquente, en un composé de formule Ib 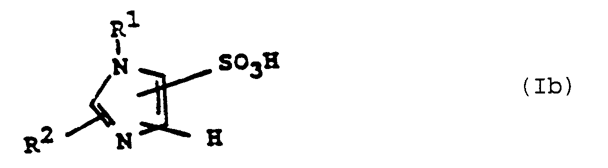 (significations de R¹ R²: comme dans la formule I)
ou
c) on hydrolyse un dérivé d'imidazole de formule générale V 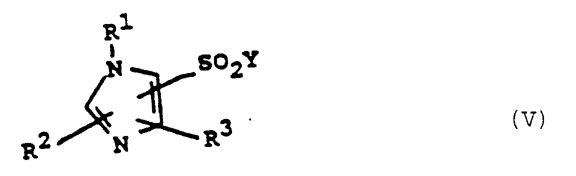 dans laquelle R¹, R² et R³ ont les significations données à propos de la formule I, et Y représente un atome d'halogène, de préférence un atome de chlore, pour aboutir aux acides imidazolsulfoniques de formule Ia avec les significations de R¹, R² et R³ qui y sont données,
ou
d) on oxyde un dérivé d'imidazole de formule générale VI ou VI' 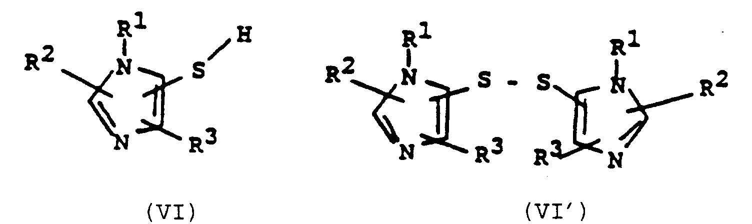 formules dans lesquelles R¹, R² et R³ ont les significations données à propos de la formule I, pour aboutir à l'acide imidazolsulfonique correspondant (formule Ia),
ou
e) on fait réagir un halogénure d'imidazolsulfonyle de formule générale V (voir variante c) avec une amine de formule H-II 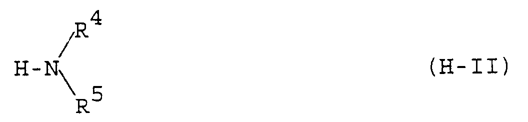 dans laquelle R⁴ et R⁵ ont les mêmes significations que dans la formule II (voir revendication 1), pour aboutir aux sulfonamides de formule Ic 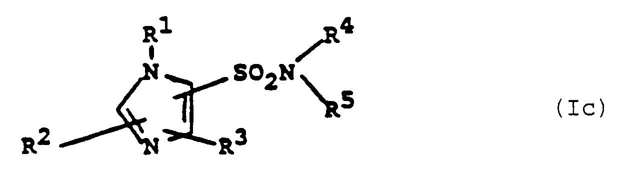 dans laquelle R¹ à R⁵ ont les significations données à propos des formules I et II,
ou
f) on fait réagir des dérivés d'imidazole de formule générale VII 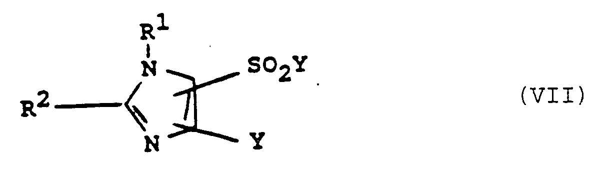 dans laquelle R¹ et R² ont les significations données à propos de la formule I, et Y représente des atomes d'halogène (Cl, Br, I) identiques ou différents, avec des amines de formule H-II, et on les soumet ensuite à une déshalogénation par hydrogénolyse, afin d'obtenir les sulfonamides non substitués en position 4 ou 5, de formule Id avec les significations indiquées dans la revendication 1 pour R¹ et R² 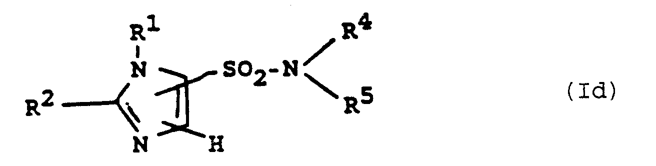 (significations de R¹, R², R⁴ et R⁵ comme dans les formules I et II),
ou
g) on fait réagir un halogénure d'imidazolsulfonyle de formule générale V (voir variante c) avec une trialkylsilylamine de formule VIII 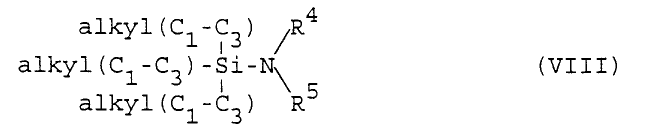 dans laquelle R⁴ et R⁵ ont les significations données à propos de la formule II, et le radical alkyle en C₁-C₃ préféré est le radical méthyle, pour obtenir les sulfonamides de formule Ic (voir variante e),
ou
h) pour la préparation des composés de formule I dans lesquels X = -N(R⁴)R⁵ (formule II) et R⁴ porte au moins un groupe NH₂ et/ou R⁵ porte au moins un groupe amino primaire ou secondaire, on fait réagir des amines de formule
H-N(R⁴)R⁵
qui portent sur leurs radicaux R⁴ et/ou R⁵ au moins un groupe amino correspondant, protégé à l'azote - de préférence N-benzylé - avec des halogénures d'imidazolsulfonyle de formule V (voir variante c), et on élimine ensuite le groupe protecteur des sulfonamides obtenus,
ou
i) pour la préparation des composés de formule générale I dans lesquels X = -N(R⁴)R⁵ (formule II) et au moins l'un des radicaux R⁴ et R⁵ porte au moins un groupe amino primaire, on fait réagir des halogénures d'imidazolsulfonyle de formule V (voir variante c) avec des aminonitriles correspondants, et les sulfonylaminonitriles obtenus sont réduits en les composés amino,
ou
j) pour la préparation des composés de type imidazole de formule générale I dans lesquels X = -N(R⁴)R⁵ et R⁵ porte au moins un groupe amino quaternaire -N^{⊕}(R⁶)₃ dans lequel les radicaux R⁶ peuvent être identiques ou différents, on soumet à une alkylation, avec un agent d'alkylation, un composé de type imidazole de formule I dans lequel X = -N(R⁴)R⁵ et R⁵ porte au moins un groupe amino tertiaire -N(R⁶)₂,
ou
k) pour la préparation des composés de type imidazole de formule générale I dans lesquels X = -N(R⁴)R⁵ et R⁵ ou R⁴ et R⁵ ensemble contiennent un ou plusieurs groupes sulfoxyde ou sulfonyle, on oxyde des composés de type imidazole de formule générale I dans lesquels X = -N(R⁴)R⁵, et R⁵ ou R⁴ et R⁵ ensemble contiennent un ou plusieurs groupes sulfure, pour aboutir aux sulfoxydes ou sulfones correspondants,
ou
l) pour la préparation des composés de type imidazole de formule I dans lesquels X = -N(R⁴)R⁵ et R⁵ ou R⁴ et R⁵ ensemble contiennent un groupe phénoléther, on soumet des composés de type imidazole de formule générale I dans lesquels X = -N(R⁴)R⁵ et R⁵ ou R⁴ et R⁵ ensemble portent au moins un radical aryle substitué par un ou plusieurs groupes hydroxy phénoliques, à une alkylation avec des réactifs d'alkylation, de préférence des dérivés d'acides gras ω-halogénés, pour aboutir aux phénoléthers correspondants qui, dans le cas d'esters d'acides gras ω, peuvent être en outre soumis à une hydrolyse ou une aminolyse, conduisant aux acides carboxyliques ou carboxamides correspondants,
ou
m) pour la préparation des composés de type imidazole de formule I dans lesquels X = -N(R⁴)R⁵ et R⁵ ou R⁴ et R⁵ ensemble contiennent un groupe ester phénolique, on convertit en les esters phénoliques correspondants les mêmes copmposés de type imidazole de départ que pour la variante l), avec des réactifs d'acylation, de préférence avec des chlorures d'alkyl(C₁-C₄)carbonyle, des chlorures de benzène- et toluènesulfonyle ainsi que des halogénures d'imidazolsulfonyle de formule générale V (voir variante c), dans laquelle R¹, R² et R³ ont les mêmes significations que dans la formule I et R¹ peut en outre représenter un atome d'hydrogène,
et éventuellement on convertit encore les composés de formule I formés dans toutes les variantes du procédé - lorsqu'ils ne se trouvent pas déjà sous forme de sels physiologiquement acceptables - en de tels sels.

7. Composés de formule I et leurs sels physiologiquement acceptables, selon une ou plusieurs des revendications 1 à 5 ou tels qu'obtenus conformément au procédé selon la revendication 6, pour utilisation en tant que médicaments.

8. Médicaments caractérisés par une teneur en au moins un composé de formule I telle que définie dans une ou plusieurs des revendications 1 à 5 et/ou au moins l'un de ses sels physiologiquement acceptables et/ou au moins un composé préparé conformément au procédé selon la revendication 6.

9. Médicaments selon la revendication 8, caractérisés en ce qu'ils sont destinés à la prophylaxie et/ou au traitement de troubles de la circulation, en particulier de troubles de la microcirculation, ainsi que des maladies qui en résultent.

10. Procédé pour la fabrication des médicaments selon la revendication 8 ou 9, caractérisé en ce que l'on met sous une forme d'administration appropriée au moins un composé de formule I telle que définie dans une ou plusieurs des revendications 1 à 5 et/ou au moins un de ses sels physiologiquement acceptables et/ou au moins un composé obtenu conformément au procédé selon la revendication 6, avec des véhicules ainsi qu'éventuellement des additifs et adjuvants usuels.

11. Utilisation des composés de formule I telle que définie dans une ou plusieurs des revendications 1 à 5 et/ou d'au moins un composé obtenu conformément au procédé selon la revendication 6, pour la prophylaxie et/ou le traitement de troubles de la circulation, en particulier de troubles de la microcirculation, ainsi que des maladies qui en résultent.

12. Chlorure de 1-méthyl-, 1,2-diméthyl- ou 1-éthyl-4-imidazolsulfonyle.

13. Procédé pour la préparation des composés mentionnés dans la revendication 12, caractérisé en ce que
a) on fait réagir du 1-méthyl- ou 1,2-diméthyl- ou 1-éthylimidazole avec de l'acide chlorosulfonique ClSO₃H, éventuellement avec addition ultérieure de SOCl₂, ou
b) on soumet à une chloration oxydante avec Cl₂ du 1-méthyl- ou 1,2-diméthyl- ou 1-éthyl-4-mercapto-imidazole.

## Revendications (Revendications pour l'(les) Etat(s) contractant(s) suivant(s): ES, GR)

1. Procédé pour la préparation des composés de type imidazole de formule I 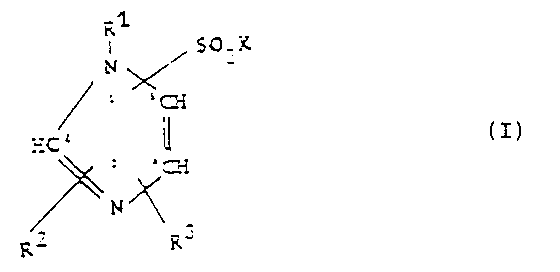 dans laquelle
R¹ est un groupe alkyle en C₁-C₆,
R² et R³ sont identiques ou différents et représentent chacun
H,
un atome d'halogène (F, Cl, Br, I) ou un groupe alkyle en C₁-C₃,
X est OH,
ou un groupe amino de formule II 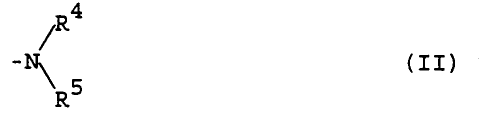 dans laquelle
R⁴ est H ou un groupe alkyle en C₁-C₇ éventuellement substitué par CN, NH₂ ou COOH,
R⁵ est un groupe alkyle en C₁-C₈ dans lequel - lorsqu'il a plus de 1 atome de carbone - un radical phénylène peut également être présent entre 2 atomes de carbone, et dont les atomes de carbone (aliphatiques) sont substitués par un ou plusieurs des groupes suivants: OH,
alcoxy en C₁-C₃,
phényle éventuellement substitué par 1-3 groupes OH,
alkyle en C₁-C₃, alcoxy(C₁-C₃)-COOH et/ou alcoxy(C₁-C₃)-alkyle(C₁-C₄),
COOH,
COO-alkyle(C₁-C₃),
CONH₂,
CN,
alcynyle en C₂-C₅,
NH₂, 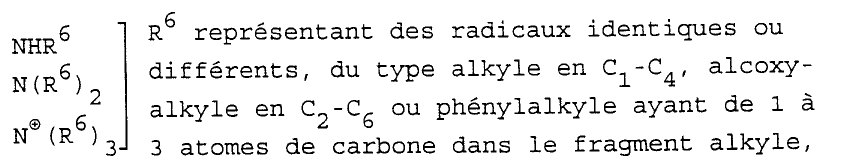 NH-CO-alkyle(C₁-C₆) 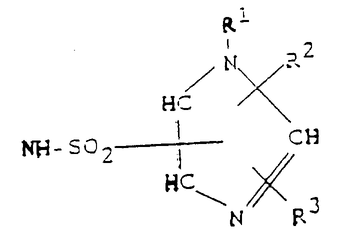 (significations de R¹, R² et R³: voir plus haut),
des radicaux hétérocycliques monocycliques à 5-7 chaînons, saturés ou insaturés, comportant dans le cycle un atome de N ainsi qu'éventuellement encore un atome de N, O ou S supplémentaire, choisis parmi les cycles thiomorpholine, pipérazine, morpholine, imidazole,
pyrrolidine et pipéridine,
éventuellement substitués par
un groupe alkyle en C₁-C₃,
phényle,
phénylalkyle ayant de 1 à 3 atomes de carbone dans le fragment alkyle,
OH et/ou oxo (=O), y compris les formes cétal ouvertes ou cycliques comportant 2-6 atomes de carbone dans le fragment cétal,
et dans lesquels l'atome de S du cycle - s'il est présent - peut également être oxydé en la forme sulfoxyde (SO) ou sulfone (SO₂),
ou dans laquelle
R⁴ et R⁵ forment, conjointement avec l'atome de N de la fonction amide auquel ils sont liés, un cycle hétérocyclique saturé à 5-7 chaînons, choisi parmi les cycles pipérazine et thiomorpholine,
et le cycle hétérocyclique peut par ailleurs être substitué par les groupes suivants:
alcoxy en C₁-C₃,
phénylalkyle ayant de 1 à 4 atomes de carbone dans le fragment alkyle,
phényle éventuellement substitué par un ou plusieurs groupes choisis parmi les suivants:
alkyle en C₁-C₃,
OH,
alcoxy en C₁-C₃,
alcoxy(C₁-C₃)-COOH,
alcoxy(C₁-C₃)-COO-alkyle(C₁-C₄), 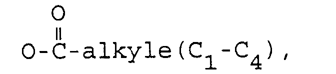 O-SO₂-C₆H₅,
O-SO₂-C₆H₄CH₃, 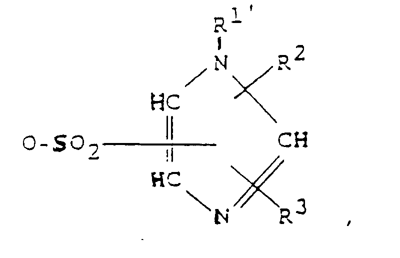 dans lequel
R^{1'} a la même signification que R¹ et en plus peut encore être H, et
R² et R³ ont les significations indiquées précédemment,
et l'atome de S du cycle - s'il est présent - peut également être oxydé en la forme sulfoxyde (SO) ou sulfone (SO₂),
et de leurs sels physiologiquement acceptables, caractérisé en ce que
a) on convertit par sulfonation un dérivé d'imidazole de formule générale III 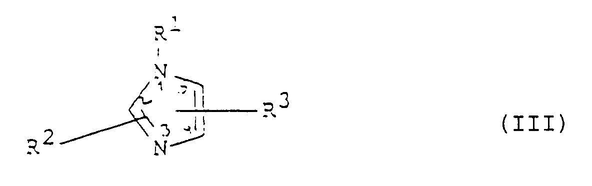 dans laquelle R¹, R² et R³ ont les significations données à propos de la formule I, et l'une des positions 4 et 5 est non substituée, en les composés de formule Ia 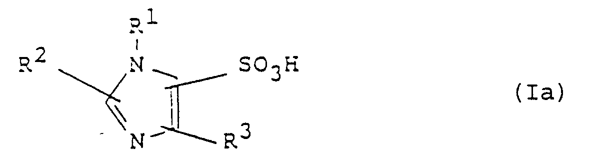 dans laquelle R¹, R² et R³ ont également les significations données à propos de la formule I, ou
b) on convertit un dérivé d'imidazole de formule générale IV 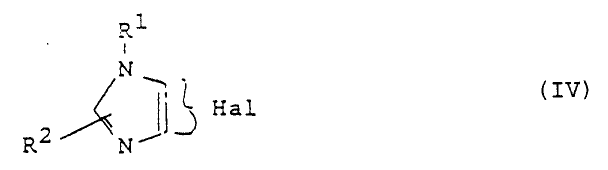 dans laquelle R¹ et R² ont les significations données à propos de la formule I, l'une des positions 4 et 5 porte un substituant halogène Hal (Cl, Br, I) et l'autre n'est pas substitué, par sulfonation et déshalogénation par hydrogénolyse subséquente, en un composé de formule Ib 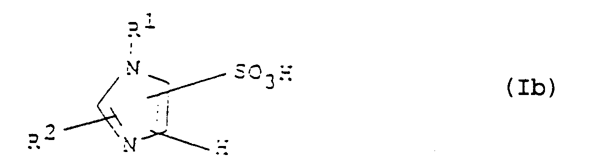 (significations de R¹ R²: comme dans la formule I)
ou
c) on hydrolyse un dérivé d'imidazole de formule générale V 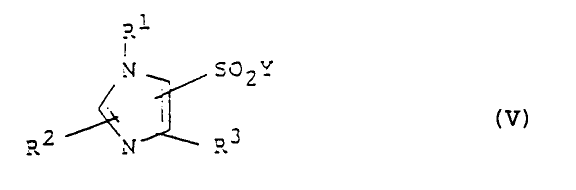 dans laquelle R¹, R² et R³ ont les significations données à propos de la formule I, et Y représente un atome d'halogène, pour aboutir aux acides imidazolsulfoniques de formule Ia avec les significations de R¹, R² et R³ qui y sont données,
ou
d) on oxyde un dérivé d'imidazole de formule générale VI ou VI' 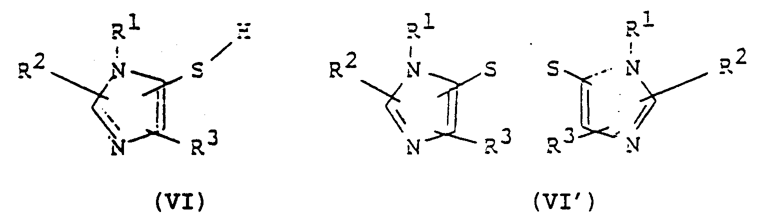 formules dans lesquelles R¹, R² et R³ ont les significations données à propos de la formule I, pour aboutir à l'acide imidazolsulfonique correspondant (formule Ia),
ou
e) on fait réagir un halogénure d'imidazolsulfonyle de formule générale V (voir variante c) avec une amine de formule H-II 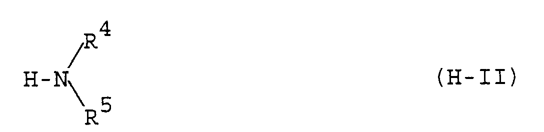 dans laquelle R⁴ et R⁵ ont les mêmes significations que dans la formule II, pour aboutir aux sulfonamides de formule Ic 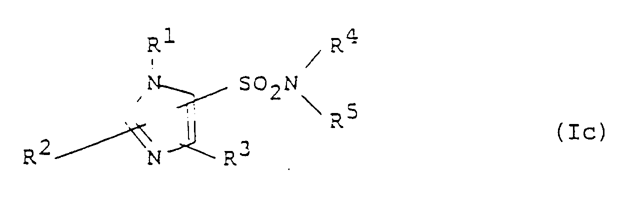 dans laquelle R¹ à R⁵ ont les significations données à propos des formules I et II,
ou
f) on fait réagir des dérivés d'imidazole de formule générale VII 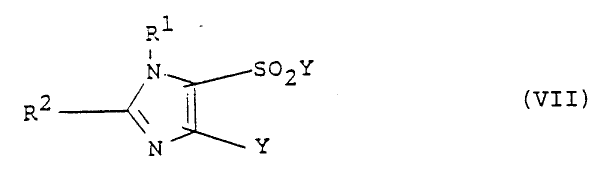 dans laquelle R¹ et R² ont les significations données à propos de la formule I, et Y représente des atomes d'halogène (Cl, Br, I) identiques ou différents, avec des amines de formule H-II, et on les soumet ensuite à une déshalogénation par hydrogénolyse, afin d'obtenir les sulfonamides non substitués en position 4 ou 5, de formule Id avec les significations indiquées plus haut pour R¹ et R² 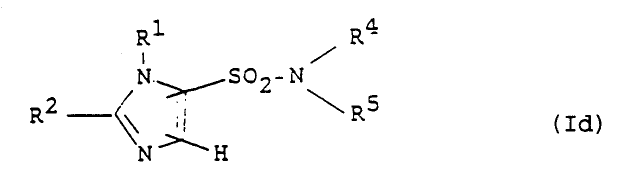 (significations de R¹, R², R⁴ et R⁵ comme dans les formules I et II),
ou
g) on fait réagir un halogénure d'imidazolsulfonyle de formule générale V (voir variante c) avec une trialkylsilylamine de formule VIII 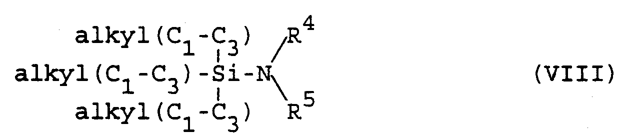 dans laquelle R⁴ et R⁵ ont les significations données à propos de la formule II, et le radical alkyle en C₁-C₃ préféré est le radical méthyle, pour obtenir les sulfonamides de formule Ic (voir variante e),
ou
h) pour la préparation des composés de formule I dans lesquels X = -N(R⁴)R⁵ (formule II) et R⁴ porte au moins un groupe NH₂ et/ou R⁵ porte au moins un groupe amino primaire ou secondaire, on fait réagir des amines de formule
H-N(R⁴)R⁵
qui portent sur leurs radicaux R⁴ et/ou R⁵ au moins un groupe amino correspondant, protégé à l'azote, avec des halogénures d'imidazolsulfonyle de formule V (voir variante c), et on élimine ensuite le groupe protecteur des sulfonamides obtenus,
ou
i) pour la préparation des composés de formule générale I dans lesquels X = -N(R⁴)R⁵ (formule II) et au moins l'un des radicaux R⁴ et R⁵ porte au moins un groupe amino primaire, on fait réagir des halogénures d'imidazolsulfonyle de formule V (voir variante c) avec des aminonitriles correspondants, et les sulfonylaminonitriles obtenus sont réduits en les composés amino,
ou
j) pour la préparation des composés de type imidazole de formule générale I dans lesquels X = -N(R⁴)R⁵ et R⁵ porte au moins un groupe amino quaternaire -N^{⊕}(R⁶)₃ dans lequel les radicaux R⁶ peuvent être identiques ou différents, on soumet à une alkylation, avec un agent d'alkylation, un composé de type imidazole de formule I dans lequel X = -N(R⁴)R⁵ et R⁵ porte au moins un groupe amino tertiaire -N(R⁶)₂,
ou
k) pour la préparation des composés de type imidazole de formule générale I dans lesquels X = -N(R⁴)R⁵ et R⁵ ou R⁴ et R⁵ ensemble contiennent un ou plusieurs groupes sulfoxyde ou sulfonyle, on oxyde des composés de type imidazole de formule générale I dans lesquels X = -N(R⁴)R⁵, et R⁵ ou R⁴ et R⁵ ensemble contiennent un ou plusieurs groupes sulfure, pour aboutir aux sulfoxydes ou sulfones correspondants,
ou
l) pour la préparation des composés de type imidazole de formule I dans lesquels X = -N(R⁴)R⁵ et R⁵ ou R⁴ et R⁵ ensemble contiennent un groupe phénoléther, on soumet des composés de type imidazole de formule générale I dans lesquels X = -N(R⁴)R⁵ et R⁵ ou R⁴ et R⁵ ensemble portent au moins un radical aryle substitué par un ou plusieurs groupes hydroxy phénoliques, à une alkylation avec des réactifs d'alkylation, de préférence des dérivés d'acides gras ω-halogénés, pour aboutir aux phénoléthers correspondants qui, dans le cas d'esters d'acides gras ω, peuvent être en outre soumis à une hydrolyse ou une aminolyse, conduisant aux acides carboxyliques ou carboxamides correspondants,
ou
m) pour la préparation des composés de type imidazole de formule I dans lesquels X = -N(R⁴)R⁵ et R⁵ ou R⁴ et R⁵ ensemble contiennent un groupe ester phénolique, on convertit en les esters phénoliques correspondants les mêmes copmposés de type imidazole de départ que pour la variante l), avec des réactifs d'acylation, de préférence avec des chlorures d'alkyl(C₁-C₄)carbonyle, des chlorures de benzène- et toluènesulfonyle ainsi que des halogénures d'imidazolsulfonyle de formule générale V (voir variante c), dans laquelle R¹, R² et R³ ont les mêmes significations que dans la formule I et R¹ peut en outre représenter un atome d'hydrogène,
et éventuellement on convertit encore les composés de formule I formés dans toutes les variantes du procédé - lorsqu'ils ne se trouvent pas déjà sous forme de sels physiologiquement acceptables - en de tels sels.

2. Procédé selon la revendication 1, caractérisé en ce que l'on prépare un composé de formule I, dans lequel est présente au moins l'une des caractéristiques suivantes:
a) R¹ = CH₃ ou C₂H₅,
b) R², R³ sont identiques ou différents et représentent chacun H, Cl ou CH₃, et
c) le radical -SO₂X se trouve en position 2 ou 4 du cycle imidazole.

3. Procédé selon la revendication 1 ou 2, caractérisé en ce que l'on prépare un composé de formule I dans lequel le radical 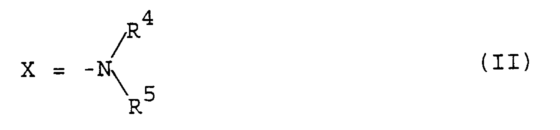 dans lequel
R⁴ = H et
R⁵ représente un radical alkyle en C₂-C₅ dans lequel éventuellement un radical phénylène est présent entre 2 atomes de carbone, et dont des atomes de carbone (aliphatiques) sont substitués par au total 1 ou 2 des groupes suivants: hydroxyphényle C₆H₄OH,
CN,
alcynyle en C₂-C₃,
NH₂, 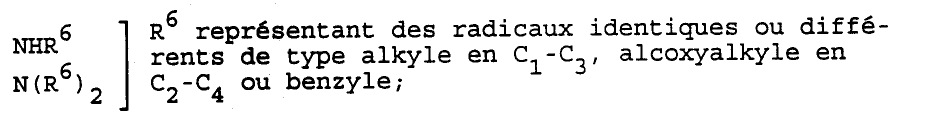 un radical monocyclique hétérocyclique saturé à 5-6 chaînons, choisi parmi: 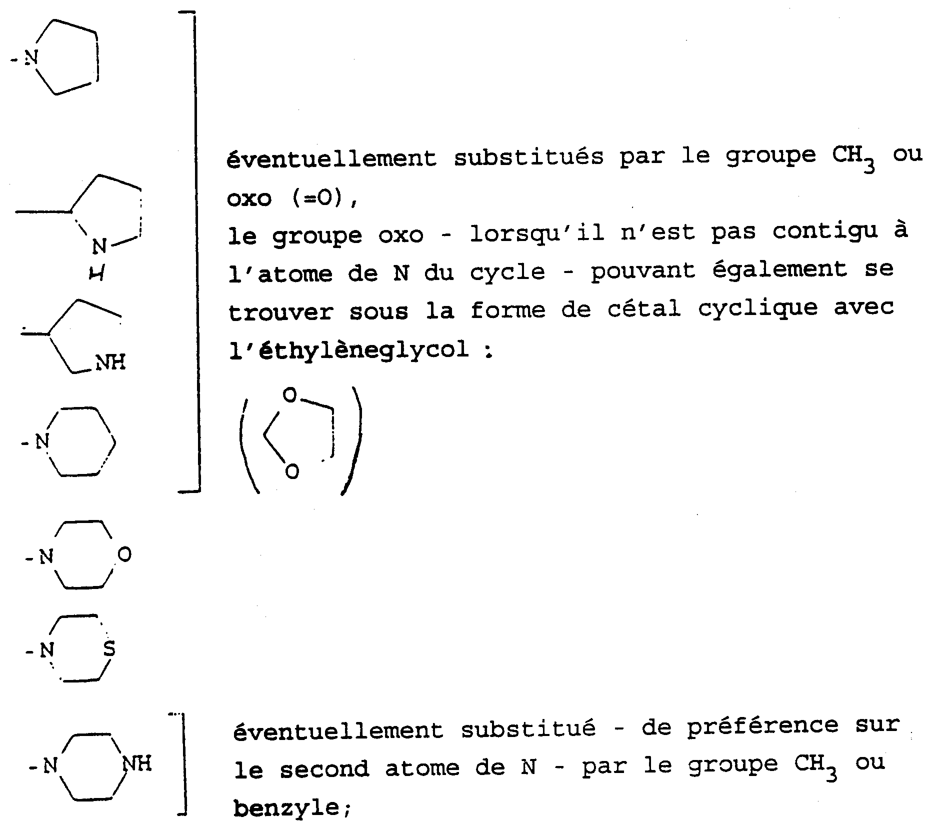 ou R⁴ et R⁵ forment ensemble, avec l'atome de N de la fonction amide auquel ils sont liés, un cycle hétérocyclique saturé à 6 chaînons, du type 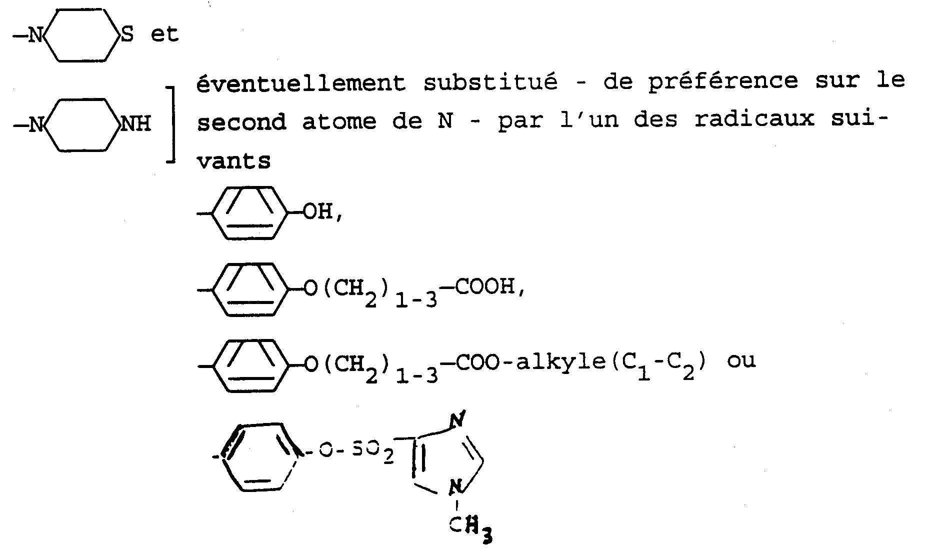 et leurs sels physiologiquement acceptables.

4. Procédé selon la revendication 1, caractérisé en ce que l'on prépare le composé de formule I selon la revendication 1 dans lequel
R¹ = CH₃,
R² = R³ = H,
le groupe SO₂X est en position 2 et 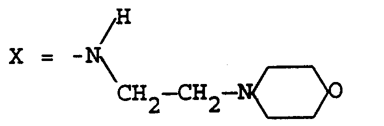 et ses sels physiologiquement acceptables.

5. Procédé selon la revendication 1, caractérisé en ce que l'on prépare le composé de formule I selon la revendication 1, dans lequel
R¹ = CH₃,
R² = R³ = H,
le groupe SO₂X est en position 4 et 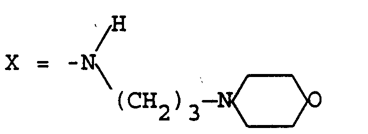 et ses sels physiologiquement acceptables.

6. Utilisation des composés de formule I ainsi que de leurs sels physiologiquement acceptables, obtenus selon une ou plusieurs des revendications 1 à 5, en tant que médicaments.

7. Utilisation d'au moins un composé de formule I obtenu selon une ou plusieurs des revendications 1 à 5 et/ou d'au moins un de ses sels physiologiquement acceptables, pour la fabrication de médicaments.

8. Utilisation d'au moins un composé de formule I obtenu selon une ou plusieurs des revendications 1 à 5 et/ou d'au moins un de ses sels physiologiquement acceptables, pour la fabrication de médicaments destinés à la prophylaxie et/ou au traitement de troubles de la circulation, en particulier de troubles de la microcirculation, ainsi que des maladies qui en résultent.

9. Procédé pour la fabrication des médicaments selon la revendication 7 ou 8, caractérisé en ce que l'on met sous une forme d'administration appropriée un composé de formule I obtenu selon une ou plusieurs des revendications 1 à 5 et/ou au moins un de ses sels physiologiquement acceptables, avec des véhicules ainsi qu'éventuellement des adjuvants et additifs usuels.

10. Utilisation des composés de formule I obtenus selon une ou plusieurs des revendications 1 à 5, pour la prophylaxie et/ou le traitement de troubles de la circulation, en particulier de troubles de la microcirculation, ainsi que des maladies qui en résultent.

11. Procédé pour la préparation du chlorure de 1-méthyl-, 1,2-diméthyl- ou 1-éthyl- 4-imidazolsulfonyle, caractérisé en ce que
a) on fait réagir du 1-méthyl- ou 1,2-diméthyl- ou 1-éthylimidazole avec de l'acide chlorosulfonique ClSO₃H, éventuellement avec addition ultérieure de SOCl₂, ou
b) on soumet à une chloration oxydante avec Cl₂ du 1-méthyl- ou 1,2-diméthyl- ou 1-éthyl-4-mercapto-imidazole.
